# EUROPEAN PATENT SPECIFICATION

(11) **EP 1 605 938 B1**
(45) Date of publication and mention of the grant of the patent: **11.02.2009**
(21) Application number: 04758428.9
(22) Date of filing: 26.03.2004
(51) Int. Cl.: C07D 471/04, C07D 491/04, C07D 498/04, C07D 513/04, C07D 519/00, A61K 31/4375, A61P 31/04

(54) **Antibacterial derivatives of napthydrin and quinoline**
Antibakterielle Naphthydrin- und Quinolinderivate
Dérivés antibacteriens de la naphthydrine et quinoline

(30) Priority: 27.03.2003 US 458147 P
(43) Date of publication of application: 21.12.2005
(73) Proprietor: GLAXO GROUP LIMITED, Greenford, Middlesex UB6 0NN (GB)
(72) Inventor: HENNESSY, Alan J., Harlow, Essex CM19 5AW (GB); MILLER, William, Henry, Collegeville, PA 19426 (US); SEEFELD, Mark, Andrew, Collegeville, PA 19426 (US)
(74) Representative: Valentine, Jill Barbara
(86) International application number: PCT/US2004/009371
(87) International publication number: WO 2004/087145

(56) References cited:
- WO-A1-00/56712
- WO-A1-02/056882
- US-A- 6 071 920
- AGRAWAL V K ET AL: "ANTIPARASITIC AGENTS: PART VI - SYNTHESIS OF 7-CHLORO-4-(4-SUBSTITUTED-PHENYLAMINO)- & 7-CHLORO-4-(4-SUBSTITUTED-P IPERAZIN-1-YL)QUINOLINES AS POTENTIAL ANTIPARASITIC AGENTS" INDIAN JOURNAL OF CHEMISTRY, JODHPUR, IN, vol. 26B, June 1987 (1987-06), pages 550-555, XP002919361

## Description

### FIELD OF THE INVENTION

This invention relates to novel compounds, compositions containing them and their use as antibacterials.

### BACKGROUND OF THE INVENTION

The emergence of pathogens resistant to known antibiotic therapy is becoming a serious global healthcare problem (Chu, et al., (1996) J. Med. Chem., 39: 3853-3874). Thus, there is a need to discover new broad spectrum antibiotics useful in combating multidrug-resistant organisms. Importantly, it has now been discovered that certain compounds have antibacterial activity, and, therefore, may be useful for the treatment of bacterial infections in mammals, particularly in humans.

WO 02/08224, WO 02/56882, WO 02/40474 and WO 02/72572 applications disclose quinoline and naphthyridine derivatives having antibacterial activity.

### SUMMARY OF THE INVENTION

This invention comprises compounds of the formula (I), as described hereinafter, which are useful in the treatment of bacterial infections. This invention is also a pharmaceutical composition comprising a compound according to formula (I) and a pharmaceutically acceptable carrier. This invention is also a method of treating bacterial infections in mammals, particularly in humans.

### DETAILED DESCRIPTION OF THE INVENTION

This invention provides a compound of formula (I): 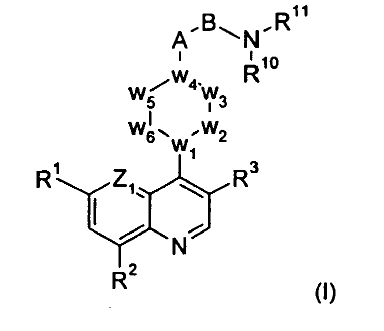 wherein:
Z₁ is N or CR^{1a};
R¹ and R^{1a} are independently hydrogen; hydroxy; (C₁₋₆)alkoxy unsubstituted or substituted by (C₁₋₆)alkoxy, hydroxy, amino, piperidyl, guanidino or amidino any of which is unsubstituted or N-substituted by one or two (C₁₋₆)alkyl, acyl, (C₁₋₆)alkylsulphonyl, CONH₂, hydroxy, (C₁₋₆)alkylthio, heterocyclylthio, heterocyclyloxy, arylthio, aryloxy, acylthio, acyloxy or (C₁₋₆)alkylsulphonyloxy; (C₁₋₆)alkoxy-substituted (C₁₋₆)alkyl; halogen; (C₁₋₆)alkyl; (C₁₋₆)alkylthio; trifluoromethyl; trifluoromethoxy; nitro; azido; cyano; acyl; acyloxy; acylthio; (C₁₋₆)alkylsulphonyl; (C₁₋₆)alkylsulphoxide; arylsulphonyl; arylsulphoxide; or an amino, piperidyl, guanidino or amidino group unsubstituted or N-substituted by one or two (C₁₋₆)alkyl, acyl or (C₁₋₆)alkylsulphonyl groups; or R¹ and R^{1a} may together form ethylenedioxy;
   provided that when Z₁ is CR^{1a} then R¹ is not H;
R² is H or halogen;
   provided that when Z₁ is N, then R² is H;
R³ is hydrogen; halogen; hydroxy; cyano; CF₃; nitro; azido; acyl; aryl; heteroaryl; CO₂H; acyloxy; acylthio; (C₁₋₆)alkyl unsubstituted or substituted by one or two (C₁₋₆)alkoxy, hydroxy, amino, piperidyl, guanidino or amidino any of which is unsubstituted or N-substituted by one or two (C₁₋₆)alkyl, acyl, (C₁₋₆)alkylsulphonyl, CONH₂, hydroxy, (C₁-₆)alkylthio, heterocyclylthio, heterocyclyloxy, arylthio, aryloxy, acylthio, acyloxy or (C₁-₆)alkylsulphonyloxy; (C₁₋₆)alkoxy unsubstituted or substituted by one or two (C₁₋₆)alkoxy, hydroxy, amino, piperidyl, guanidino or amidino any of which is unsubstituted or N-substituted by one or two (C₁₋₆)alkyl, acyl, (C₁₋₆)alkylsulphonyl, CONH₂, hydroxy, (C₁-₆)alkylthio, heterocyclylthio, heterocyclyloxy, arylthio, aryloxy, acylthio, acyloxy or (C₁-₆)alkylsulphonyloxy; (C₃₋₇)cycloalkyl; (C₁₋₆)alkoxy-substituted (C₁₋₆)alkyl; (C₁-₆)alkylthio; trifluoromethoxy; (C₁₋₆)alkylsulphonyl; (C₁₋₆)alkylsulphoxide; arylsulphonyl; or arylsulphoxide; or an amino, piperidyl, guanidino or amidino group unsubstituted or N-substituted by one or two (C₁₋₆)alkyl, acyl or (C₁₋₆)alkylsulphonyl groups;
w₁ is N, C, or CR⁴;
w₂ is C=O, CR⁴, or CR⁴R⁵;
w₃ is C=O or CR⁴R⁵;
w₄ is N or CR⁴;
w₅ is C=O or CR⁴R⁵;
w₆ is C=O, CR⁴, or CR⁴R⁵;
Alternatively, one of w₂, w₃, w₅ and w₆ is CR⁴R⁵CR⁴R⁵ and the others are defined as above;
wherein
each R⁴ and R⁵ is independently hydrogen; halogen; hydroxy; cyano; CF₃; nitro; azido; acyl; aryl; heteroaryl; CO₂H; acyloxy; acylthio; (C₁₋₆)alkyl unsubstituted or substituted by one or two (C₁₋₆)alkoxy, hydroxy, amino, piperidyl, guanidino or amidino any of which is unsubstituted or N-substituted by one or two (C₁₋₆)alkyl, acyl, (C₁₋₆)alkylsulphonyl, CONH₂, hydroxy, (C₁₋₆)alkylthio, heterocyclylthio, heterocyclyloxy, arylthio, aryloxy, acylthio, acyloxy or (C₁₋₆)alkylsulphonyloxy; (C₁₋₆)alkoxy unsubstituted or substituted by one or two (C₁₋₆)alkoxy, hydroxy, amino, piperidyl, guanidino or amidino any of which is unsubstituted or N-substituted by one or two (C₁₋₆)alkyl, acyl, (C₁₋₆)alkylsulphonyl, CONH₂, hydroxy, (C₁₋₆)alkylthio, heterocyclylthio, heterocyclyloxy, arylthio, aryloxy, acylthio, acyloxy or (C₁₋₆)alkylsulphonyloxy; (C₃₋₇)cycloalkyl; (C₁₋₆)alkoxy-substituted (C₁₋₆)alkyl; (C₁₋₆)alkylthio; trifluoromethoxy; (C₁₋₆)alkylsulphonyl; (C₁₋₆)alkylsulphoxide; arylsulphonyl; or arylsulphoxide; or an amino, piperidyl, guanidino or amidino group unsubstituted or N-substituted by one or two (C₁₋₆)alkyl, acyl or (C₁₋₆)alkylsulphonyl groups; or two R⁵ groups are joined together to form bicycloheptane;
A is CR⁶R⁷ or C(O);
B is CR⁸R⁹ or C(O);
R⁶, R⁷, R⁸, and R⁹ are independently hydrogen; halogen; hydroxy; cyano; CF₃; nitro; azido; acyl; aryl; heteroaryl; CO₂H; acyloxy; acylthio; (C₁₋₆)alkyl unsubstituted or substituted by one or two (C₁₋₆)alkoxy, hydroxy, amino, piperidyl, guanidino or amidino any of which is unsubstituted or N-substituted by one or two (C₁₋₆)alkyl, acyl, (C₁₋₆)alkylsulphonyl, CONH₂, hydroxy, (C₁₋₆)alkylthio, heterocyclylthio, heterocyclyloxy, arylthio, aryloxy, acylthio, acyloxy or (C₁₋₆)alkylsulphonyloxy; (C₁₋₆)alkoxy unsubstituted or substituted by one or two (C₁₋₆)alkoxy, hydroxy, amino, piperidyl, guanidino or amidino any of which is unsubstituted or N-substituted by one or two (C₁₋₆)alkyl, acyl, (C₁₋₆)alkylsulphonyl, CONH₂, hydroxy, (C₁₋₆)alkylthio, heterocyclylthio, heterocyclyloxy, arylthio, aryloxy, acylthio, acyloxy or (C₁₋₆)alkylsulphonyloxy; (C₃₋₇)cycloalkyl; (C₁₋₆)alkoxy-substituted(C₁₋₆)alkyl; (C₁₋₆)alkylthio; trifluoromethoxy; (C₁₋₆)alkylsulphonyl; (C₁₋₆)alkylsulphoxide; arylsulphonyl; or arylsulphoxide; or an amino, piperidyl, guanidino or amidino group unsubstituted or N-substituted by one or two (C₁₋₆)alkyl, acyl or (C₁₋₆)alkylsulphonyl groups;
R¹⁰ is hydrogen; aryl; heteroaryl; (C₁₋₆)alkyl unsubstituted or substituted by one or two (C₁₋₆)alkoxy, acyloxy, carboxy, hydroxy, amino, piperidyl, piperazinyl, morpholino, guanidino, or amidino, any of which is unsubstituted or N-substituted by one or two aryl, heteroaryl, halogen, unsubstituted (C₁₋₆)alkyl, acyl, (C₁₋₆)alkylsulphonyl, arylsulphonyl, hydroxy, (C₁₋₆)alkylthio, heterocyclylthio, heterocyclyloxy, arylthio, aryloxy, acylthio, acyloxy, or (C₁₋₆)alkylsulphonyloxy, so long as the substitution does not lead to an unstable compound; (C₁₋₆)alkylcarbonyl; (C₂₋₆)alkenylcarbonyl; (C₁₋₆)alkoxycarbonyl; CO₂H; or CF₃;
R¹¹ is a group -U-R¹² where R¹² is a substituted or unsubstituted bicyclic carbocyclic or heterocyclic ring system (A): 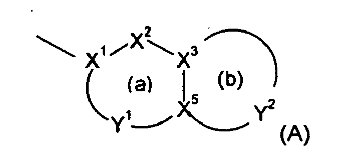 containing up to four heteroatoms in each ring in which
at least one of rings (a) and (b) is aromatic;
X¹ is C or N when part of an aromatic ring or CR¹⁴ when part of a non aromatic ring;
X² is N, NR¹³, O, S(O)ₓ, CO or CR¹⁴ when part of an aromatic or non-aromatic ring or may in addition be CR¹⁴R¹⁵ when part of a non aromatic ring;
X³ and X⁵ are independently N or C;
Y¹ is a 0 to 4 atom linker group each atom of which is independently selected from N, NR¹³, O, S(O)ₓ, CO and CR¹⁴ when part of an aromatic or non-aromatic ring or may additionally be CR¹⁴R¹⁵ when part of a non aromatic ring,
Y² is a 2 to 6 atom linker group, each atom of Y² being independently selected from N, NR¹³,O, S(O)ₓ, CO and CR¹⁴ when part of an aromatic or non-aromatic ring or may additionally be CR¹⁴R¹⁵ when part of a non aromatic ring; each of R¹⁴ and R¹⁵ is independently selected from: H; (C₁₋₄)alkylthio; halo; (C₁₋₄)alkyl; (C₂₋₄)alkenyl; hydroxy; hydroxy(C₁₋₄)alkyl; mercapto(C₁₋₄)alkyl; (C_{1- 4})alkoxy; trifluoromethoxy; nitro; cyano; carboxy; amino or aminocarbonyl unsubstituted or substituted by (C₁₋₄)alkyl;
each R¹³ is independently H; trifluoromethyl; (C₁₋₄)alkyl unsubstituted or substituted by hydroxy, carboxy, (C₁₋₄)alkoxy, (C₁₋₆)alkylthio, halo or trifluoromethyl; (C₂₋₄)alkenyl; or aminocarbonyl wherein the amino group is optionally substituted (C₁₋₄)alkyl;
each x is independently 0, 1 or 2;
U is CO, SO₂, CH₂, or CR¹⁶R¹⁷; and
R¹⁶ and R¹⁷ are independently selected from H; aryl; heteroaryl; (C₁-₆)alkyl; (C₁₋₆)alkyl substituted by (C₁₋₆)alkoxy, hydroxy, amino, piperidyl, piperazinyl, morpholino, guanidino, or amidino, any of which is substituted or N-substituted by one or two H, aryl, heteroaryl, halogen, cyano, CF₃, (C₁₋₆)alkyl, acyl, (C₁₋₆)alkylsulphonyl, arylsulphonyl, hydroxy, (C₁₋₆)alkylthio, heterocyclylthio, heterocyclyloxy, arylthio, aryloxy, acylthio, acyloxy, or (C₁₋₆)alkylsulphonyloxy, provided that the substitution does not lead to an unstable compound; (C₁₋₆)alkoxy-substituted (C₁₋₆)alkyl; hydroxy-substituted (C₁₋₆)alkyl; amino-substituted (C₁₋₆)alkyl, which is N-substituted by one or two (C₁₋₆)alkyl, acyl, (C₁₋₆)alkylsulphonyl, or arylsulphonyl; (C₁₋₆)alkylcarbonyl; (C₂₋₆)alkenylcarbonyl; (C₁₋₆)alkoxycarbonyl; CO₂H; or CF₃; or
a pharmaceutically acceptable salt thereof.

The invention also provides a pharmaceutical composition, in particular for use in the treatment of bacterial infections in mammals, particularly humans, comprising a compound of formula (I), or a pharmaceutically acceptable salt thereof, and a pharmaceutically acceptable carrier.

The invention also provides the use of a compound of formula (I), or a pharmaceutical acceptable salt thereof in the manufacture of a medicament for use in the treatment of bacterial infections in mammals, particularly in humans.

Preferably R¹ is F, Cl, OCH₃, methyl, or SCH₃. Most preferably R¹ is F, Cl, or OCH₃.

Preferably, R¹ a is H, OCH₃, or OCH₂CH₂OCH₃.

Preferably, R² is H or F. Most preferably R² is H.

Preferably, R³ is Cl or F.

Preferably w₁ is N or CR⁴.

Preferably w₂, w₃, w₅, and w₆ are CR⁴R⁵.

Preferably each R⁴ is independently H, methyl, OH, -COOH, NH₂, OCH₃ or - CH₂OH.

Preferably R⁵ is H.

Preferably A is CR⁶R⁷.

Preferably B is CR⁸R⁹.

R6 and R8 are preferably H.

Preferably R7 is H or OH.

Preferably R9 is H or OH.

Preferably R¹⁰ is H.

The group -U- is preferably -CH₂-.

Preferably R¹² is an aromatic heterocyclic ring (A) having 8-11 ring atoms including 2-4 heteroatoms of which at least one is N or NR¹³, in which preferably Y² contains 2-3 heteroatoms, one of which is S and 1-2 are N, with one N bonded to X³.

Alternatively and preferably the heterocyclic ring (A) has ring (a) aromatic selected from optionally substituted benzo and pyrido and ring (b) non-aromatic and Y² has 3-5 atoms including a heteroatom bonded to X⁵ selected from NR¹³, O or S and NHCO bonded via N to X³, or O bonded to X³.

Examples of rings (A) include optionally substituted:

### (a) and (b) aromatic

1H-pyrrolo[2,3-b]-pyridin-2-yl, 1H-pyrrolo[3,2-b]-pyridin-2-yl, 3H-imidazo[4,5-b]-pyrid-2-yl, 3H-quinazolin-4-one-2-yl, benzimidazol-2-yl, benzo[1,2,3]-thiadiazol-5-yl, benzo[1,2,5]-oxadiazol-5-yl, benzofur-2-yl, benzothiazol-2-yl, benzo[b]thiophen-2-yl, benzoxazol-2-yl, chromen-4-one-3-yl, imidazo[1,2-a]pyridin-2-yl, imidazo-[1,2-a]-pyrimidin-2-yl, indol-2-yl, indol-6-yl, isoquinolin-3-yl, [1,8]-naphthyridine-3-yl, oxazolo[4,5-b]-pyridin-2-yl, quinolin-2-yl, quinolin-3-yl, quinoxalin-2-yl, indan-2-yl, naphthalen-2-yl, 1,3-dioxoisoindol-2yl, benzimidazol-2-yl, benzothiophen-2-yl, 1H-benzotriazol-5-yl, 1H-indol-5-yl, 3H-benzooxazol-2-one-6-yl, 3H-benzooxazol-2-thione-6-yl, 3H-benzothiazol-2-one-5-yl, 3H-quinazolin-4-one-2-yl, 3H-quinazolin-4-one-6-yl, 4-oxo-4H-pyrido[1,2-a]pyrimidin-3-yl, benzo[1,2,3]thiadiazol-6-yl, benzo[1,2,5]thiadiazol-5-yl, benzo[1,4]oxazin-2-one-3-yl, benzothiazol-5-yl, benzothiazol-6-yl, cinnolin-3-yl, imidazo[1,2-a]pyridazin-2-yl, imidazo[1,2-b]pyridazin-2-yl, pyrazolo[1,5-a]pyrazin-2-yl, pyrazolo[1,5-a]pyridin-2-yl, pyrazolo[1,5-a]pyrimidin-6-yl, pyrazolo[5,1-c][1,2,4]triazin-3-yl, pyrido[1,2-a]pyrimdin-4-one-2-yl, pyrido[1,2-a]pyrimidin-4-one-3-yl, quinazolin-2-yl, quinoxalin-6-yl, thiazolo[3,2-a]pyrimidin-5-one-7-yl, thiazolo[5,4-b]pyridin-2-yl, thieno[3,2-b]pyridin-6-yl, thiazolo[5,4-b]pyridin-6-yl, 4-oxo-4H-pyrido[1,2-a]pyrimidin-2-yl, 1-oxo-1,2-dihydro-isoquinolin-3-yl, thiazolo[4,5-b]pyridin-5-yl, [1,2,3]thiadiazolo[5,4-b]pyridin-6-yl, 2H-isoquinolin-1-one-3-yl

### (a) is non aromatic

(2S)-2,3-dihydro-1 H-indol-2-yl, (2S)-2,3-dihydro-benzo[1,4]dioxine-2-yl, 3-(R,S)-3,4-dihydro-2H-benzo[1,4]thiazin-3-yl, 3-(R)-2,3-dihydro-[1,4]dioxino[2,3-b]pyridin-3-yl, 3-(S)-2,3-dihydro-[1,4]dioxino[2,3-b]pyridin-3-yl, 2,3-dihydro-benzo[1,4]dioxan-2-yl, 3-substituted-3H-quinazolin-4-one-2-yl, 2,3-dihydro-benzo[1,4]dioxan-2-yl, 1-oxo-1,3,4,5-tetrahydrobenzo[c]azepin-2-yl.

### (b) is non aromatic

1,1,3-trioxo-1,2,3,4-tetrahydro-1 β-benzo[1,4] thiazin-6-yl, benzo[1,3]dioxol-5-yl, 2,3-dihydro-benzo[1,4]dioxin-6-yl, 2-oxo-2,3-dihydro-benzooxazol-6-yl, 4H-benzo[1,4]oxazin-3-one-6-yl (3-oxo-3,4-dihydro-2H-benzo[1,4]oxazin-6-yl), 4H-benzo[1,4]thiazin-3-one-6-yl (3-oxo-3,4-dihydro-2H-benzo[1,4]thiazin-6-yl), 4H-benzo[1,4]oxazin-3-one-7-yl, 4-oxo-2,3,4,5-tetrahydro-benzo[b][1,4]thiazepine-7-yl, 5-oxo-2,3-dihydro-5H-thiazolo[3,2-a]pyrimidin-6-yl, benzo[1,3]dioxol-5-yl, 2-oxo-2,3-dihydro-1 H-pyrido[2,3-b][1,4]thiazin-7-yl, 2-oxo-2,3-dihydro-1H-pyrido[3,4-b][1,4]thiazin-7-yl, 3-oxo-3,4-dihydro-2H-pyrido[3,2-b][1,4]thiazin-6-yl, 2,3-dihydro-[1,4]dioxino[2,3-b]pyridin-6-yl, 2,3-dihydro-[1,4]dioxino[2,3-c]pyridin-7-yl, 2,3-dihydro-[1,4]dioxino[2,3-b]pyridin-7-yl,6,7-dihydro-[1,4]dioxino[2,3-d]pyrimidin-2-yl, 3-oxo-3,4-dihydro-2H-pyrido[3,2-b][1,4]oxazin-6-yl, 2-oxo-2,3-dihydro-1H-pyrido[3,4-b][1,4]oxazin-7-yl, 2-oxo-2,3-dihydro-1H-pyrido[2,3-b][1,4]oxazin-7-yl, 6-oxo-6,7-dihydro-5H-8-thia-1,2,5-triaza-naphthalen-3-yl, 3,4-dihydro-2H-benzo[1,4]oxazin-6-yl, 3-substituted-3H-benzooxazol-2-one-6-yl, 3-substituted-3H-benzooxazole-2-thione-6-yl, 3-substituted-3H-benzothiazol-2-one-6-yl, 2,3-dihydro-1 H-pyrido[2,3-b][1,4]thiazin-7-yl, 3,4-dihydro-2H-benzo[1,4]thiazin-6-yl, 3,4-dihydro-1H-quinolin-2-one-7-yl, 3,4-dihydro-1H-quinoxalin-2-one-7-yl, 6,7-dihydro-4H-pyrazolo[1,5-a]pyrimidin-5-one-2-yl, 5,6,7,8-tetrahydro-[1,8]naphthyridin-2-yl, 2-oxo-3,4-dihydro-1*H* [1,8]naphthyridin-6-yl, 3,4-dihydro-2H-pyrido[3,2-b][1,4]thiazin-6-yl.

R¹³ is preferably H if in ring (a) or in addition (C₁₋₄)alkyl such as methyl or isopropyl when in ring (b). More preferably, in ring (b) R¹³ is H when NR¹³ is bonded to X³ and (C₁₋₄)alkyl when NR¹³ is bonded to X⁵.

R¹⁴ and R¹⁵ are preferably independently selected from hydrogen, halo, hydroxy, (C₁₋₄) alkyl, (C₁₋₄)alkoxy, trifluoromethoxy; nitro, cyano, aryl(C₁₋₄)alkoxy and (C₁₋₄)alkylsulphonyl.

More preferably R¹⁵ is hydrogen.

More preferably each R¹⁴ is selected from hydrogen, chloro, fluoro, hydroxy, methyl, methoxy, trifluoromethoxy, benzyloxy, nitro, cyano and methylsulphonyl. Most preferably R¹⁴ is selected from hydrogen, hydroxy, fluorine or nitro. Preferably 0-3 groups R¹⁴ are substituents other than hydrogen.

Preferred groups R¹² include:
[1,2,3]thiadiazolo[5,4-b]pyridin-6-yl;
1 H-Pyrrolo[2,3-b]pyridin-2-yl;
2,3-Dihydro-[1,4]dioxino[2,3-b]pyridin-6-yl;
2,3-Dihydro-[1,4]dioxino[2,3-b]pyridin-7-yl;
2,3-Dihydro-[1,4]dioxino[2,3-c]pyridin-7-yl;
2,3-dihydro-benzo[1,4]dioxin-6-yl;
2-oxo-2,3-dihydro-1H-pyrido[2,3-b][1,4]oxazin-7-yl;
2-oxo-2,3-dihydro-1H-pyrido[2,3-b][1,4]thiazin-7-yl;
3,4-dihydro-2H-benzo[1,4]oxazin-6-yl;
3-Methyl-2-oxo-2, 3-dihydro-benzooxazol-6-yl;
3-oxo-3,4-dihydro-2H-benzo[1,4]oxazin-6-yl;
3-oxo-3,4-dihydro-2H-pyrido[3,2-b][1,4]oxazin-6-yl;
4H-benzo[1,4] thiazin-3-one-6-yl;
4-oxo-4H-pyrido[1,2-a]pyrimidin-2-yl;
6-nitro-benzo[1,3]dioxol-5-yl;
7-fluoro-3-oxo-3,4-dihydro-2H-benzo[1,4] oxazin-6-yl;
8-Hydroxy-1-oxo-1,2-dihydro-isoquinolin-3-yl;
8-hydroxyquinolin-2-yl;
benzo[1,2,3]thiadiazol-5-yl;
benzo[1,2,5]thiadiazol-5-yl;
benzothiazol-5-yl;
thiazolo-[5,4-b]pyridin-6-yl;
3-oxo-3,4-dihydro-2*H*-pyrido[3,2-b][1,4]thiazin-6-yl ;
7-chloro-3-oxo-3,4-dihydro-2*H-*pyrido[3,2-b][1,4]thiazin-6-yl;
7-fluoro-3-oxo-3,4-dihydro-2*H*-pyrido[3,2-b][1,4]thiazin-6-yl, and
2-oxo-2,3-dihydro-1*H*-pyrido[3,4-b][1,4]thiazin-7-yl.

Most preferred groups R¹² include:
benzo[1,2,5]thiadiazol-5-yl;
4H-benzo[1,4] thiazin-3-one-6-yl;
2,3-dihydro-benzo[1,4]dioxin-6-yl;
benzo[1,2,3]thiadiazol-5-yl;
3-oxo-3,4-dihydro-2H-benzo[1,4]oxazin-6-yl;
7-fluoro-3-oxo-3,4-dihydro-2H-benzo[1,4] oxazin-6-yl;
2-oxo-2,3-dihydro-1H-pyrido[2,3-b][1,4]thiazin-7-yl;
2,3-dihydro-[1,4]dioxino[2,3-c]pyridin-7-yl;
3-oxo-3,4-dihydro-2H-pyrido[3,2-b][1,4]oxazin-6-yl;
[1,2,3]thiadiazolo[5,4-b]pyridin-6-yl;
3-oxo-3,4-dihydro-2H-pyrido[3,2-b][1,4]thiazin-6-yl;
7-chloro-3-oxo-3,4-dihydro-2H-pyrido[3,2-b][1,4]thiazin-6-yl;
7-fluoro-3-oxo-3,4-dihydro-2H-pyrido[3,2-b][1,4]thiazin-6-yl, and
2-oxo-2,3-dihydro-1*H*-pyrido[3,4-b][1,4]thiazin-7-yl.

Most especially preferred groups R¹² include:
3-oxo-3,4-dihydro-2*H*-pyrido[3,2-b][1,4]thiazin-6-yl,
3-oxo-3,4-dihydro-2H-pyrido[3,2-b][1,4]oxazin-6-yl, and
2,3-dihydro-[1,4]dioxino[2,3-c]pyridin-7-yl.

Preferred compounds of this invention include:
6-({2-[1-(6-methoxyquinolin-4-yl)piperidin-4-yl]ethylamino}methyl)-4*H*-pyrido[3,2-b][1,4]oxazin-3-one;
6-({2-[1-(6-methoxyquinolin-4-yl)piperidin-4-yl]ethylamino}methyl)-4*H*-pyrido[3,2-b][1,4]thiazin-3-one;
(2,3-dihydro-[1,4]dioxino[2,3-c]pyridin-7-ylmethyl)-2-[1-(6-methoxy quinolin-4-yl)piperidin-4-yl]ethyl}amine;
6-({2-[1-(6-methoxynaphthyridin-4-yl)piperidin-4-yl]ethylamino} methyl)-4*H-*pyrido[3,2-b][1,4]oxazin-3-one;
6-({2-[1-(6-methoxynaphthyridin-4-yl)piperidin-4-yl]ethylamino} methyl)-4*H-*pyrido[3,2-b][1,4]thiazin-3-one;
(2,3-dihydro-[1,4]dioxino[2,3-c]pyridin-7-ylmethyl)-{2-[1-(6-methoxy naphthyridin-4-yl)piperidin-4-yl]ethyl}amine;
6-({2-[1-(3-chloro-6-methoxy-[1,5]quinolin-4-yl)phenyl]ethylamino} methyl)-4*H-*pyrido[3,2-b][1,4]oxazin-3-one;
6-({2-[1-(3-chloro-6-methoxy-[1,5]quinolin-4-yl)phenyl]ethylamino} methyl)-4*H* pyrido[3,2-*b*][1,4]thiazin-3-one;
{2-[1-(3-chloro-6-methoxyquinolin-4-yl)piperidin-4-yl]ethyl}-(2,3-dihydro [1,4]dioxino[2,3-c]pyridin-7-ylmethyl)amine;
6-({2-[1-(3-chloro-6-methoxy-[1,5]naphthyridin-4-yl)phenyl] ethylamino}methyl) - 4*H*-pyrido[3,2-*b*][1,4]oxazin-3-one;
6-({2-[1-(3-chloro-6-methoxy-[1,5]naphthyridin-4-yl)phenyl] ethylamino}methyl) - 4*H*-pyrido[3,2-*b*][1,4]thiazin-3-one;
{2-[1-(3-chloro-6-methoxynaphthyridin-4-yl)piperidin-4-yl]ethyl}-(2,3-dihydro[1,4]dioxino[2,3-c]pyridin-7-ylmethyl)amine;
6-({2-[4-(6-methoxyquinolin-4-yl)piperazin-1-yl]ethylamino}methyl)-4*H*-pyrido[3,2-b][1,4]oxazin-3-one;
6-({2-[4-(6-methoxyquinolin-4-yl)piperazin-1-yl]ethylamino}methyl)-4*H*-pyrido[3,2-b][1,4]thiazin-3-one;
(2,3-dihydro-[1,4]dioxino[2,3-c]pyridin-7-ylmethyl)-{2-[4-(6-methoxyquinolin-4-yl)piperizinyl]ethyl}amine;
6-({2-[4-(6-methoxynaphthyridin-4-yl)piperazin-1-yl]ethylamino} methyl-4*H-*pyrido[3,2-b][1,4]oxazin-3-one;
6-({2-[4-(6-methoxynaphthyridin-4-yl)piperazin-1-yl]ethylamino} methyl)-4*H-*pyrido[3,2-b][1,4]thiazin-3-one;
(2,3-dihydro-[1,4]dioxino[2,3-c]pyridin-7-ylmethyl)-{2-[4-(6-methoxynaphthyridin-4-yl)piperizin-1-yl]ethyl}amine;
6-({2-[4-(3-chloro-6-methoxyquinolin-4-yl)piperazin-1-yl] ethylamino}methyl)-4*H-*pyrido[3,2-b][1,4]oxazin-3-one;
6-({2-[4-(3-chloro-6-methoxyquinolin-4-yl)piperazin-1-yl] ethylamino} methyl)-4H-pyrido[3,2-*b*][1,4]thiazin-3-one;
{2-[4-(3-chloro-6-methoxyquinolin-4-yl)piperazin-1-yl]ethyl}-(2,3-dihydro[1,4]dioxino[2,3-c]pyridin-7-ylmethyl)amine;
6-({2-[4-(3-chloro-6-methoxynaphthyridin-4-yl)piperazin-1-yl] ethylamino} methyl)-4*H*-pyrido[3,2-*b*][1,4]oxazin-3-one;
6-({2-[4-(3-chloro-6-methoxynaphthyridin-4-yl)piperazin-1-yl] ethylamino} methyl)-4*H*-pyrido[3,2-*b*][1,4]thiazin-3-one;
{2-[4-(3-chloro-6-methoxynaphthyridin-4-yl)piperazin-1-yl]ethyl}-(2,3-dihydro[1,4]dioxino[2,3-*c*]pyridin-7-ylmethyl)amine;
6-({2-[4-(6-methoxy-[1,5]naphthyridin-4-yl)-3,6-dihydro-2H -pyridin-1-yl]-2-oxo-ethylamino}-methyl) -4 H -pyrido[3,2-b][1,4]thiazin-3-one;
N-(2-{1-[6-(methyloxy)-1,5-naphthyridin-4-yl]-4-piperidinyl}ethyl)-3-oxo-3,4-dihydro-2H-pyrido[3,2-b][1,4]thiazine-6-carboxamide;
N-(2-{1-[6-(methyloxy)-1,5-naphthyridin-4-yl]-4-piperidinyl}ethyl)-3-oxo-3,4-dihydro-2H-1,4-benzothiazine-6-sulfonamide;
N-methyl-N-(2-{1-[6-(methyloxy)-1,5-naphthyridin-4-yl]-4-piperidinyl) ethyl)-3-oxo-3,4-dihydro-2H-pyrido[3,2-b][1,4]thiazine-6-carboxamide;
N-methyl-N-(2-{1-[6-(methyloxy)-1,5-naphthyridin-4-yl]-4-piperidinyl} ethyl)-3-oxo-3,4-dihydro-2H-1,4-benzothiazine-6-sulfonamide;
N-(2-{1-[3-chloro-6-(methyloxy)-1,5-naphthyridin-4-yl]-4-piperidinyl}ethyl)-3-oxo-3,4-dihydro-2H-1,4-benzothiazine-6-sulfonamide;
N-(2-{4-[6-(methyloxy)-1,5-naphthyridin-4-yl]-1-piperazinyl}ethyl)-3-oxo-3,4-dihydro-2H-pyrido[3,2-b][1,4]thiazine-6-carboxamide;
N-methyl-N-(2{4-[6-(methyloxy)-1,5-naphthyridin-4-yl]-1-piperazinyl} ethyl)-3-oxo-3,4-dihydro-2H-pyrido[3,2-b][1,4]thiazine-6-carboxamide;
N-(2-{4-[6-(methyloxy)-1,5-naphthyridin-4-yl]-1-piperazinyl}ethyl)-3-oxo-3,4-dihydro-2H-1,4-benzothiazine-6-sulfonamide;
N-methyl-N-(2-{4-[6-(methyloxy)-1,5-naphthyridin-4-yl]-1-piperazinyl} ethyl)-3-oxo-3,4-dihydro-2H-1,4-benzothiazine-6-sulfonamide;
6-{[(2-{4-[6-(methyloxy)-1,5-naphthyridin-4-yl]hexahydro-1H-1,4-diazepin-1-yl}ethyl)amino]methyl}-2H-pyrido[3,2-b][1,4]thiazin-3(4H)-one;
N-(2-{4-[6-(methyloxy)-1,5-naphthyridin-4-yl]hexahydro-1H-1,4-diazepin-1-yl}ethyl)-3-oxo-3,4-dihydro-2H-1,4-benzothiazine-6-sulfonamide;
6-{[(2-{(1R,4R)-5-[6-(methyloxy)-1,5-naphthyridin-4-yl]-2,5-diazabicyclo[2.2.1]hept-2-yl}ethyl)amino]methyl}-2H-pyrido[3,2-b][1,4]thiazin-3(4H)-one;
6-{[(2-{4-hydroxy-1-[6-(methyloxy)-1,5-naphthyridin-4-yl]-4-piperidinyl}ethyl) amino]methyl}-2H-pyrido[3,2-b][1,4]thiazin-3(4H)-one;
6-{[(2-{4-hydroxy-1-[6-(methyloxy)-1,5-naphthyridin-4-yl]-4-piperidinyl}ethyl) amino]methyl}-2H-pyrido[3,2-b][1,4]oxazin-3(4H)-one;
N-(2-{4-hydroxy-1-[6-(methyloxy)-1,5-naphthyridin-4-yl]-4-piperidinyl}ethyl)-3-oxo-3,4-dihydro-2H-1,4-benzothiazine-6-sulfonamide; or
a pharmaceutically acceptable salt thereof.

Unless otherwise defined, the term (C₁₋₆)alkyl when used alone or when forming part of other groups (such as the 'alkoxy' group) includes substituted or unsubstituted, straight or branched chain alkyl groups containing 1 to 6 carbon atoms. Examples of (C₁₋₃)alkyl include methyl, ethyl, n-propyl, and isopropyl groups.

The term (C₂₋₆)alkenyl means a substituted or unsubstituted alkyl group of 2 to 6 carbon atoms, wherein one carbon-carbon single bond is replaced by a carbon-carbon double bond. Examples of (C₂₋₆)alkenyl include ethylene, 1-propene, 2-propene, 1-butene, 2-butene, and isobutene. Both cis and trans isomers are included.

The term (C₃₋₇)cycloalkyl refers to subsituted or unsubstituted carbocyclic system of three to seven carbon atoms, which may contain up to two unsaturated carbon-carbon bonds. Examples of (C₃₋₇)cycloalkyl include cyclopropyl, cyclobutyl, cyclopentyl, cyclopentenyl, cyclohexyl, cyclohexenyl, and cycloheptyl.

Unless otherwise defined, suitable substituents for any (C₁₋₆)alkyl, (C₁₋₆)alkoxy, (C₂₋₆)alkenyl, and (C₃₋₇)cycloalkyl groups includes up to three substituents selected from the group consisting of hydroxy, halogen, nitro, cyano, carboxy, amino, amidino, sulphonamido, unsubstituted (C₁₋₃)alkoxy, trifluromethyl, acyloxy.

Halo or halogen includes fluoro, chloro, bromo and iodo.

Haloalkyl moieties include 1-3 halogen atoms.

Unless otherwise defined, the term "heterocyclic" as used herein includes optionally substituted aromatic and non-aromatic, single and fused, rings suitably containing up to four hetero-atoms in each ring selected from oxygen, nitrogen and sulphur, which rings may be unsubstituted or C-substituted by, for example, up to three groups selected from (C₁₋₄)alkylthio; halo; halo(C₁₋₄)alkoxy; halo(C₁₋₄)alkyl; (C₁₋₄)alkyl; (C₂₋₄)alkenyl; hydroxy; hydroxy, (C₁₋₄)alkyl; mercapto(C₁₋₄)alkyl; (C₁₋₄)alkoxy; nitro; cyano, carboxy; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl.

Each heterocyclic ring suitably has from 4 to 7, preferably 5 or 6, ring atoms. A fused heterocyclic ring system may include carbocyclic rings and need include only one heterocyclic ring.

Compounds within the invention containing a heterocyclyl group may occur in two or more tautometric forms depending on the nature of the heterocyclyl group; all such tautomeric forms are included within the scope of the invention.

Where an amino group forms part of a single or fused non-aromatic heterocyclic ring as defined above suitable optional substituents in such substituted amino groups include H; trifluoromethyl; (C₁₋₄)alkyl optionally substituted by hydroxy,(C₁₋₄)alkoxy, (C₁₋₄)alkylthio, halo or trifluoromethyl; (C₂₋₄)alkenyl;

When used herein the term "aryl", includes optionally substituted phenyl and naphthyl.

Aryl groups may be optionally substituted with up to five, preferably up to three, groups selected from (C₁₋₄)alkylthio; halo; halo(C₁₋₄)alkoxy; halo(C₁₋₄)alkyl-, (C₁₋₄ alkyl; (C₂₋₄)alkenyl; hydroxy; hydroxy(C₁₋₄)alkyl; mercapto(C₁₋₄)alkyl; (C₁₋₄)alkoxy; nitro; cyano; carboxy; amino or aminocarbonyl optionally substituted by (C₁₋₄)alkyl; (C₁₋₄)alkylsulphonyl; (C₂₋₄)alkenylsulphonyl.

The term "acyl" includes formyl and (C₁₋₄)alkylcarbonyl group.

Some of the compounds of this invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

Since the compounds of formula (I) are intended for use in pharmaceutical compositions it will readily be understood that they are each provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions; these less pure preparations of the compounds should contain at least 1%, more suitably at least 5% and preferably from 10 to 59% of a compound of the formula (I) or pharmaceutically acceptable salt thereof.

Pharmaceutically acceptable salts of the above-mentioned compounds of formula (I) include their acid addition or quaternary ammonium salts, for example their salts with mineral acids e.g. hydrochloric, hydrobromic, sulphuric nitric or phosphoric acids, or organic acids, e.g. acetic, fumaric, succinic, maleic, citric, benzoic, p-toluenesulphonic, methanesulphonic, naphthalenesulphonic acid or tartaric acids.

Certain of the compounds of formula (I) may exist in the form of optical isomers, e.g. diastereoisomers and mixtures of isomers in all ratios, e.g. racemic mixtures. The invention includes all such forms, in particular the pure isomeric forms. For example the invention includes compound in which an A-B group CH(OH)-CH₂ is in either isomeric configuration, the R-isomer is preferred. The different isomeric forms may be separated or resolved one from the other by conventional methods, or any given isomer may be obtained by conventional synthetic methods or by stereospecific or asymmetric syntheses.

The compounds of the present invention were prepared by the methods illustrated in Schemes I, II, and VIII.

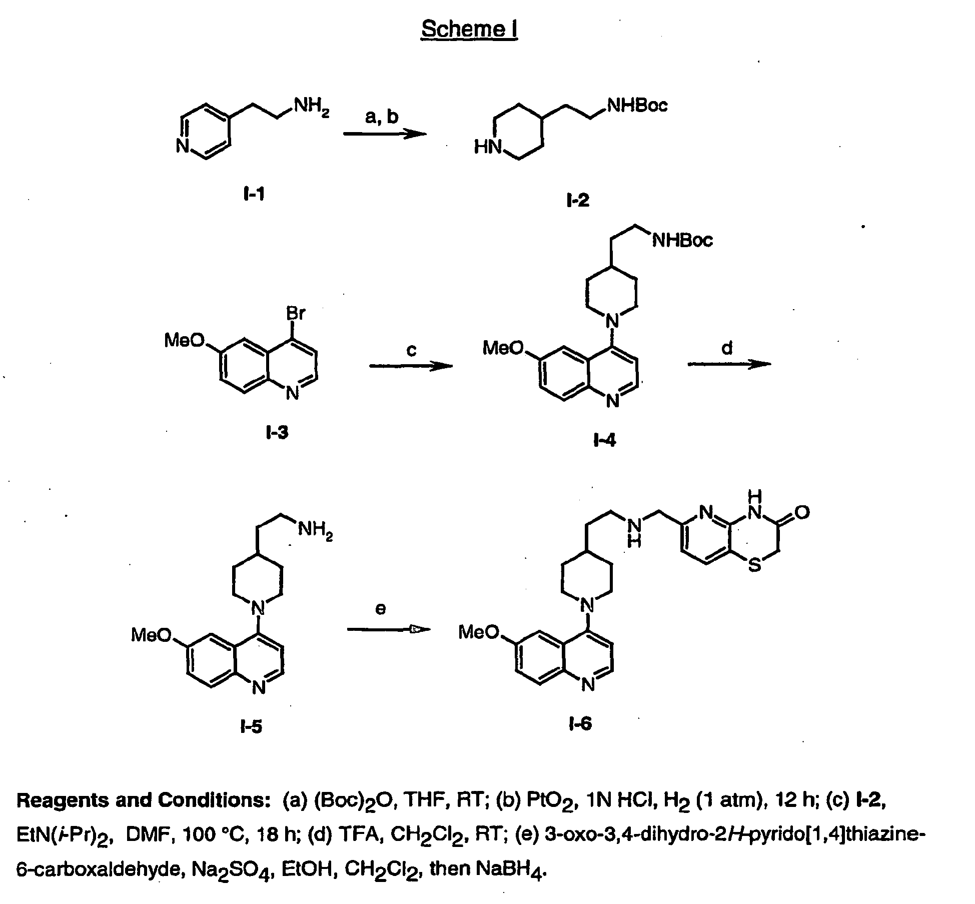

Pyridine (**I-1**) is reacted with di-*tert*-butyl dicarbonate or a similar commercially available Boc-reagent to afford **I-2.** The use of protecting groups to mask reactive functionality is well-known to those of skill in the art, and other protecting groups are listed in standard reference volumes, such as Greene, "Protective Groups in Organic Synthesis" (published by Wiley-Interscience).

Hydrogenation of the pyridine moiety under acidic conditions using an appropriate catalyst, such as Pt₂O, or others listed in standard reference books such as Rylander, "Hydrogenation Methods" (published by Academic Press) provides the piperidine **I-2.** Reaction of the piperidine **I-2** with an appropriately substituted pyridine electrophile **I-3** under thermal conditions provides compound **1-4.** See (J. Med. Chem. 2002, 45, 4975) for similar reaction examples. Removal of the Boc protecting group is carried out under standard acidic conditions to give the free amine **I-5.** The primary amine derivative is then converted to a secondary amine **I-6** by reaction with an aldehyde and a suitable reducing agent.

For example, 2-[1-(6-methoxyquinolin-4-yl)piperidin-4-yl]ethylamine is converted to an imine by reaction with an aldehyde in protic or aprotic solvents such as DMF, CH₂Cl₂, EtOH or CH₃CN. The imine is subsequently or simultaneously reacted with a suitable reducing agent such as NaBH₄, NaBH(OAc)₃ or NaBH₃CN in solvent. Depending on whether acid neutralization is required, an added base, such as triethylamine (Et₃N), diisopropylethylamine ((i-Pr)₂NEt), or K₂CO₃, may be used. Many additional methods for reductive aminations are known, and can be found in standard reference books, such as "Compendium of Organic Synthetic Methods", Vol. I - VI (published by Wiley-Interscience).

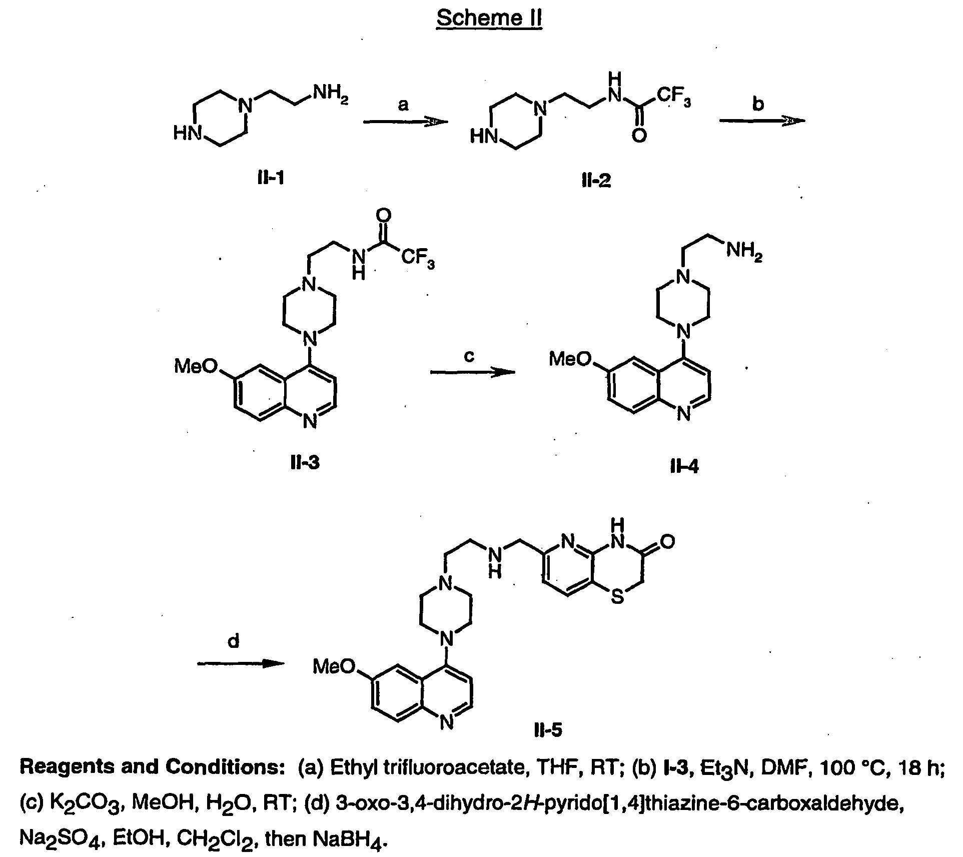

Piperazine **(II**-**1**) is reacted with ethyl trifluoroacetate or a similar commercially available acylating reagent to afford (**II-2**). The use of protecting groups to mask reactive functionality is well-known to those of skill in the art, and other protecting groups are listed in standard reference volumes, such as Greene; "Protective Groups in Organic Synthesis" (published by Wiley-Interscience).

Reaction of the piperazine **(II-2)** with an appropriately substituted pyridine electrophile **(1-3)** under thermal conditions provides compound **(II-3).** See (J. Med. Chem. 2002, 45, 4975) for similar reaction examples. Removal of the acyl protecting group is carried out under standard saponification conditions to give the free amine **(II-4).** The primary amine derivative is then converted to a secondary amine **(II-5)** by reaction with an aldehyde and a suitable reducing agent.

For example, 2-[4-(6-methoxyquinolin-4-yl)piperazin-1-yl]ethylamine is converted to an imine by reaction with an aldehyde in protic or aprotic solvents such as DMF, CH₂Cl₂, EtOH or CH₃CN. The imine is subsequently or simultaneously reacted with a suitable reducing agent such as NaBH₄, NaBH(OAc)₃ or NaBH₃CN in solvent. Depending on whether acid neutralization is required, an added base, such as triethylamine (Et₃N), diisopropylethylamine ((i-Pr)₂NEt), or K₂CO₃, may be used. Many additional methods for reductive aminations are known, and can be found in standard reference books, such as "Compendium of Organic Synthetic Methods", Vol. I - VI (published by Wiley-Interscience).

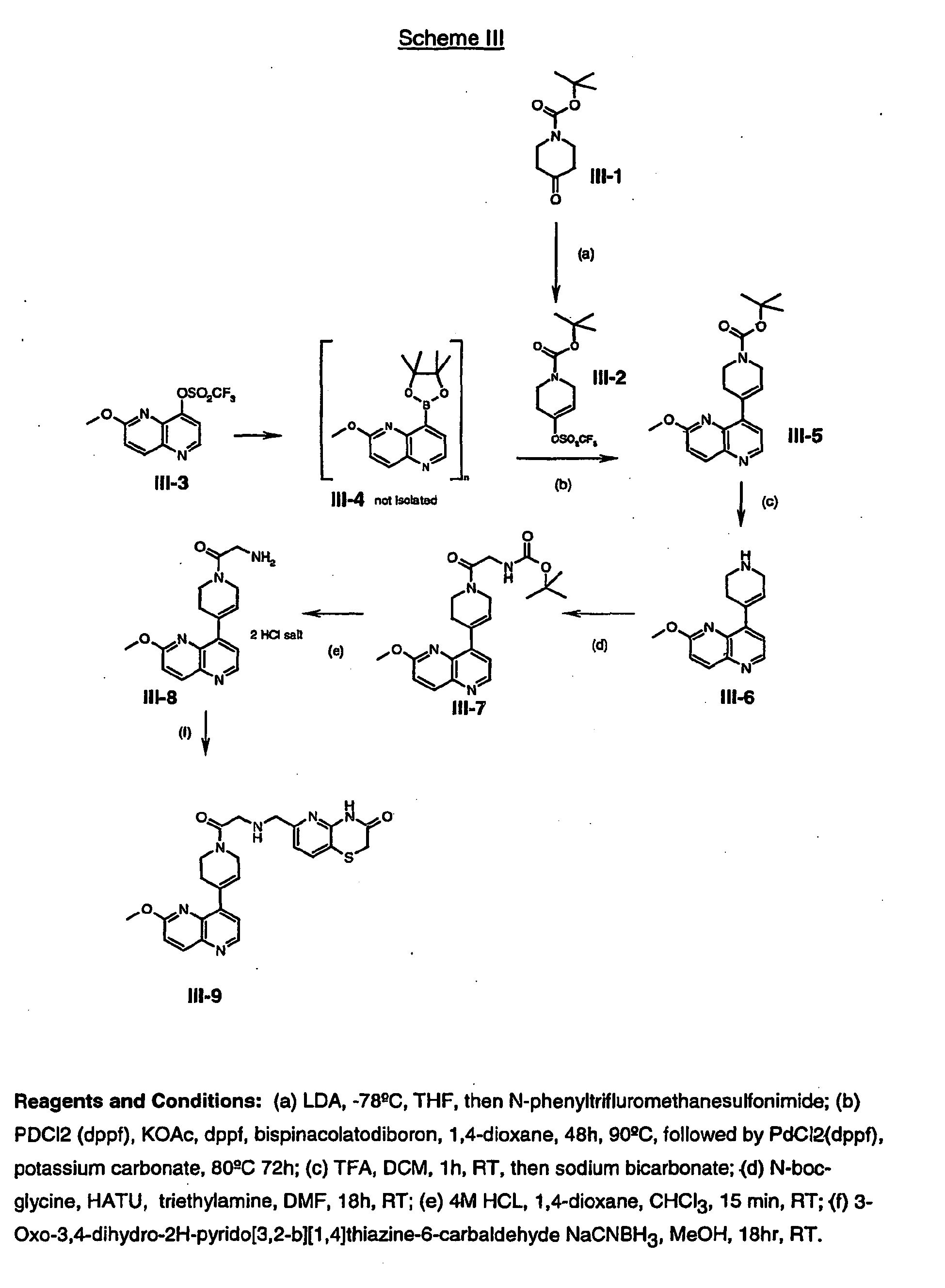

Ketone (**III-1**) is reacted with LDA and then with N-phenyltrifluoromethane sulfonimide to give triflate **(I-2)** see (Synthesis 1991, 993). Triflate (III-3) is reacted under palladium catalysis to provide the crude intermediate **(III-4)** which is further reacted with **(III-2)** to give **(III-5),** see (Tetrahedron Letters 1997, 38 3447).

Removal of the Boc protecting group is carried out under standard acidic conditions to give the free amine **(III-6).** The amine derivative is then coupled with N-Boc-glycine under standard HATU coupling conditions to give **(III-7).**

Removal of the Boc protecting group is carried out under standard acidic conditions to give the free amine **(III-8).** The primary amine derivative is then converted to a secondary amine **(III-9)** by reaction with an aldehyde and a suitable reducing agent. For example, 2-Amino-1-[4-(6-methoxy-[1,5]naphthyridin-4-yl)-3,6-dihydro-2H -pyridin-1-yl]-ethanone is converted to an imine by reaction with an aldehyde in protic or aprotic solvents such as DMF, CH₂Cl₂, EtOH or CH₃CN.

The imine is subsequently or simultaneously reacted with a suitable reducing agent such as NaBH₄, NaBH(OAc)₃ or NaBH₃CN in solvent. Depending on whether acid neutralization is required, an added base, such as triethylamine (Et₃N), diisopropylethylamine ((i-Pr)₂NEt), or K₂CO₃, may be used. Many additional methods for reductive aminations are known, and can be found in standard reference books, such as "Compendium of Organic Synthetic Methods", Vol. I - VI (published by Wiley-Interscience).

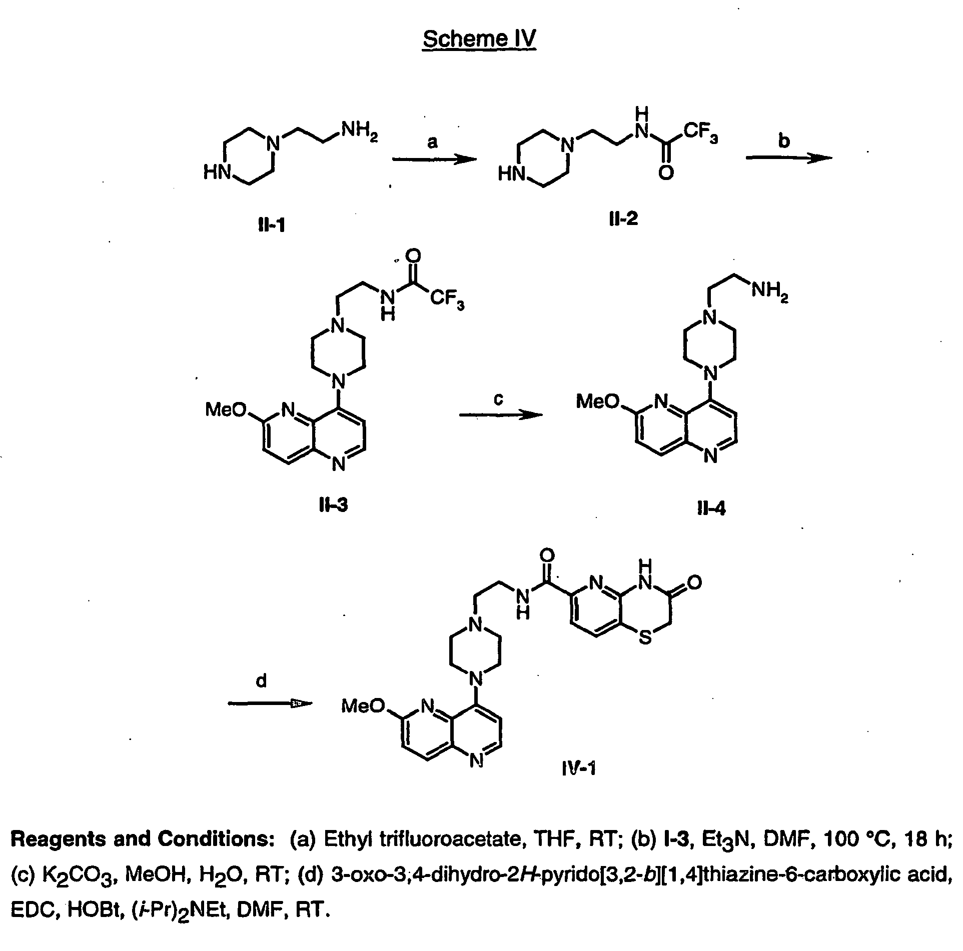

Piperazine **(Ii-1)** is reacted with ethyl trifluoroacetate or a similar commercially available acylating reagent to afford **(II-2).** The use of protecting groups to mask reactive functionality is well-known to those of skill in the art, and other protecting groups are listed in standard reference volumes, such as Greene, "Protective Groups in Organic Synthesis" (published by Wiley-Interscience).

Reaction of the piperazine **(II-2)** with an appropriately substituted pyridine electrophile **(I-3)** under thermal conditions provides compound **(II-3).** See (J. Med. Chem. 2002, 45, 4975) for similar reaction examples. Removal of the acyl protecting group is carried out under standard saponification conditions to give the free amine **(II-4).** The primary amine derivative is then converted to a carboxamide **(IV-1)** by reaction with an acid and a suitable peptide-coupling agent.

For example, 2-[4-(6-methoxyquinolin-4-yl)piperazin-1-yl]ethylamine is converted to an amide by reaction with an acid in aprotic solvents such as DMF, CH₂Cl₂, or CH₃CN. Depending on whether acid neutralization is required, an added base, such as triethylamine (Et₃N), diisopropylethylamine ((i-Pr)₂NEt), or K₂CO₃, may be used. The active coupling reagent (DCC, EDC, BOP-Cl) is present in situ with the amine and acid reactants. Many additional methods for peptide couplings are known, and can be found in standard reference books.

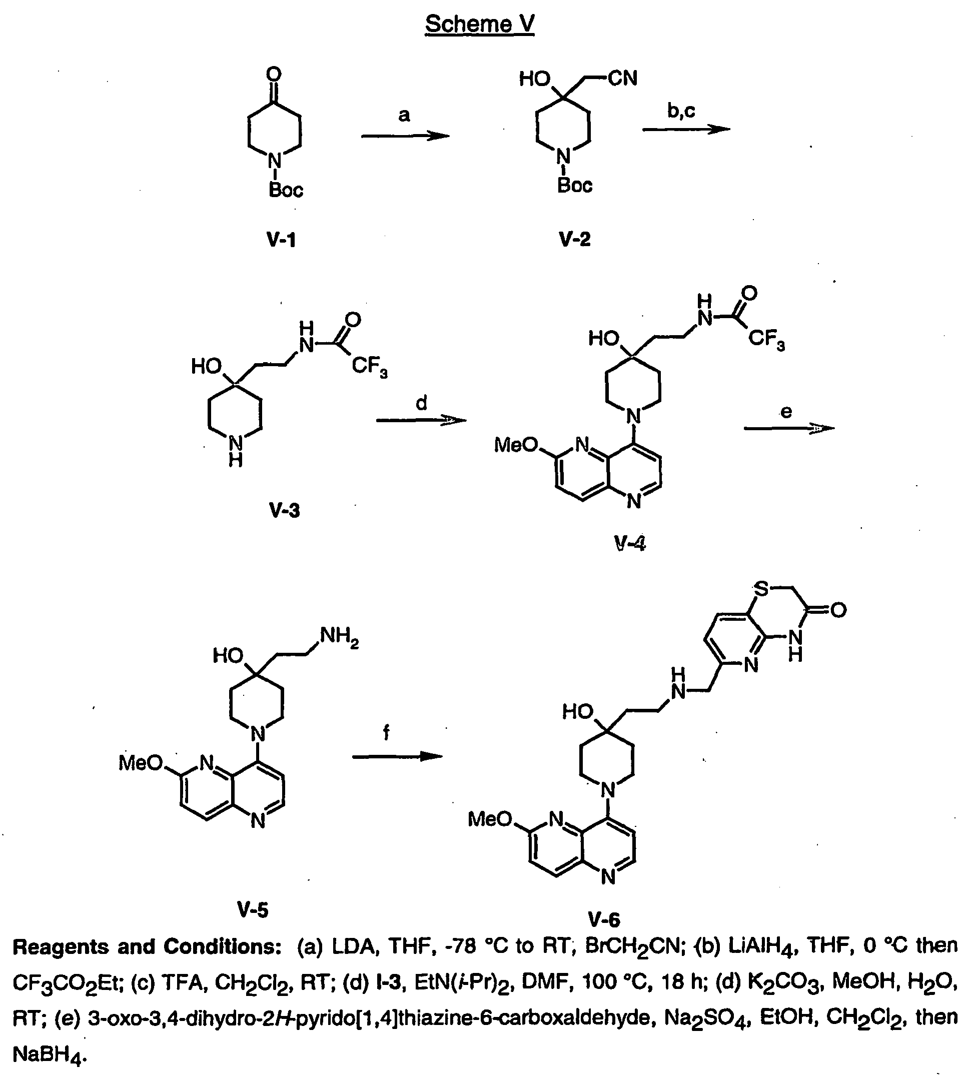

Pyridone **(V-1)** is reacted with the lithium anion formed from bromoacetonitrile and butyllithium to form the alkylated product **(V-2).** The generation of lithium nucleophiles for the purpose of alkylation is well known to those skilled in the art.

The nitrile **(V-2)** is reduced with a hydride reagent such as LiAlH4 or other similar commercially available reagent to afford a primary amine product.

This product is subsequently protected as a trifluoromethyl amide to give **(V-3).** The use of protecting groups to mask reactive functionality is well-known to those of skill in the art, and other protecting groups are listed in standard reference volumes, such as Greene, protective Groups in Organic Synthesis" (published by Wiley-Interscience).

Reaction of the piperidine **(V-3)** with an appropriately substituted pyridine electrophile such as **(I-3)**, under thermal conditions provides compound **(V-4).** See (J. Med. Chem. 2002, 45, 4975) for similar reaction examples. Removal of the trifluoroacetamide group under basic aqueous conditions provides the primary amine (V-5). The primary amine derivative is then converted to a secondary amine **(V-6)** by reaction with an aldehyde and a suitable reducing agent.

For example, 2-[1-(6-methoxyquinolin-4-yl)piperidin-4-yl]ethylamine is converted to an imine by reaction with an aldehyde in protic or aprotic solvents such as DMF, CH₂Cl₂, EtOH or CH₃CN. The imine is subsequently or simultaneously reacted with a suitable reducing agent such as NaBH₄, NaBH(OAc)₃ or NaBH₃CN in solvent. Depending on whether acid neutralization is required, an added base, such as triethylamine (Et₃N), diisopropylethylamine ((i-Pr)₂NEt), or K₂CO₃, may be used. Many additional methods for reductive aminations are known, and can be found in standard reference books, such as "Compendium of Organic Synthetic Methods", Vol.1 - VI (published by Wiley-Interscience).

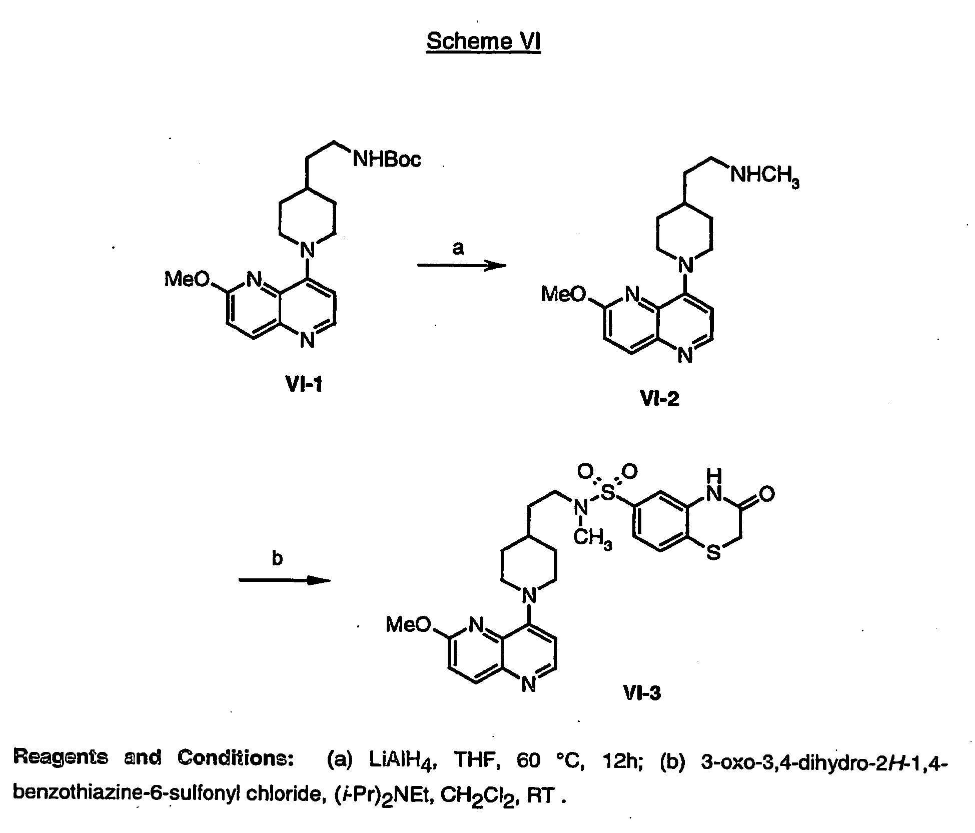

The Boc-protected primary amine **(VI-1)** is converted to a secondary N-methyl amine by heating with LiAIH₄ in THF or in another suitable polar aprotic solvent.

The secondary amine **(VI-2)** was then converted to a sulfonamide by reaction with a sulfonyl chloride compound in the presence of an acid scavenger reagent, such as i-Pr₂NEt or Et₃N, to give the product **(VI-3).**

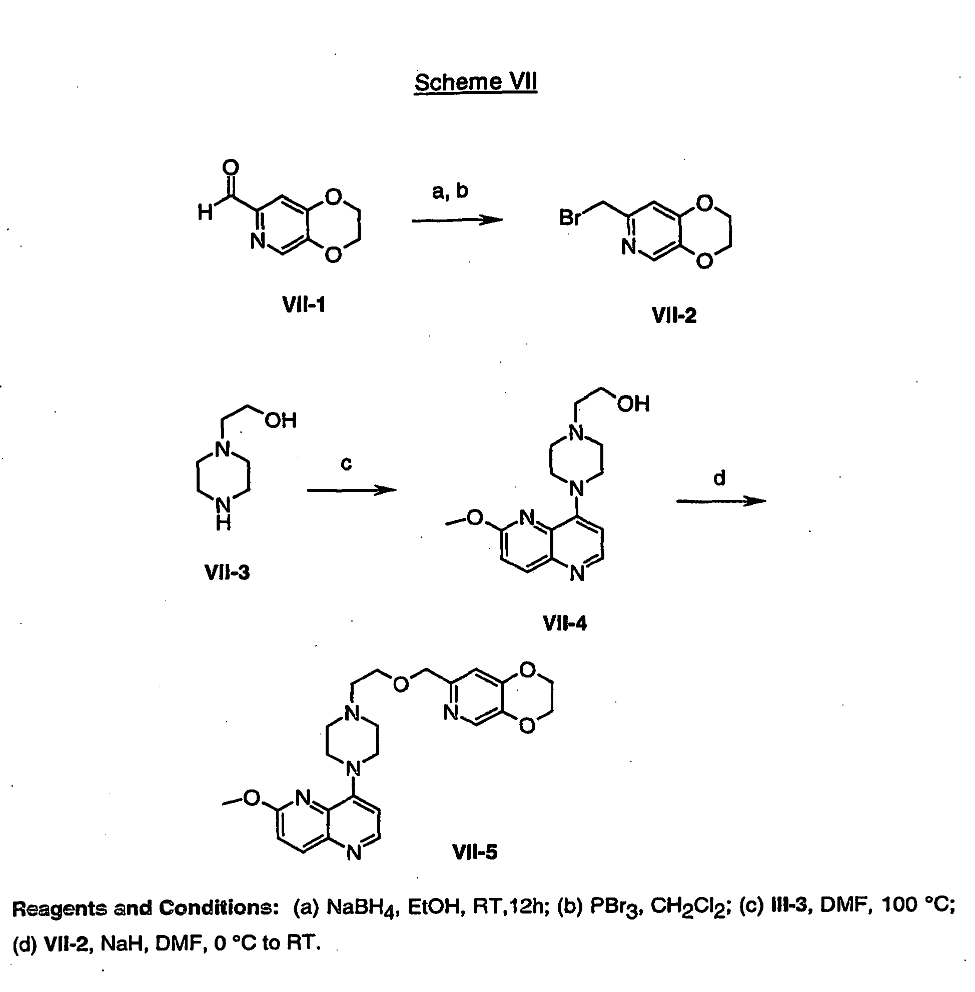

Aldehyde **(VII-1)** was reduced with a hydride reducing agent such as NaBH₄ to provide the corresponding alcohol. The alcohol is then converted to a bromide using a brominating reagent (PBr₃, HBr, etc.) to afford compound **(VII-2).**

The piperazine alcohol **(VII-3)** was reacted under thermal conditions with a naphthyridine electrophile to give product **(VII-4).** The alcohol **(VII-4)** was then fully deprotonated using a strong base, such as NaH, and subsequently reacted with bromide **(VII-2)** in a polar aprotic solvent to afford the ether **(VII-5).**

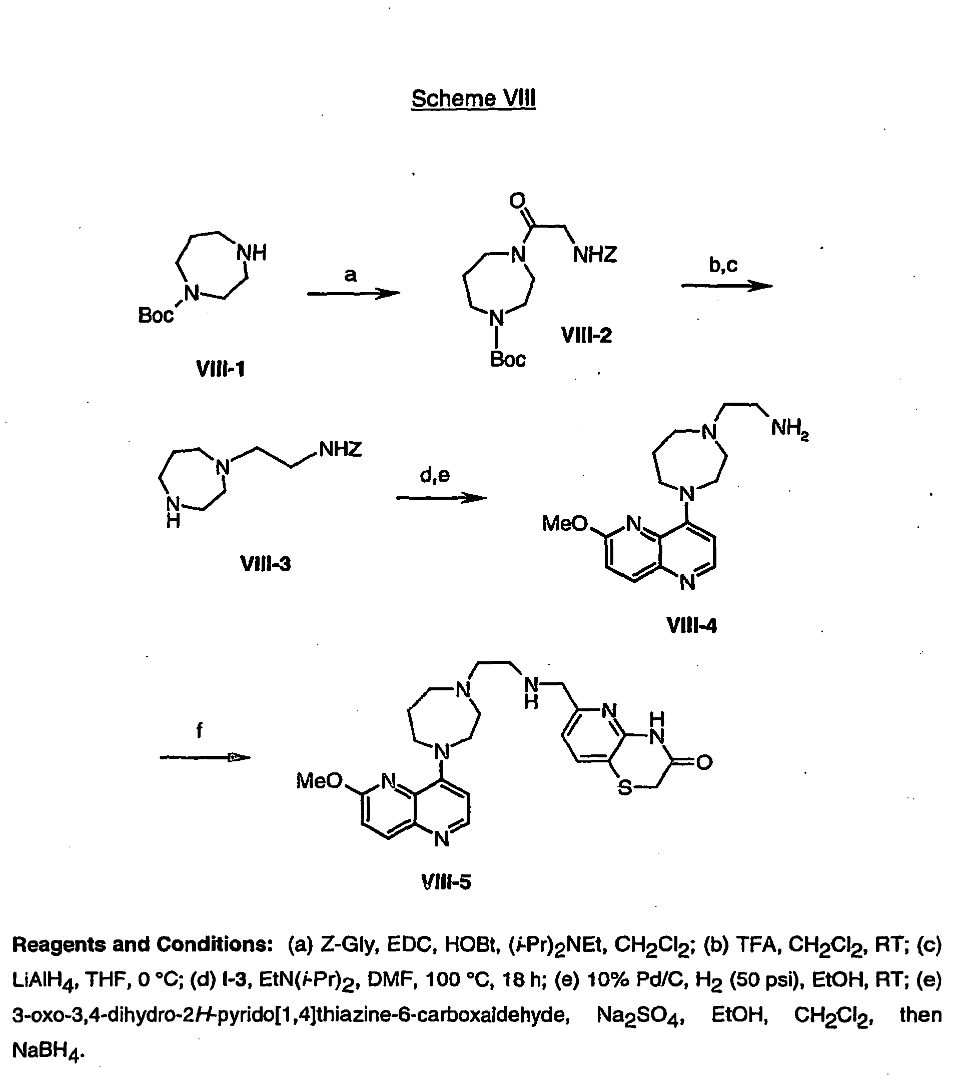

The secondary amine derivative **(VIII-1)** is then converted to a carboxamide **(VIII-2)** by reaction with an acid and a suitable peptide-coupling agent. For example, Z-Glycine is converted to an amide by reaction with an amine in aprotic solvents such as DMF, CH₂Cl₂, or CH₃CN. Depending on whether acid neutralization is required, an added base, such as triethylamine (Et₃N), diisopropylethylamine ((i-Pr)₂NEt), or K₂CO₃, may be used.

The active coupling reagent (DCC, EDC, BOP-Cl) is present in situ with the amine and acid reactants. Many additional methods for peptide couplings are known, and can be found in standard reference books. The Boc group of **(VIII-2)** is removed under acidic conditions using TFA. The amide functionality is reduced with a hydride reagent, such as LiAIH₄, to give the secondary amine **(VIII-3).** The amine is then heated together with a suitably reactive electrophilic heterocycle to afford the naphthyridine compound **(VIII-4).**

The primary amine derivative **(VIII-4)** is then converted to a secondary amine **(VIII-5)** by reaction with an aldehyde and a suitable reducing agent. For example, amine **(VIII-4)** is converted to an imine by reaction with 3-oxo-3,4-dihydro-2*H*-pyrido[1,4]thiazine-6-carboxaldehyde in protic or aprotic solvents such as DMF, CH₂Cl₂, EtOH or CH₃CN. The imine is subsequently or simultaneously reacted with a suitable reducing agent such as NaBH₄, NaBH(OAc)₃ or NaBH₃CN in solvent to give **(VIII-5)**. Depending on whether acid neutralization is required, an added base, such as triethylamine (Et₃N), diisopropylethylamine ((i-Pr)₂NEt), or K₂CO₃, may be used. Many additional methods for reductive aminations are known, and can be found in standard reference books, such as "Compendium of Organic Synthetic Methods", Vol. I - VI (published by Wiley-Interscience).

The antibacterial compounds according to the invention may be formulated for administration in any convenient way for use in human or veterinary medicine, by analogy with other antibacterials.

The pharmaceutical compositions of the invention include those in a form adapted for oral, topical or parenteral use and may be used for the treatment of bacterial infection in mammals including humans.

The composition may be formulated for administration by any route. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl *p*-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

No toxicological effects are indicated when a compound of formula (I) or a pharmaceutically acceptable derivative thereof is administered in the above-mentioned dosage range.

The compound of formula (I) may be the sole therapeutic agent in the compositions of the invention or a combination with other antibacterials. If the other antibacterial is a β-lactam then a β-lactamase inhibitor may also be employed.

Compounds of formula (I) are active against a wide range of organisms including both Gram-negative and Gram-positive organisms.

All publications, including but not limited to patents and patent applications, cited in this specification are herein incorporated by reference as if each individual publication were specifically and individually indicated to be incorporated by reference herein as though fully set forth.

The following examples illustrate the preparation of certain compounds of formula (I) and the activity of certain compounds of formula (I) against various bacterial organisms.

Abbreviations in the examples, include:
RT = room temperature;
ES = Electrospray mass spec;
LCMS = Liquid chromatography mass spec; and
APCI+ = Atmospheric pressure chemical ionisation mass spec.

Certain reagents also are abbreviated herein. DCC refers to dicyclohexylcarbodiimide, DMAP refers to dimethylaminopyridine, DIEA refers to diisopropylethyl amine, EDC refers to 1-(3-dimethylaminopropyl)-3-ethylcarbodiimide, hydrochloride. HOBt refers to 1-hydroxybenzotriazole, THF refers to tetrahydrofuran, DIEA refers to diisopropylethylamine, DEAD refers to diethyl azodicarboxylate, PPh₃ refers to triphenylphosphine, DIAD refers to diisopropyl azodicarboxylate, DME refers to dimethoxyethane, DMF refers to dimethylformamide, NBS refers to N-bromosuccinimide, Pd/C refers to a palladium on carbon catalyst, PPA refers to polyphosphoric acid, DPPA refers to diphenylphosphoryl azide, BOP refers to benzotriazol-1-yloxy-tris(dimethylamino)phosphonium hexafluorophosphate, HF refers to hydrofluoric acid, TEA refers to triethylamine, TFA refers to trifluoroacetic acid, PCC refers to pyridinium chlorochromate.

### EXAMPLES

Standard spectroscopic, characterization or identification techniques as conventionally known in the art were used to analyze, identify or characterize the compounds of the present invention.

Proton nuclear magnetic resonance (¹H NMR) spectra were recorded at 300 MHz, and chemical shifts are reported in parts per million (δ) downfield from the internal standard tetramethylsilane (TMS). Abbreviations for NMR data are as follows: s = singlet, d = doublet, t = triplet, q = quartet, m = multiplet, dd = doublet of doublets, dt = doublet of triplets, app = apparent, br = broad. J indicates the NMR coupling constant measured in Hertz. CDCl₃ is deuteriochloroform, DMSO-d₆ is hexadeuteriodimethylsulfoxide, and CD₃OD or d₄-CH₃OH is tetradeuteriomethanol.

Mass spectra were obtained using electrospray (ES) ionization techniques. Elemental analyses were performed by Quantitative Technologies Inc., Whitehouse, NJ.

Melting points were obtained on a Thomas-Hoover melting point apparatus and are uncorrected.

All temperatures are reported in degrees Celsius. E. Merck Silica Gel 60 F-254 thin layer plates were used for thin layer chromatography. Flash chromatography was carried out on E. Merck Kieselgel 60 (230-400 mesh) silica gel. Analytical HPLC was performed on Beckman chromatography systems. Preparative HPLC was performed using Gilson chromatography systems. ODS refers to an octadecylsilyl derivatized silica gel chromatographic support. YMC ODS-AQ® is an ODS chromatographic support and is a registered trademark of YMC Co. Ltd., Kyoto, Japan. PRP-1 ® is a polymeric (styrenedivinylbenzene) chromatographic support, and is a registered trademark of Hamilton Co., Reno, Nevada. Celite® is a filter aid composed of acid-washed diatomaceous silica, and is a registered trademark of Manville Corp., Denver, Colorado.

### Preparation 1

### Preparation of (2-Piperidin-4-ylethyl) carbamic acid tert-butyl ester

### a) (2-Pyridin-4-ylethyl) carbamic acid tert-butyl ester

To solution of 4-(2-aminoethyl)piperidine (10.0 g, 81.8 mmole) in THF at RT was added di-*tert*-butyl dicarbonate (17.9 g, 81.8 mmole). After 1 hr, the reaction solution was concentrated and purified on silica (EtOAc) to give the title compound as a colorless oil (18.0 g, 99%): LC-MS (ES) m/e 223 (M + H)⁺.

### b) (2-Piperidin-4-ylethyl) carbamic acid tert-butyl ester

To solution of (2-pyridin-4-ylethyl) carbamic acid *tert*-butyl ester (18.0 g, 81.0 mmole) in MeOH (250 mL) at RT was added 6N HCl (13.6 mL) and PtO₂ (900 mg). After 18 hr under a balloon of H₂ with vigorous stirring, the reaction solution was filtered through Celite® and concentrated under vacuum. The residue was dissolved in CH₂Cl₂ and washed with saturated NaHCO₃. The organic phase was dried over Na₂SO₄ and concentrated to give the title compound as a colorless viscous oil (17.6 g, 95%): LC-MS (ES) m/e 229 (M + H)⁺.

### Preparation 2

### Preparation of 1,1,1-trifluoro-N-(2-piperazin-1-ylethyl)acetamide

To solution of 1-(2-aminoethyl)piperazine (8.0 g, 61.9 mmole) in THF (100 mL) at 0 °C was added ethyl trifluoroacetate (7.38 mL, 61.9 mmole). The reaction solution was allowed to warm to RT over 2 hr and then was concentrated to give the title compound as a pale yellow solid (13.9 g, 99%). ¹H NMR (400 MHz, CDCl₃) δ 7.23(s, 1 H), 3.40 ( q, *J*= 5 Hz, 2H), 2.88 ( t, *J* = 5 and 4.8 Hz, 4 H), 2.53 (t, *J* = 6 and 5.8 Hz, 2H), 2.44 (s, 4H), and 1.86 (s, 1H). LC-MS (ES) m/e 226 (M + H)⁺.

### Preparation 3

### Preparation of 2,2,2-trifluoro-N-[2-(4-hydroxy-4-piperidinynethyl)acetamide

### a) 4-Cyanomethyl-4-hydroxy-piperidine-1-carboxylic acid tert-butyl ester

To an oven-dried flask equipped with a stirring bar and rubber septum was added anhydrous THF (250 mL). Diisopropylamine (7.0 mL, 50.2 mmole) was added and the solution cooled to -78 °C. To the cooled solution was added n-butyllithium (1.8 M in hexanes, 31 mL, 50.2 mmole) over 10 minutes. The reaction mixture was stirred for 60 min and *N*-Boc-4-piperidone (10.0 g, 50.2 mmole, in 10 mL of *anhydrous* THF) bromoacetonitrile (4.2 mL, 60.2 mmole) and HMPA (20 mL) was added and the mixture stirred for an additional 2 h at RT. The reaction mixture was quenched with brine and concentrated in vacuo. The residue was taken up in ethyl acetate (500 mL) and washed with sat. NaHCO₃ (2 x 200 mL). The organic layer was dried over sodium sulfate and concentrated in vacuo to obtain the title compound (7.0g, 58%) as a yellow oil: ¹HNMR (400 MHz, CDCl₃) δ 3.90 Hz (bs, 2H), 3.16 Hz ( m, 2H), 2.55 Hz (s, 2H), 1.74 Hz ( m, 2H), 1.50 ( m, 2H), and 1.47 (s, 9H). LC-MS (ES) m/e 241.2 (M + H).

### b) 4-Hydroxy-4-[2-(2,2,2-trifluoro-ethanoylamino)-ethyl]-piperidine-1-carboxylic acid tert-butyl ester.

To a round bottom flask equipped with a stirring bar was added 4-cyanomethyl-4-hydroxy-piperidine-1-carboxylic acid tert-butyl ester (2.9 g, 0.012 mol) in anhydrous THF (150 mL). The reaction mixture was placed under a stream of nitrogen, cooled to 0 °C, and lithium aluminum hydride (0.013 mol, 0.504 g) was added. After 2 h, the reaction was quenched by the sequential addition of water (13 mL), 15% aq. NaOH (42 mL), and water (13 mL). The reaction mixture was filtered through a cake of Celite and concentrated to a yellow oil. The oil was immediately dissolved in THF (100 mL) and cooled to 0 °C. Ethyl trifluoroacetate (0.0144 mol, 1.7 mL) was added to the reaction mixture. The reaction mixture was stirred at RT for 96 h and then concentrated in vacuo to yield the title compound (1.6 g, 39%) as a yellow foam: ¹H NMR (400 MHz, CDCl₃) δ 3.80 Hz (m, 4H), 3.57 Hz (m, 1 H), 3.11 (m, 4H), 1.87 Hz (m, 2H), 1.78 Hz (m, 1 H), 1.62 (m, 2H), and 1.47 (s, 9H). LC-MS (ES) m/e 241.2 (M + H - Boc ).

### c) 2,2,2-trifluoro-N-[2-(4-hydroxy-4-piperidinyl)ethyl]acetamide

To a round bottom flask equipped with a stirring bar was added 4-hydroxy-4-[2-(2,2,2-trifluoro-ethanoylamino)-ethyl]-piperidine-1-carboxylic acid tert-butyl ester (1.6 g, 47.0 mmole) in 50% TFA/dichloromethane (100 mL). The reaction mixture was placed under a stream of nitrogen and stirred for 2 h. The reaction was concentrated to a brown oil and used directly. LC-MS (ES) m/e 241 (M+H)⁺.

### Example 1

### Preparation of 6-({2-[-1-(6-methoxyquinolin-4-yl)piperidin-4-yl]ethylamino}methyl)-4H-pyrido[3,2-b][1,4]thiazin-3-one

### a) {2-[1-(6-methoxyquinolin-4-yl)piperidin)-4-yl]ethyl}carbamic acid tert-butyl ester

To a solution of (2-piperidin-4-ylethyl) carbamic acid *tert*-butyl ester (2.1 g, 9.2 mmole) in DMF (5 mL) at RT was added 4-bromo-6-methoxyquinoline (2.0 g, 8.4 mmole) and Et₃N (0.86 g, 8.37 mmole). After 18 hour at 100 °C, the reaction solution was concentrated under vacuum and purified by flash chromatography on silica gel (CHCl₃/MeOH containing 5% NH₄0H, 9:1) to afford the title compound as a tan solid (2.39 g, 74%): ¹H NMR (400 MHz, CDCl₃) 8.61 (m, 1 H), 8.03 (m, 1H), 7.37 (m, 1 H), 7.22 (m, 1H), 6.85 (m, 1 H), 4.57 (br s, 1H), 3.98 (s, 3H), 3.72 (m, 1 H), 3.25 (m, 1H), 2.99 (app s, 2H), 2.90 (app s, 2H), 2.80 (m, 2H), 1.95 (m, 1H), 1.65-1.50 (m, 4H), 1.48 (s, 9H). LC-MS (ES) m/e 386 (M + H)⁺

### b) 2-[1-(6-methoxyquinolin-4-yl)piperidin-4-yl]ethylamine

To a solution of {2-[1-(6-methoxyquinolin-4-yl)piperidin-4-yl]ethyl}carbamic acid *tert*-butyl ester (2.39 g, 6.20 mmole) in CH₂Cl₂ at RT was added TFA (1:1, v/v). After 2 hrs, the solution was concentrated to dryness under vacuum and the residue redissolved in CH₂Cl₂/ MeOH (9:1, v/v). The solution was washed with saturated aqueous NaHCO₃ solution, dried over Na₂SO₄, and concentrated under vacuum to give the title compound (1.62 g, 92%) as a waxy yellow solid: LC-MS (ES) m/e 286 (M + H)⁺.

### c) 6-({2-[1-(6-methoxyquinolin-4-yl)piperidin-4-yl]ethylamino}methyl)-4H-pyrido [3,2-b][1,4]thiazin-3-one

To a solution of 2-[1-(6-methoxyquinolin-4-yl)piperidin-4-yl]ethylamine (0.15 g, 0.53 mmole) in CH₂Cl₂ (25 mL) and EtOH (25 mL) was added Na₂SO₄ (50 mg) and 3-oxo-3,4-dihydro-2*H*-pyrido[1,4]thiazine-6-carboxaldehyde (0.11 g, 0.55 mmole). After 12 hr at RT, NaBH₄ (21 mg, 0.55 mmole) was added and the reaction solution was allowed to stir overnight. Silica gel (-5 g) was added to the reaction solution and the contents were concentrated under vacuum. The silica-adsorbed reaction contents were added directly to a silica gel column (CHCl₃/MeOH containing 5% NH₄OH, 9:1) to give the title compound (0.21 g, 86 %) as an off-white solid: ¹H NMR (400 MHz, d₄-MeOH) 8.47 (d, *J*= 6.5 Hz, 1H), 7.91 (d, *J*= 9.3 Hz, 1H), 7.83 (d, *J*= 7.8 Hz, 1H), 7.61 (d, *J*= 9.3 Hz, 1 H), 7.38 (d, *J* = 2.4 Hz, 1 H), 7.17 (m, 2H), 4.35 (s, 2H), 4.14 (m, 2H), 4.01 (s, 3H), 3.58 (s, 2H), 3.33 (m, 1 H), 3.27 (m, 2H), 2.05 (m, 2H), 1.84 (m, 4H), 1.63 (m, 2H). LC-MS (ES) m/e 464 (M + H)⁺.

### Example 2

### Preparation of 6-({2-[1-(6-methoxyquinolin-4-yl)piperidin-4-yl]ethylamino}methyl)-4H-pyrido[3,2-b][1,4]oxazin-3-one

According to the procedure of Example 1c, except substituting 3-oxo-3,4-dihydro-2*H*-pyrido[3,2-*b*][1,4]oxazine-6-carboxaldehyde (0.10 g, 0.55 mmole) for 3-oxo-3,4-dihydro-2*H*-pyrido[3,2-*b*][1,4]thiazine-6-carboxaldehyde, the title compound (0.19 g, 81 %) was prepared as an off-white solid following flash chromatography on silica gel (CHCl₃/MeOH, 9:1, containing 5% NH₄OH): ¹H NMR (400 MHz, d₄-MeOH) 8.46 (d, *J*= 6.5 Hz, 1 H), 7.93 (d, *J*= 9.3 Hz, 1H), 7.66 (d, *J*= 9.3 Hz, 1 H), 7.40 (m, 2H), 7.21 (d, *J*= 6.6 Hz, 1 H), 7.13 (d, *J*= 8.0 Hz, 2H), 4.72 (s, 2H), 4.29 (s, 2H), 4.23 (m, 2H), 4.02 (s, 3H), 3.45 (m, 2H), 3.21 (m, 2H), 2.06 (m, 2H), 1.86 (m, 4H), 1.66 (m, 2H). LC-MS (ES) m/e 448 (M + H)⁺.

### Example 3

### Preparation of (2,3-dihydro-[1,4]dioxino[2,3-c]pyridin-7-ylmethyl)-{2-[1-(6-methoxyquinolin-4-yl)piperidin-4-yl]ethyl}amine

According to the procedure of Example 1c, except substituting 2,3-dihydro[1,4]dioxino[2,3-*c*]pyridine-7-carboxaldehyde (0.25 g, 1.51 mmole) for 3-oxo-3,4-dihydro-2*H*-pyrido[3,2-*b*][1,4]thiazine-6-carboxaldehyde, the title compound (0.55 g, 84 %) was prepared as an off-white solid following flash chromatography on silica gel (CHCl₃/MeOH, 9:1, containing 5% NH₄OH): ¹H NMR (400 MHz, *d*₄-MeOH) 8.46 (m,2H), 7.92 (d, *J* = 9.3 Hz, 1 H), 7.66 (d, *J* = 9.3 Hz, 1 H), 7.52 (s, 1 H), 7.40 (d, *J*=2.5 Hz, 1 H), 7.21 (d, *J*= 6.9 Hz, 1 H), 4.60 (m, 2H), 4.50 (m, 2H), 4.49 (s, 2H), 4.26 (m, 2H), 4.02 (s, 3H), 3.45 (m, 2H), 3.33 (s, 2H), 3.30 (m, 2H), 2.08 (m, 2H), 1.95 (m, 1H), 1.86 (m, 2H), 1.66 (m, 2H). LC-MS (ES) m/e 435 (M + H)⁺.

### Example 4

### Preparation of 6-({2-[1-(6-methoxynaphthyridin-4-yl)piperidin-4-yl]ethylamino}methyl)-4H-pyrido[3,2-b][1,4]thiazin-3-one

### a) {2-[1-(6-methoxynaphthyridin-4-yl)piperidin-4-yl)ethyl}carbamic acid tert-butyl ester

To a solution of (2-piperidin-4-ylethyl) carbamic acid *tert*-butyl ester (0.81 g, 3.57 mmole) in DMF (5 mL) at RT was added 1,1,1-trifluoromethane sulfonic acid 6-methoxy[1,5]naphthyridin-4-yl ester (1.0 g, 3.24 mmole) and Et₃N (0.33 g, 3.24 mmole). After 18 hour at 100 °C, the reaction solution was concentrated under vacuum and purified by flash chromatography on silica gel (CHCl₃/MeOH containing 5% NH₄OH, 9:1) to afford the title compound as a tan solid (1.19 g, 95%): ¹H NMR (400 MHz, CDCl₃) 8.51 (d, *J* = 5.3 Hz, 1 H), 8.18 (d, *J* = 9.0 Hz, 1 H), 7.09 (d, *J* = 9.0 Hz, 1H), 6.85 (d, *J* = 5.3 Hz, 1 H), 4.55 (br s, 1H), 4.36 (m, 2H), 4.06 (s, 3H), 3.25 (m, 2H), 2.90 (m, 2H), 1.92 (m, 2H), 1.57 (m, 4H), 1.47 (s, 9H). LC-MS (ES) m/e 387 (M + H)⁺.

### b) 2-[1-(6-methoxynaphthyridin-4-yl)piperidin-4-ylmethylamine

To a solution of {2-[1-(6-methoxynaphthyridin-4-yl)piperidin-4-yl]ethyl}carbamic acid *tert*-butyl ester (1.19 g, 3.08 mmole) in CH₂Cl₂ at RT was added TFA (1:1, v/v). After 2 hrs, the solution was concentrated to dryness under vacuum and the residue redissolved in CH₂Cl₂/ MeOH (9:1, v/v). The solution was washed with saturated aqueous NaHCO₃ solution, dried over Na₂SO₄, and concentrated under vacuum to give the title compound (0.79 g, 90%) as a waxy yellow solid: LC-MS (ES) m/e 287 (M + H)⁺.

### c) 6-({2-[1-(6-methoxynaphthyridin-4-yl)piperidin-4-yl]ethylamino}methyl-4H-pyrido[3,2-b][1,4]thiazin-3-one

To a solution of 2-[1-(6-methoxynaphthyridin-4-yl)piperidin-4-yl]ethylamine (1.25 g, 4.37 mmole) in CH₂Cl₂ (70 mL) and EtOH (50 mL) was added Na₂SO₄ (100 mg) and 3-oxo-3,4-dihydro-2*H*-pyrido[1,4]thiazine-6-carboxaldehyde (0.89 g, 4.59 mmole). After 12 hr at RT, NaBH₄ (0.17 mg, 4.59 mmole) was added and the reaction solution was allowed to stir overnight. Silica gel (-5 g) was added to the reaction solution and the contents were concentrated under vacuum. The silica-adsorbed reaction contents were added directly to a silica gel column (CHCl₃/MeOH containing 5% NH₄OH, 9:1) to give the title compound (1.58 g, 78 %) as an off-white solid: ¹H NMR (400 MHz, CDCl₃) 8.51 (d, *J*= 5.4 Hz, 1 H), 8.21 (d, *J* = 9.0 Hz, 1H), 7.62 (d, *J* = 7.8 Hz, 1 H), 7.09 (d, *J* = 9.0 Hz, 1 H), 7.03 (d, *J*= 7.8 Hz, 1 H), 6.85 (d, *J*= 5.4 Hz, 2H), 4.37 (m, 2H), 4.06.(s, 3H), 3.94 (s, 2H), 3.50 (s, 2H), 2.94 (m, 4H), 1.61-1.92 (m, 7H). LC-MS (ES) m/e 465 (M + H)⁺.

### Example 5

### Preparation of 6-({2-[1-(6-methoxynaphthyridin-4-yl)piperidin-4-yl]ethylamino}methyl)-4H-pyrido[3,2-b][1,4]oxazin-3-one

According to the procedure of Example 4c, except substituting 3-oxo-3,4-dihydro-2*H*-pyrido[3,2-*b*][1,4]oxazine-6-carboxaldehyde (0.82 g, 4.59 mmole) for 3-oxo-3,4-dihydro-2*H*-pyrido[3,2-*b*][1,4]thiazine-6-carboxaldehyde, the title compound (1.37 g, 70 %) was prepared as an off-white solid following flash chromatography on silica gel (CHCl₃/MeOH, 9:1, containing 5% NH₄OH): ¹H NMR (400 MHz, CDCl₃) 8.53 (d, *J* = 5.4 Hz, 1H), 8.17 (d, *J* = 9.0 Hz, 1 H), 7.24 (d, *J*= 8.0 Hz, 1 H), 7.08 (d, *J* = 9.0 Hz, 1 H), 6.98 (d, *J*= 8.0 Hz, 1 H), 6.85 (d, *J*= 5.4 Hz, 2H), 4.67 (s, 2H), 4.35 (m, 2H), 4.06 (s, 3H), 3.87 (s, 2H), 2.88 (m, 2H), 2.76 (m, 2H), 1.87 (m, 2H), 1.64 (m, 5H). LC-MS (ES) m/e 449 (M + H)⁺.

### Example 6

### Preparation of (2,3-dihydro-[1,4]dioxino[12,3-c]pyridin-7-ylmethyl)-{2-[1-(6-methoxynaphthyridin-4-yl)piperidin-4-yl]ethyl}amine

According to the procedure of Example 4c, except substituting 2,3-dihydro[1,4] dioxino[2,3-*c*]pyridine-7-carboxaldehyde (0.25 g, 1.51 mmole) for 3-oxo-3,4-dihydro-2*H-*pyrido[3,2-*b*][1,4]thiazine-6-carboxaldehyde, the title compound (1.19 g, 79 %) was prepared as an off-white solid following flash chromatography on silica gel (CHCl₃/MeOH, 9:1, containing 5% NH₄OH): ¹H NMR (400 MHz, CDCl₃) 8.33 (d, *J* = 6.3 Hz, 1 H), 8.15 (m, 2H), 7.35 (d, *J*= 9.1 Hz, 1 H), 7.14 (d, *J*= 6.7 Hz, 1H), 7.03 (s, 1 H), 4.96 (m, 2H), 4.39 (m, 2H), 4.26 (m, 2H), 4.24 (s, 2H), 4.08 (s, 3H), 3.31 (m, 2H), 3.21 (s, 2H), 2.05 (m, 2H), 1.91 (m, 1 H), 1.79 (m, 2H), 1.61 (m, 2H). LC-MS (ES) m/e 436 (M + H)⁺.

### Example 7

### Preparation of 6-({2-[1-(3-chloro-6-methoxyquinolin-4-yl)piperidin-4-yl]ethylamino}methyl)-4H-pyrido[3,2-b][1,4]thiazin-3-one

### a) {2-[1-(3-chloro-6-methoxyquinolin-4-yl)piperidin-4-yl]ethyl}carbamic acid tert-butyl ester

To a solution of (2-piperidin-4-ylethyl) carbamic acid *tert*-butyl ester (1.20 g, 5.27 mmole) in DMF (5 mL) at RT was added 1,1,1-trifluoromethane sulfonic acid 3-chloro-6-methoxy[1,5]quinolin-4-yl ester (1.50 g, 4.39 mmole) and Et₃N (0.45 g, 4.39 mmole). After 18 hour at 100 °C, the reaction solution was concentrated under vacuum and purified by flash chromatography on silica gel (EtOAc) to afford the title compound as a tan solid (1.20 g, 65%): ¹H NMR (400 MHz, CDCl₃) 8.55 (m, 1H), 7.97 (d, *J*= 9.2 Hz, 1 H), 7.45 (m, 1H). 7.33 (m, 1 H), 4.51 (br s, 1 H), 3.97 (s, 3H), 3.46 (m, 2H), 3.33 (m, 3H), 3.25 (m, 2H), 1.87 (m, 2H), 1.60 (m, 4H), 1.48 (s, 9H). LC-MS (ES) m/e 420 (M + H)⁺.

### b) 2-[1-(3-chloro-6-methoxyquinolin-4-yl)piperidin-4-yl]ethylamine

To a solution of {2-[1-(3-chloro-6-methoxyquinolin-4-yl)piperidin-4-yl]ethyl}carbamic acid *tert*-butyl ester (1.19 g, 3.08 mmole) in CH₂Cl₂ (50 mL) at RT was added TFA (1:1, v/v). After 2 hrs, the solution was concentrated to dryness under vacuum and the residue redissolved in CH₂Cl₂/ MeOH (9:1, v/v). The solution was washed with saturated aqueous NaHCO₃ solution, dried over Na₂SO₄, and concentrated under vacuum to give the title compound (0.82 g, 90%) as a tan solid: LC-MS (ES) m/e 320 (M+ H)⁺.

### c) 6-({2-[1-3-chloro-6-methoxyquinolin-4-yl)piperidin-4-yl]ethylamino}methyl)-4H-pyrido[3,2-b][1,4]thiazin-3-one

To a solution of 2-[1-(3-chloro-6-methoxyquinolin-4-yl)piperidin-4-yl]ethylamine (0.40 g, 1.26 mmole) in CH₂Cl₂ (75 mL) and EtOH (50 mL) was added Na₂SO₄ (100 mg) and 3-oxo-3,4-dihydro-2*H*-pyrido[1,4]thiazine-6-carboxaldehyde (0.26 g, 1.32 mmole). After 12 hr at RT, NaBH₄ (21 mg, 0.55 mmole) was added and the reaction solution was allowed to stir overnight. Silica gel (-5 g) was added to the reaction solution and the contents were concentrated under vacuum. The silica-adsorbed reaction contents were added directly to a silica gel column (CHCl₃/MeOH containing 5% NH₄OH, 9:1) to give the title compound (0.50 g, 80 %) as an off-white solid: ¹H NMR (400 MHz, CDCl₃) 8.54 (s, 1 H), 7.94 (d, *J*= 9.1 Hz, 1 H), 7.64 (d, *J*= 7.8 Hz, 1 H), 7.46(m, 1 H), 7.32(m, 1 H), 7.05 (d, *J* = 7.8 Hz, 1 H), 4.01 (s, 2H), 3.97 (s, 3H), 3.50 (s,2H), 3.42 (m, 2H), 3.26 (m, 2H), 2.92 (m, 2H), 1.55-1.80 (m, 7H). LC-MS (ES) m/e 482 (M + H)⁺.

### Example 8

### Preparation of 6-({2-[1-(3-chloro-6-methoxyquinolin-4-yl)piperidin-4-yl]ethylamino}methyl)-4H-pyrido[3,2-b][1,4]oxazin-3-one

According to the procedure of Example 7c, except substituting 3-oxo-3,4-dihydro-2*H*-pyrido[3,2-*b*][1,4]oxazine-6-carboxaldehyde (0.23 g, 1.32 mmole) for 3-oxo-3,4-dihydro-2*H-*pyrido[3,2-*b*][1,4]thiazine-6-carboxaldehyde, the title compound (0.47 g, 78 %) was prepared as an off-white solid following flash chromatography on silica gel (CHCl₃/MeOH, 9:1, containing 5% NH₄OH): ¹H NMR (400 MHz, CDCl₃) 8.53 (s, 1H), 7.94 (d, *J* = 9.1 Hz, 1H), 7.46 (m, 1H), 7.28 (m, 2H), 7.06 (d, *J*= 8.1 Hz, 1 H), 4.68 (s, 2H), 4.04 (s, 2H), 3.96 (s, 3H), 3.46 (m, 2H), 3.25 (m, 2H), 2.94 (m,2H), 1.52-1.84 (m, 7H). LC-MS (ES) m/e 498 (M + H)⁺.

### Example 9

### Preparation of {2-[1-(3-chloro-6-methoxyquinolin-4-yl)piperidin-4-yl]ethyl}-(2,3-dihydro[1,4]dioxino[2,3-c]pyridin-7-ylmethyl)amine

According to the procedure of Example 7c, except substituting 2,3-dihydro[1,4]dioxino[2,3-*c*]pyridine-7-carboxaldehyde (0.14 g, 0.83 mmole) for 3-oxo-3,4-dihydro-2*H*-pyrido[3,2-*b*][1,4]thiazine-6-carboxaldehyde, the title compound (0.32 g, 83 %) was prepared as an off-white solid following flash chromatography on silica gel (CHCl₃/MeOH, 9:1, containing 5% NH₄OH): ¹H NMR (400 MHz, CD₃OD) 8.83 (s, 1H), 8.54 (s, 1H), 7.94 (d, *J* = 9.2 Hz, 1H), 7.70 (m, 1 H), 7.64 (s, 1 H), 7.53 (s, 1 H), 4.65 (m, 3H), 4.53 (m, 3H), 4.10 (m, 2H), 4.05 (s, 3H), 3.77 (m, 2H), 3.32 (m, 2H), 2.10 (m, 2H), 1.87 (m, 2H), 1.66 (m, 2H). LC-MS (ES) m/e 469 (M)⁺.

### Example 10

### Preparation of 6-({2-[1-(3-chloro-6-methoxynaphthyridin-4-yl)piperidin-4-yl]ethylamino} methyl)-4H-pyrido[3,2-b][1,4]thiazin-3-one

### a) {2-[1-(3-chloro-6-methoxynaphthyridin-4-yl)piperidin-4-yl]ethyl}carbamic acid tert-butyl ester

To a solution of (2-piperidin-4-ylethyl) carbamic acid *tert-*butyl ester (1.83 g, 8.02 mmole) in DMF (10 mL) at RT was added 1, 1,1-trifluoromethane sulfonic acid 3-chloro-6-methoxy[1,5]naphthyridin-4-yl ester (2.50 g, 7.29 mmole) and Et₃N (0.74 g, 7.30 mmole). After 18 hour at 100 °C, the reaction solution was concentrated under vacuum and purified by flash chromatography on silica gel (EtOAc/hexanes, 1:1) to afford the title compound as a tan solid (2.0 g, 65%): ¹H NMR (400 MHz, CDCl₃) 8.55 (s, 1 H), 8.13 (d, *J* = 9.0 Hz, 1H), 7.06 (d, *J* = 9.0 Hz, 1 H), 4.52 (br s, 1 H), 4.05 (s, 3H), 3.78 (m, 2H), 3.50 (m, 2H), 3.25 (m, 2H), 1.84 (m, 2H), 1.48-1.54 (m, 5H). LC-MS (ES) m/e 421 (M + H)⁺.

### b) 2-[-(3-chloro-6-methoxynaphthyridin-4-yl)piperidin-4-yl]ethylamine

To a solution of {2-[1-(3-chloro-6-methoxynaphthyridin-4-yl)piperidin-4-yl]ethyl)carbamic acid *tert*-butyl ester (2.0 g, 4.76 mmole) in CH₂Cl₂ (50 mL) at RT was added TFA (1:1, v/v). After 2 hrs, the solution was concentrated to dryness under vacuum and the residue redissolved in CH₂Cl₂/ MeOH (9:1, v/v). The solution was washed with saturated aqueous NaHCO₃ solution, dried over Na₂SO₄, and concentrated under vacuum to give the title compound (1.29 g, 85%) as a tan viscous solid: LC-MS (ES) m/e 321 (M + H)⁺.

### c) 6-({2-[1-(3-chloro-6-methoxynaphthyridin-4-yl)piperidin-4-yl]ethylamino}methyl)-4H-pyrido[3,2-b][1,4]thiazin-3-one

To a solution of 2-[1-(3-chloro-6-methoxyquinolin-4-yl)piperidin-4-yl]ethylamine (0.74 g, 2.33 mmole) in CH₂Cl₂ (100 mL) and EtOH (50mL) was added Na₂SO₄ (100 mg) and 3-oxo-3,4-dihydro-2*H*-pyrido[1,4]thiazine-6-carboxaldehyde (0.47 g, 2.45 mmole). After 12 hr at RT, NaBH₄ (93 mg, 2.45 mmole) was added and the reaction solution was allowed to stir overnight. Silica gel (-5 g) was added to the reaction solution and the contents were concentrated under vacuum. The silica-adsorbed reaction contents were added directly to a silica gel column (CHCl₃/MeOH containing 5% NH₄OH 9:1) to give the title compound (0.90 g, 77 %) as an off-white solid: ¹H NMR (400 MHz,CDCl₃) 8.54 (s, 1 H), 8.11 (d, *J*= 9.0 Hz, 1H), 7.63 (d, *J*= 7.8 Hz, 1 H), 7.05 (d, *J*= 9.0 Hz, 1 H), 7.00 (d, *J* = 7.8 Hz, 1 H), 4.70 (s, 1 H), 4.04 (s, 3H), 3.98 (s, 2H), 3.78 (m, 2H), 3.49 (m, 4H), 2.88 (m, 2H), 1.56-1.79 (m, 6H). LC-MS (ES) m/e 499 (M + H)⁺.

### Example 11

### Preparation of 6-({2-1-(3-chloro-6-methoxynaphthyridin-4-yl)piperidin-4-yl]ethylamino)methyl)-4H-pyrido[3,2-b][1,4]oxazin-3-one

According to the procedure of Example 10c, except substituting 3-oxo-3,4-dihydro-2*H*-pyrido[3,2-*b*][1,4]oxazine-6-carboxaldehyde (0.44 g, 2.45 mmole) for 3-oxo-3,4-dihydro-2*H*-pyrido[3,2-*b*][1,4]thiazine-6-carboxaldehyde, the title compound (1.0 g, 89 %) was prepared as an off-white solid following flash chromatography on silica gel (CHCl₃/MeOH, 9:1, containing 5% NH₄OH): ¹H NMR (400 MHz,CDCl₃) 8.55 (s, 1 H), 8.12 (d, *J* = 9.0 Hz, 1H), 7.25 (d, *J* = 8.0 Hz, 1H), 7.05 (d, *J* = 9.0 Hz, 1H), 7.01 (d, *J* = 8.0 Hz, 1 H), 4.68 (m, 3H), 4.04 (s, 3H), 3.91 (s, 2H), 3.76 (m, 2H), 3.50 (m, 2H), 2.80 (m, 2H), 1.81 (m, 2H), 1.67 (m, 2H), 1.54 (m, 2H). LC-MS (ES) m/e 483 (M + H)⁺.

### Example 12

### Preparation of {2-[1-(3-chloro-6-methoxynaphthyridin-4-yl)piperidin-4-yl]ethyl]}-(2,3-dihydro[1,4]dioxino[2,3-c]pyridin-7-ylmethyl)amine

According to the procedure of Example 10c, except substituting 2,3-dihydro[1,4]dioxino[2,3-*c*]pyridine-7-carboxaldehyde (0.15 g, 0.93 mmole) for 3-oxo-3,4-dihydro-2*H*-pyrido[3,2-*b*][1,4]thiazine-6-carboxaldehyde, the title compound (0.35 g, 80 %) was prepared as an off-white viscous oil following flash chromatography on silica gel (CHCl₃/MeOH, 9:1, containing 5% NH₄OH): ¹H NMR (400 MHz, CD₃OD) 8.79 (s, 1H), 8.55 (s, 1 H), 8.25 (d, *J* = 9.2 Hz, 1 H), 7.69 (s, 1 H), 7.46 (d, *J* = 9.2 Hz, 1 H), 4.67 (m, 2H), 4.54 (m, 6H), 4.14 (s, 3H), 3.72 (m, 2H), 3.32 (m, 2H), 2.06 (m, 2H), 1.87 (m,2H), 1.69 (m, 2H). LC-MS (ES) m/e 470 (M)⁺.

### Example 13

### Preparation of 6-({2-[4-(6-methoxyquinolin-4-yl)piperazin-1-yl]ethylamino}methyl)-4H-pyrido[3,2-b][1,4]oxazin-3-one

### (a) 2,2,2-trifluoro-N-{2-[4-(6-methoxyquinolin-4-yl)piperazin-1-yl]ethyl}acetamide

To solution of 1,1,1-trifluoro-*N*-(2-piperazin-1-ylethyl)acetamide (2.3 g, 10.0 mmole) in DMF (5 mL) at RT was added 4-bromo-6-methoxyquinoline (2.0 g, 8.4 mmole) and triethylamine (0.86 mL, 8.4 mmole). After 18 hr at 100 °C, the reaction solution was concentrated under vacuum and purified on silica gel (CHCl₃/MeOH, 9:1, containing 5% NH₄OH to give the title compound as an off-white solid (2.32 g, 73 %): LC-MS (ES) m/e 383 (M + H)⁺.

### (b) 2-[4-(6-methoxyquinolin-4-yl)pierazin-1-ylethylamine

To solution of 2,2,2-trifluoro-N-{2-[4-(6-methoxyquinolin-4-yl)piperazin-1-yl]ethyl}acetamide (1.11 g, 2.9 mmole) in methanol (50 mL) at RT was added K₂CO₃ (2.0 g, 14.5 mmole) and H₂O (25 mL). After 18 hr at RT, the reaction solution was concentrated under vacuum and purified on silica gel (CHCl₃/MeOH, 9:1, containing 5% NH₄OH) to give the title compound as a thick oil (0.75 g, 90 %): ¹H NMR (400 MHz, CDCl₃) δ 8.65 (d, *J* = 5 Hz, 1H), 7.99 ( d, *J* = 9 Hz, 1H), 7.36 (d, *J* = 2.8 Hz, 1H), 7.32 (m, 1 H), 6.88 (d, *J* = 5 Hz, 1 H), 3.95 (s, 3H), 3.25 (s, 4H), 2.89 (t, *J* = 6 Hz, 2H), 2.80 (s, 4H), 2.58 (t, *J* = 6 Hz, 2H), and 1.62 (s, 2H).

### (c) 6-({2-[4-(6-methoxyquinolin-4-yl)piperazin-1-yl]ethylamino}methyl-4H-pyrido [3,2-b][1,4]oxazin-3-one

To a solution of 2-[4-(6-methoxyquinolin-4-yl)piperazin-1-yl]ethylamine (0.35 g, 1.22 mmole) in CH₂Cl₂ (25 mL) and EtOH (25 mL) was added Na₂SO₄ (50 mg) and 3-oxo-3,4-dihydro-2*H*-pyrido[1,4]oxazine-6-carboxaldehyde (0.22g, 1.22 mmole). After 12 hr at RT, NaBH₄ (46 mg, 1.22 mmole) was added and the reaction solution was allowed to stir overnight. Silica gel (-5 g) was added to the reaction solution and the contents were concentrated under vacuum. The silica-adsorbed reaction contents were added directly to a silica gel column (CHCl₃/MeOH containing 5% NH₄OH 9:1) to give the title compound (0.27 g, 50 %) as an off-white solid: ¹H NMR (400 MHz, *d*₄-MeOH) 8.71 (d, *J*= 6.3 Hz, 1H), 8.03 ( d, *J*= 9 Hz, 1 H), 7.75 ( dd, *J*= 9 and 2.8 Hz, 1H), 7.42 (m, 3H), 7.15 (d, *J* = 8 Hz, 1 H), 4.73 (s, 2H), 4.40 (s, 2H), and 3.97 ( m, 15H). LC-MS (ES) m/e 449 ( M + H)⁺.

### Example 14

### Preparation of 6-({2-[4-(6-methoxyquinolin-4-yl)piperazin-1-yl]ethylamino}methyl)-4H-pyrido[3,2-b][1,4]thiazin-3-one

According to the procedure of Example 13c, except substituting 3-oxo-3,4-dihydro-2*H*-pyrido[3,2-*b*][1,4]thiazine-6-carboxaldehyde (0.24 g, 1.26mmole) for 3-oxo-3,4-dihydro-2*H*-pyrido[3,2-*b*][1,4]oxazine-6-carboxaldehyde, the title compound (0.22 g, 45 %) was prepared as an off-white solid following flash chromatography on silica gel (CHCl₃/MeOH, 9:1, containing 5% NH₄OH): ¹H NMR (400 MHz, *d*₄-CH₃OH) 8.68 (d, *J*= 6.6 Hz, 1 H), 8.02 ( d, *J*= 9.3 Hz, 1 H), 7.86 (d, *J*= 7.8 Hz, 1H), 7.74 (d, *J*= 9.3 Hz, 1 H), 7.43 (m, 2H), 7.18 (d, *J* = 7.8 Hz, 1H), 4.46 (s, 2H), and 3.83 ( m, 16H). LC-MS (ES) m/e 465(M+H)⁺.

### Example 15

### Preparation of (2,3-dihydro-[1,4]dioxino[2,3-c]pyridin-7-ylmethyl)-{2-[4-(6-methoxyquinolin-4-yl)piperizin-1-yl]ethyl}amine

According to the procedure of Example 13c, except substituting 2,3-dihydro[1,4]dioxino[2,3-*c*]pyridine-7-carboxaldehyde (0.21 g, 1.26 mmole) for 3-oxo-3,4-dihydro-2*H*-pyrido[3,2-*b*][1,4]oxazine-6-carboxaldehyde, the title compound (86 mg, 25 %) was prepared as an off-white solid following flash chromatography on silica gel (CHCl₃/MeOH, 9:1, containing 5% NH₄OH): ¹H NMR (400 MHz, *d*₄-CH₃OH) 8.59 (d, *J* = 7.0 Hz, 1H), 8.29 (s, 1 H), 7.91 ( d, *J* = 9.4 Hz, 1 H), 7.63 (d, *J* = 9.1 Hz, 1 H), 7.34 (d, *J* = 7.0 Hz, 1 H), 4.47 (s, 2H), 4.35 (m, 4H), 4.01 (m, 4H), 3.96 (s, 3H), 3.67 (m, 4H) and 3.58 (m, 4H). LC-MS (ES) m/e 436 (M + H)⁺.

### Example 16

### Preparation of 6-({2-[4-(6-methoxynaphthyridin-4-yl)piperazin-1-yl]ethylamino}methyl)-4H-pyrido[3,2-b][1,4]thiazin-3-one

### (a) 2,2,2-trifluoro-N-{2-[4-(6-methoxynaphthyridin-4-yl)piperazin-1-yl]ethyl}acetamide

To solution of 1,1,1-trifluoro-*N*-(2-piperazin-1-ylethyl)acetamide (2.2 g, 9.73 mmole) in DMF (5 mL) at RT was added 1 1,1-trifluoromethanesulfonic acid 6-methoxy[1,5]naphthyridin-4-yl ester (3.0 g, 9.73 mmole) and triethylamine (1.63 mL, 11.68 mmole). After 18 hr at 100°C, the reaction solution was concentrated under vacuum and purified on silica gel (CHCl₃/MeOH, 9:1, containing 5% NH₄OH) to give the title compound as an off-white solid (2.70 g, 72 %): LC-MS (ES) m/e 384 (M + H)⁺.

### (b) 2-[4-(6-methoxynaphthyridin-4-yl)piperazin-1-yl]ethylamine

To solution of 2,2,2-trifluoro-N-{2-[4-(6-methoxyquinolin-4-yl)piperazin-1-yl]ethyl)acetamide (2.70 g, 7.0 mmole) in methanol (100 mL) at RT was added K₂CO₃ (4.86 g, 35.2 mmole) and H₂O (25 mL). After 18 hr at RT, the reaction solution was concentrated under vacuum and purified on silica gel (CHCl₃/MeOH, 9:1, containing 5% NH₄OH) to give the title compound as a thick oil (1.75 g, 86 %): LC-MS (ES) m/e 288 (M +H)⁺.

### (c) 6-({2-[4-(6-methoxynaphthyridin-4-yl)piperazin-1-yl]ethylamino}methyl)-4H-pyrido[3,2-b][1,4]thiazin-3-one

To a solution of 2-[4-(6-methoxynaphthyridin-4-yl)piperazin-1-yl]ethylamine (1.0 g, 3.48 mmole) in CH₂Cl₂ (50 mL) and EtOH (50 mL) was added Na₂SO₄ (50 mg) and 3-oxo-3,4-dihydro-2*H*-pyrido[1,4]thiazine-6-carboxaldehyde (0.68 g, 3.48 mmole). After 12 hr at RT, NaBH₄ (132 mg, 3.48 mmole) was added and the reaction solution was allowed to stir overnight. Silica gel (-5 g) was added to the reaction solution and the contents were concentrated under vacuum. The silica-adsorbed reaction contents were added directly to a silica gel column (CHCl₃/MeOH containing 5% NH₄OH, 9:1) to give the title compound (0.90 g, 50 %) as an off-white solid: ¹H NMR (400 MHz, d₄-MeOH) 8.53 (d, *J*= 7 Hz, 1H), 8.27 (d, *J* = 9.2 Hz, 1H), 7.85 (d, *J* = 7.8 Hz, 1 H), 7.51 (d, *J* = 9.2 Hz, 1 H), 7.39 (d, *J*= 7 Hz, 1H), 7.17 (d, *J* = 7.8 Hz, 1 H), 4.46 (s, 2H), 4.12 (s, 3H), 3.80 ( m, 8 H), and 3.59 (s, 2H). LC-MS (ES) m/e 466 ( M + H)⁺.

### Example 17

### Preparation of 6-({2-[4-(6-methoxynaphthyridin-4-yl)piperazin-1-yl]ethylamino}-methyl)-4H-pyrido[3,2-b][1,4]oxazin-3-one

According to the procedure of Example 16c, except substituting 3-oxo-3,4-dihydro-2*H*-pyrido[3,2-*b*][1,4]oxazine-6-carboxaldehyde (0.50 g, 2.82 mmole) for 3-oxo-3,4-dihydro-2*H*-pyrido[3,2-*b*][1,4]thiazine-6-carboxaldehyde, the title compound (0.75 g, 60 %) was prepared as an off-white solid following flash chromatography on silica gel (CHCl₃/MeOH, 9:1, containing 5% NH₄OH): ¹H NMR (400 MHz, *d*₄-CH₃OH) 8.49 (d, *J*= 7.0 Hz, 1H), 8.24 ( d, *J*= 9.2 Hz, 1 H), 7.49 (d, *J*= 9.2 Hz, 1H), 7.40 (d, *J*= 8.1 Hz, 1 H), 7.34 (d, *J* = 7 Hz, 1H), 7.14 (d, *J* = 8.1 Hz, 1H), 4.73 (s, 2H), 4.38 (s, 2H), 4.11 (s, 3H), and 3.59 (m, 8H). LC-MS (ES) m/e 450 (M + H)⁺.

### Example 18

### Preparation of (2,3-dihydro[1,4]dioxino[2,3-c]pyridin-7-ylmethyl){2-[4-(6-methoxy[1,5]naphthyridin-4-yl)piperazin-1-yl]ethyl}amine

According to the procedure of Example 16c, except substituting 2,3-dihydro[1,4]dioxino[2,3-*c*]pyridine-7-carboxaldehyde (0.08 g, 0.47 mmole) for 3-oxo-3,4-dihydro-2*H*-pyrido[3,2-*b*][1,4]thiazine-6-carboxaldehyde, the title compound (0.10 g, 50 %) was prepared as an off-white solid following flash chromatography on silica gel (CHCl₃/MeOH, 9:1, containing 5% NH₄OH): ¹H NMR (400 MHz, *d*₄-CH₃OH) 8.52 (d, *J*= 7.2 Hz, 1 H), 8.38 (s, 1 H), 7.27 (d, *J* = 9.2 Hz, 1 H), 7.50 (d, *J* = 9.2 Hz, 1 H), 7.42 (s, 1 H), 7.38 (d, *J*= 7.2 Hz, 1H), 4.73 (s, 4H), 4.56 (m, 2H), 4.46 (m, 2H), 4.43 (s, 2H), 4.12 (s, 3H), 3.75 (m, 4H), and 3.65 (s, 4H). LC-MS (ES) m/e 437 (M + H)⁺.

### Example 19

### Preparation of 6-({2-[4-(3-chloro-6-methoxyquinolin-4-yl)piperazin-1-yl] ethylamino}methyl)-4H-pyrido[3,2-b][1,4]thiazin-3-one

### (a) 2,2,2-trifluoro-N-{2-[4-(3-chloro-6-methoxyquinolin-4-yl)piperazin-1-yl] ethyl}acetamide

To solution of 1,1,1-trifluoro-*N*-(2-piperazin-1-ylethyl)acetamide (2.01 g, 8.78 mmole) in DMF (5 mL) at RT was added 1 1,1-trifluoromethanesulfonic acid 3-chloro-6-methoxy[1,5]quinolin-4-yl ester (3.0 g, 8.78 mmole) and triethylamine (1.50mL), 10.5 mmole). After 18 hr at 100 °C, the reaction solution was concentrated under vacuum and purified on silica gel (CHCl₃/MeOH, 9:1, containing 5% NH₄OH to give the title compound as an off-white solid (2.40 g, 66 %): LC-MS (ES) m/e 417 (M + H)⁺.

### (b) 2-[4-(3-chloro-6-methoxyquinolin-4-yl)piperazin-1-yl]ethylamine

To solution of 2,2,2-trifluoro-N-{2-[4-(3-chloro-6-methoxyquinolin-4-yl)piperazin-1-yl]ethyl}acetamide (2.40 g, 5.75 mmole) in methanol (100 mL) at RT was added K₂CO₃ (4.10 g, 30.0 mmole) and H₂O (25 mL). After 18 hr at RT, the reaction solution was concentrated under vacuum and purified on silica gel (CHCl₃/MeOH, 9:1, containing 5% NH₄OH to give the title compound as a waxy yellow solid (1.40 g, x %): LC-MS (ES) m/e 321 (M + H)⁺.

### (c) 6-({2-[4-(3-chloro-6-methoquinolin-4-yl)piperazin-1-yl]ethylamino}methyl)-4H-pyrido[3,2-b][1,4]thiazin-3-one

To a solution of 2-[4-(3-chloro-6-methoxyquinolin-4-yl)piperazin-1-yl]ethylamine (0.56 g, 1.74 mmole) in CH₂Cl₂ (50 mL) and EtOH (50 mL) was added Na₂SO₄ (50 mg) and 3-oxo-3,4-dihydro-2*H*-pyrido[1,4]thiazine-6-carboxaldehyde (0.34 g, 1.74 mmole). After 12 hr at RT, NaBH₄ (66 mg, 1.74 mmole) was added and the reaction solution was allowed to stir overnight. Silica gel (-5 g) was added to the reaction solution and the contents were concentrated under vacuum. The silica-adsorbed reaction contents were added directly to a silica gel column (CHCl₃/MeOH containing 5% NH₄OH, 9:1) to give the title compound (0.49 g, 57 %) as an off-white solid: ¹H NMR (400 MHz, CD₃OD) 9.04 (s, 1H), 8.07 (d, *J* = 9.2 Hz, 1H), 7.85 (d, *J* = 7.8 Hz, 1H), 7.75 (d, *J* = 9.2 Hz, 1H), 7.52 (m, 1 H), 7.18 (d, *J*= 7.8 Hz, 1 H), 4.48 (s, 2H), 4.25 (m, 4H), 4.13 (s, 3H), 3.84 (m, 4H), 3.75 (m, 2H), 3.60 (m, 2H), 3.37 (m, 2H). LC-MS (ES) m/e 499 (M)⁺.

### Example 20

### Preparation of 6-({2-[4-(3-chloro-6-methoxyquinolin-4-yl)piperazin-1-yl]ethylamino} methyl)-4H-pyrido[3,2-b][1,4]oxazin-3-one

According to the procedure of Example 19c, except substituting 3-oxo-3,4-dihydro-2*H*-pyrido[3,2-*b*][1,4]oxazine-6-carboxaldehyde (0.28 g, 1.56 mmole) for 3-oxo-3,4-dihydro-2*H*-pyrido[3,2-*b*][1,4]thiazine-6-carboxaldehyde, the title compound (0.56 g, 70 %) was prepared as an off-white solid following flash chromatography on silica gel (CHCl₃/MeOH, 9:1, containing 5% NH₄OH): ¹H NMR (400 MHz, DMSO-*d*₆) 10.10 (br s, 1 H), 8.89 (s, 1 H), 8.14 ( d, *J* = 9.2 Hz, 1 H), 7.61 (d, *J* = 9.2 Hz, 1H), 7.45 (m, 2H), 7.34 (d, *J*= 8.1 Hz, 1 H), 4.70 (s, 2H), 4.23 (m, 2H), 4.04 (s, 3H), 3.85-3.60 (m, 12H). LC-MS (ES) m/e 483 (M)⁺.

### Example 21

### Preparation of {2-[4-(3-chloro-6-methoxyquinolin-4-yl)piperazin-1-yl)ethyl}-(2,3-dihydro[1,4]dioxino[2,3-c]pyridin-7-ylmethyl)amine

According to the procedure of Example 19c, except substituting 2,3-dihydro[1,4]dioxino[2,3-*c*]pyridine-7-carboxaldehyde (0.10 g, 0.62 mmole) for 3-oxo-3,4-dihydro-2*H*-pyrido[3,2-*b*][1,4]thiazine-6-carboxaldehyde, the title compound (0.25 g, 86 %) was prepared as an off-white solid following flash chromatography on silica gel (CHCl₃/MeOH, 9:1, containing 5% NH₄OH): ¹H NMR (400. MHz, DMSO-*d*₆) 8.80 (s, 1H), 8.36 (s, 1H), 8.07 (d, *J*= 9.2 Hz, 1 H), 7.55 (d, *J*= 9.2 Hz, 1H), 7.43 (m, 2H), 4.48 is, 2H), 4.39 (s, 2H), 4.35 (m, 2H), 4.02 (s, 3H), 3.83-3.61 (m, 12H). LC-MS (ES) m/e 470 (M)⁺.

### Example 22

### Preparation of 6-{2-[4-(3-chloro-6-methoxvnaohthyridin-4-yl)piperazin-1-yl]ethylamino}methyl)-4H-pyrido[3,2-b][1,4]thiazin-one

### (a) 2,2,2-trifluoro-N-{2-[4-(3-chloro-6-methoxynaphthyridin-4-yl)piperazin-1-yl] ethyl}acetamide

To solution of 1,1,1-trifluoro-*N*-(2-piperazin-1-ylethyl)acetamide (2.10 g, 9.31 mmole) in DMF (5 mL) at RT was added 1 1,1-trifluoromethanesulfonic acid 3-chloro-6-methoxy[1,5]quinolin-4-yl ester (3.19 g, 9.31 mmole) and triethylamine (1.30 mL, 9.31 mmole). After 18 hr at 100 °C, the reaction solution was concentrated under vacuum and purified on silica gel (CHCl₃/MeOH, 9:1, containing 5% NH₄OH) to give the title compound as an off-white solid (2.61 g, 67 %): LC-MS (ES) m/e 418 (M + H)⁺.

### (b) 2-[4-(3-chloro-6-methoxyquinolin-4-yl)piperazin-1-yl]ethylamine

To solution of 2,2,2-trifluoro-N-{2-[4-(3-chloro-6-methoxynaphthyridin-4-yl)piperazin-1-yl]ethyl}acetamide (2.61 g, 6,24 mmole) in methanol (100 mL) at RT was added K₂CO₃ (4.31 g, 31.2 mmole) and H₂O (25 mL). After 18 hr at RT, the reaction solution was concentrated under vacuum and purified on silica gel (CHCl₃/MeOH, 9:1, containing 5% NH₄OH) to give the title compound as a light yellow solid (2.0 g, 99 %): LC-MS (ES) m/e 322 (M + H)⁺.

### (c) 6-({2-[4-(3-chloro-6-methoxynaphthyridin-4-yl)piperazin-1-yl]ethylamino}methyl)-4H-pyrido[3,2-b][1,4]thiazin-3-one

To a solution of 2-[4-(3-chloro-6-methoxynaphthyridin-4-yl)piperazin-1-yl]ethylamine (1.0 g, 3.11 mmole) in CH₂Cl₂ (50 mL) and EtOH (50 mL) was added Na₂SO₄ (50 mg) and 3-oxo-3,4-dihydro-2*H-*pyrido[1,4]thiazine-6-carboxaldehyde (0.61 g, 3.11 mmole). After 12 hr at RT, NaBH₄ (120 mg, 3.11 mmole) was added and the reaction solution was allowed to stir overnight. Silica gel (-5 g) was added to the reaction solution and the contents were concentrated under vacuum. The silica-adsorbed reaction contents were added directly to a silica gel column (CHCl₃/MeOH containing 5% NH₄OH, 9:1) to give the title compound (0.82 g, 53 %) as an off-white solid: ¹H NMR (400 MHz, DMSO-d₆) 10.10 (br s, 1H), 8.89 (s, 1 H), 8.42 (d, *J* = 9.2 Hz, 1 H), 7.91 (d, *J* = 7.0 Hz, 1H), 7.42 (d, *J*= 9.2 Hz, 2H), 7.35 (d, J = 7.0 Hz, 1 H), 4.28 (s, 2H), 4.12 (s, 4H), 4.05 (s, 3H), 3.81 (m, 2H), 3.73 (m, 4H), 3.61 (s, 2H), 3.42 (m, 2H). LC-MS (ES) m/e 500 (M + H)⁺.

### Example 23

### Preparation of 6-({2-[4-(3-chloro-6-methoxynaphthyridin-4-yl)piperazin-1-yl]ethylamino} methyl)-4H-pyrido[3,2-b][1,4]oxazin-3-one

According to the procedure of Example 22c, except substituting 3-oxo-3,4-dihydro-2*H*-pyrido[3,2-*b*][1,4]oxazine-6-carboxaldehyde (0.56 g, 3.11 mmole) for 3-oxo-3,4-dihydro-2*H-*pyrido[3,2*-b*][1,4]thiazine-6-carboxaldehyde, the title compound (0.98 g, 65 %) was prepared as an off-white solid following flash chromatography on silica gel (CHCl₃/MeOH, 9:1, containing 5% NH₄OH): ¹H NMR (400 MHz, DMSO-d₆) 9.84 (br s, 1 H), 8.70 (s, 1 H), 8.24 ( d, *J* = 9.2 Hz, 1 H), 7.37 (d, *J* = 9.2 Hz, 1 H), 7.25 (m, 2H), 4.61 (s, 2H), 4.13 (s, 2H), 4.05 (m, 2H), 3.95 (s, 3H), 3.89 (m, 2H), 3.70 (m, 2H), 3.58 (m, 4H), 3.28 (m, 2H). LC-MS (ES) m/e 484 (M + H)⁺.

### Example 24

### Preparation of (2,3-dihydro[1,4]dioxino[2,3-c]pyridin-7-ylmethyl)-{2-[4-(3-chloro-6-methoxy[1,5]naphthyridin-4-yl]piperazin-1-yl]ethyl}amine

According to the procedure of Example 22c, except substituting 2,3-dihydro[1,4]dioxino[2,3-c]pyridine-7-carboxaldehyde (0.23 g, 1.40 mmole) for 3-oxo-3,4-dihydro-2*H-*pyrido[3,2*-b*][1,4]thiazine-6-carboxaldehyde, the title compound (0.54g, 82 %) was prepared as an off-white solid following flash chromatography on silica gel (CHCl₃/MeOH, 9:1, containing 5% NH₄OH): ¹H NMR (400 MHz, DMSO-d₆) 8.81 (s, 1H), 8.33 (m, 2H), 7.34 (m, 2H), 4.45 (s, 2H), 4.38 (s, 2H), 4.28 (m, 2H), 4.05 (s, 3H), 4.02 (m 2H), 3.66 (m, 2H), 3.58 (m, 2H), 3.45-3.33 (m, 6H). LC-MS (ES) m/e 469 (M)⁺.

### Example 25

### Preparation of 6-({2-[4-(6-methoxy-[1,5]naphthyridin-4-yl)-3,6-dihydro-2H-pyridin-1-yl]-2-oxo-ethylamino}-methyl)-4H-pyrido[3,2-b][1,4]thiazin-3-one dihydrochloride

### (a) 4-Trifluoromethanesulfonyloxy-3,6-dihydro-2H -pyridine-1-carboxylic acid tert-butyl ester

According to the procedure of Wustrow and Wise (Synthesis 1991, 993) to a solution of N-Boc-piperidone (7.99 g) in THF (50 mL) at -78°C was added LDA (2M in THF) and the solution was stirred for 30 min. N-phenyltrifluoromethanesulfonimide (3.08 g) was then added and the reaction warmed to room temperature, treated with water (100 mL). The reaction was then extracted with dichloromethane (2 x 200 mL) and the organic fraction dried (MgSO₄). The product was purified by column chromatography (9:1 petrol:ethyl acetate) to give the product as a colourless solid (10.64 g). MS (+ve ion electrospray) m/z 332 (MH⁺).

### (b) 4-(6-Methoxy-[1,5]naphthyridin-4-yl)-3,6-dihydro-2H-pyridine-1-carboxylic acid tert-bulyl ester

According to the procedure of Ishiyama, Itoh, Kitano and Miyaura (Tetrahedron Letters 1997, 38 3447) to a solution of 1,1,1-trifluoro-methanesulfonic acid 6-methoxy-[1,5]naphthyridin-4-yl ester (4.15 g) in 1,4-dioxane (60 mL) was added PdCl₂(dppf) (296mg), KOAc (3.95 g), dppf (221 mg) and bispinacolatodiboron (3.76 g). This mixture was heated at 90°C for 24h. After this time another 592 mg of PdCl₂(dppf) was added and the reaction was stirred at 90°C for a further 24h. The reaction mixture was then treated with water (100 mL) and extracted with dichloromethane (2 x 200mL) and the organic fraction dried (MgSO₄) and evaporated *in vacuo.* To this crude material was added triflate (a), potassium carbonate, PdCl₂(dppf) (591 mg) and DMF (100 mL) and the mixture was stirred at 80°C for 72h. The reaction mixture was then treated with water (100 mL) and extracted with dichloromethane (3 x 100 mL) and the organic fraction dried (MgSO₄) and purified by column chromatography (4:1 petrol:ethyl acetate) to give the product as a yellow solid (2.53 g).
MS (+ve ion electrospray) m/z 342 (MH⁺).

### (c) 2-Methoxy-8-(1,2,3,6-tetrahydro-pyridin-4-yl)-[1,5]naphthyridine

To a solution of compound (b) (2.54 g) in DCM (50 mL) was added TFA (10 mL) and the reaction was stirred at room temperature for 1 h before being evaporated in vacuo and basified with saturated sodium bicarbonate (10 mL) and extracted with 9:1 MeOH:DCM (3 x 100 mL). The combined organic fractions were dried (MgSO₄) and evaporated *in vacuo* to give (c) as a yellow solid (1.64 g).
MS (+ve ion electrospray) m/z 242 (MH⁺).

### (d) {2-[4-(6-Methoxy-[1,5]naphthyridin-4-yl)-3,6-dihydro-2H-pyridin-1-yl]-2-oxo-ethyl}-carbamic acid tert-butyl ester

To a solution of N-Boc-glycine (239 mg) in DMF (10ml) was added triethylamine (0.68 mL) was added HATU (517 mg) and the solution was stirred for 10 min. Compound (c) (262 mg) was then added and the solution was stirred for 18h. The reaction mixture was then treated with water (20 mL) and extracted with dichloromethane (2 x 50 mL) and the organic fraction dried (MgSO₄) and purified by column chromatography (1:1 petrol:ethyl acetate) to give (d) as a yellow solid (416 mg).
MS (+ve ion electrospray) m/z 399 (MH⁺).

### (e) 2-Amino-1-[4-(6-methoxy-[1,5]naphthyridin-4-yl)-3,6-dihydro-2H-pyridin-1-yl]-ethanone dihydrochloride

To a solution of (d) (410 mg) in methanol (5 mL) was added 4M HCl in dioxane (10 mL) and the solution was stirred at room temperature for 15 min. The reaction mixture was then evaporated in vacuo to give (e) (297 mg) as a yellow solid. MS (+ve ion electrospray) m/z 299 (MH⁺).

### (f) Title compound

To a mixture of (e) (182 mg) in methanol (3 mL) and molecular sieves was added sodium cyanoborohydride (25 mg) and 3-Oxo-3,4-dihydro-2H-pyrido[3,2-b][1,4]thiazine-6-carbaldehyde (95 mg) and the resultant mixture was stirred at room temperature for 18h. The reaction mixture was then treated with water (10 mL) and extracted with 9:1 dichloromethane:methanol (3 x 50 mL) and the organic fraction dried (MgSO₄) and purified by column chromatography (9:1 dichloromethane:methanol) to give (f) as a yellow solid (71 mg).
¹H NMR (DMSO, 400MHz), 10.90 (s, 1H), 8.74 (d, *J* = 4.5 Hz, 1 H), 8.26 (d, *J* = 9.0 Hz, 1 H), 7.73-7.77 (m, 1 H), 7.52-7.54 (m, 1 H), 7.26 (d, *J* = 9.0 Hz, 1 H), 7.09 (d, *J* = 9.0 Hz, 1 H), 6.35 (br s, 1H), 4.20 (s, 2H), 3.97 (s, 3H), 3.77 (s, 2H), 3.63-3.66 (m, 1 H), 3.52-3.55 (m, 6H), 2.78-2.84 (m, 2H).
MS (ES) *m*/*z* 477 (M + H)⁺.

This material was converted to the dihydrochloride by dissolving in chloroform and adding 2 equivalents of 1 M HCl/ether then evaporating to dryness.

### Example 26

### Preparation of N-(2-{1-[6-(methyloxy)-1,5-naphthyridin-4-yl]-4-piperidinyl}ethyl)-3-oxo-3,4-dihydro-2H-pyrido[3,2-b][1,4]thiazine-6-carboxamide

### a) {2-[1-(6-methoxynaphthyridin-4-yl)piperidin-4-yl]ethyl}carbamic acid tert-butyl ester

To a solution of (2-piperidin-4-ylethyl) carbamic acid *tert*-butyl ester (0.81 g, 3.57 mmole) in DMF (5 mL) at RT was added 1,1,1-trifluoromethane sulfonic acid 6-methoxy[1,5]naphthyridin-4-yl ester (1.0 g, 3.24 mmole) and Et₃N (0.33 g, 3.24 mmole). After 18 hour at 100 °C, the reaction solution was concentrated under vacuum and purified by flash chromatography on silica gel (CHCl₃/MeOH containing 5% NH₄OH, 9:1) to afford the title compound as a tan solid (1.19 g, 95%): ¹H NMR (400 MHz, CDCl₃) 8.51 (d, *J* = 5.3 Hz, 1 H), 8.18 (d, *J* = 9.0 Hz, 1 H), 7.09 (d, *J* = 9.0 Hz, 1 H), 6.85 (d, *J* = 5.3 Hz, 1 H), 4.55 (br s, 1H), 4.36 (m, 2H), 4.06 (s, 3H), 3.25 (m, 2H), 2.90 (m, 2H), 1.92 (m, 2H), 1.57 (m, 4H), 1.47 (s, 9H). LC-MS (ES) m/e 387 (M + H)⁺.

### b) 2-[1-(6-methoxynaphthyridin-4-yl)piperidin-4-yl]ethylamine

To a solution of {2-[1-(6-methoxynaphthyridin-4-yl)piperidin-4-yl]ethyl}carbamic acid *tert*-butyl ester (1.19 g, 3.08 mmole) in CH₂Cl₂ at RT was added TFA (1:1, v/v). After 2 hrs, the solution was concentrated to dryness under vacuum and the residue redissolved in CH₂Cl₂/ MeOH (9:1, v/v). The solution was washed with saturated aqueous NaHCO₃ solution, dried over Na₂SO₄, and concentrated under vacuum to give the title compound (0.79 g, 90%) as a waxy yellow solid: LC-MS (ES) m/e 287 (M + H)⁺.

### c) N-(2-{1-[6-(methyloxy)-1,5-naphthyridin-4-yl]-4-piperidinyl)ethyl)-3-oxo-3,4-dihydro-2H-pyrido[3,2-b]thiazine-6-carboxamide

To a solution of 2-[1-(6-methoxynaphthyridin-4-yl)piperidin-4-yl]ethylamine (1.04 mmole) in DMF (20 mL) was added HOBt (0.15 g, 1.14 mmole), EDC (0.22 g, 1.14 mmole), diisopropylethylamine (0.72 mL, 4.16 mmole) and 3-oxo-3,4-dihydro-2*H-*pyrido[3,2*-b*][1,4]thiazine-6-carboxylic acid (0.22 g, 1.04 mmole). After 12 hr at RT, the reaction contents were concentrated under vacuum and purified on silica gel (CHCl₃/MeOH containing 5% NH₄OH, 9:1) to give the title compound (0.39 g, 78 %) as an off-white solid: ¹H NMR (400 MHz, CD₃OD) 8.28 (m, 1 H), 8.17 (d, *J* = 9.0 Hz, 1 H), 7.89 (d, *J* = 7.7 Hz, 1 H), 7.71 (d, *J* = 7.7 Hz, 1H), 7.40 (d, *J* = 9.0 Hz, 1 H), 7.19 (d, *J* = 6.6 Hz, 1H), 4.06 (s, 3H), 3.61 (m, 2H), 3.51 (m, 4H), 3.05 (m, 2H), 2.91 (m, 2H), 2.12 (m, 2H), 1.93 (m, 1 H), 1.69 (m, 2H). LC-MS (ES) m/e 479 (M + H)⁺.

### Example 27

### Preparation of N-(2-{1-[6-(methyloxy)-1,5-naphthyridin-4-yl]-4-piperidinyl}ethyl)-3-oxo-3,4-dihydro-2H-1,4-benzothiazine-6-sulfonamide

To a solution of 2-[1-(6-methoxynaphthyridin-4-yl)piperidin-4-yl]ethylamine (1.04 mmole) in CH₂Cl₂ (20 mL) was added 3-oxo-3,4-dihydro-2H-benzo[1,4]thiazine-6-sulfonyl chloride (0.16 g, 0.63 mmole). After 24h at RT, the reaction solution was concentrated in vacuo. The remaining solid was purified on silica (CHCl₃/MeOH containing 5% NH₄OH; 9:1) to give the title compound as an off-white solid (0.39 g, 78%): ¹H NMR (400 MHz, CDCl₃) δ 8.64 Hz (br s, 1H,), 8.54 Hz (d, *J* = 5.8 Hz, 1 H), 8.38 (d, *J* = 9.0 Hz), 7.49 (m, 2H), 7.13 (d, *J*= 9.0 Hz, 1 H), 6.86 (d, *J* = 5.8 Hz, 1 H), 5.07 (m, 2H), 4.57 (m, 2H), 4.04 (s, 3H), 3.50 (s, 2H), 3.08 (m, 4H), 1.92 (m, 2H), 1.58 (m, 4H). LC-MS (ES) m/e 514 (M + H)⁺.

### Example 28

### Preparation of N-methyl-N-(2-{1-[6-(methyloxy)-1,5-naphthyridin-4-yl]-4-piperidinyl}ethyl)-3-oxo-3,4-dihydro-2H-pyrido[3,2-b][1,4]thiazine-6-carboxamide

### a) N-methyl-2-{1-[6-(methyloxy)-1,5-naphthyridin-4-yl]-4-piperidinyl}ethanamine

To a solution of {2-[1-(6-methoxynaphthyridin-4-yl)piperidin-4-yl]ethyl}carbamic acid *tert*-butyl ester (4.22 g, 10.9 mmole) in THF (75 mL) was added LiAIH₄ (6.1 mL, 1 M in THF). The reaction contents were heated to 60 °C for 18 h and the allowed to cool to RT. The reaction was quenched sequentially with H₂O (0.25 mL), 1 N NaOH (0.5 mL) and H₂O (0.6 mL). The slurry was filtered through a scinter-glass funnel (Et₂O), concentrated, and the remaining yellow oil was used without further purification: LC-MS (ES) m/e 301 (M+H)⁺.

### b) N-methyl-N-(2-{1-[6-(methyloxy)-1,5-naphthyridin-4-yl]-4-piperidin}ethyl)-3-oxo-3,4-dihydro-2H-pyrido[3,2-b][1,4]thiazine-6-carboxamide

To a solution of *N*-methyl-2-{1-[6-(methyloxy)-1,5-naphthyridin-4,yl]-4-piperidinyl} ethanamine (0.21 g, 0.71 mmole) in DMF (20 mL) was added HOBt (0.11 g, 0.78 mmole), EDC (0.15 g, 0.78 mmole), diisopropylethylamine (0.13 mL, 0.78 mmole) and 3-oxo-3,4-dihydro-2*H-*pyrido[3,2-*b*][1,4]thiazine-6-carboxylic acid (0.15 g, 0.71 mmole). After 12 hr at RT, the reaction contents were concentrated under vacuum and purified on silica gel (CHCl₃/MeOH containing 5% NH₄OH, 9:1) to give the title compound (0.19 g, 55 %) as an off-white solid: ¹H NMR (400 MHz, CDCl₃) 8.52 (m, 1 H), 8.24 (m, 1H), 8.03 (m, 1 H), 7.75 (d, *J* = 8.0 Hz, 1 H), 7.29 (d, *J* = 5.6 Hz, 1 H), 6.86 (d, *J*= 5.6 Hz, 2H), 4.04 (s, 3H), 4.79 (m, 2H), 4.69 (m, 2H), 3.56 (s, 2H), 3.15 (m, 2H), 2.96 (m, 2H), 2.91 (m, 2H), 2.77 (m, 5H). LC-MS (ES) m/e 493 (M + H)⁺.

### Example 29

### Preparation of N-methyl-N(2-{1-[6-(methyloxy)-1,5-naghthridin-4-yl]-4-piperidinyl}ethyl)-3-oxo-3,4-dihydro-2H-1,4-benzothiazine-6-sulfonamide

According to the procedure for Example 2, except substituting *N*-methyl-2-{1-[6-(methyloxy)-1,5-naphthyridin-4-yl]-4-piperidinyl}ethanamine (0.18 g, 1.0 mmole) for 2-[1-(6-methoxynaphthyridin-4-yl)piperidin-4-yl]ethylamine, the title compound (0.41g, 78 %) was prepared as an off-white solid following flash chromatography on silica gel (CHCl₃/MeOH, 9:1, containing 5% NH₄OH): ¹H NMR (400 MHz, CDCl₃) 8.51 (d, *J* = 5.6 Hz, 1H), 8.24 (d, *J* = 8.1 Hz, 1 H), 7.49 (d, *J* = 8.1 Hz, 1H), 7.42 (d, *J* = 8.1 Hz, 1H), 7.10 (d, *J* = 9.0 Hz, 1 H), 6.86 (d, *J* = 5.6 Hz, 2H), 4.41 (m, 2H), 4.06 (s, 3H), 3.51 (s, 2H), 3.13 (m, 2H), 2.96 (m, 2H), 2.78 (s, 3H), 2.00 (m, 2H), 1.72 (m, 1H), 1.65 (m, 2H). LC-MS (ES) m/e 528 (M + H)⁺.

### Example 30

### Preparation of N-(2-{1-[3-chloro-6-(methyloxy)-1,5-naphthyridin-4-yl]-4-piperidinyl}ethyl)-3-oxo-3,4-dihydro-2H-1,4-benzothiazine-6-sulfonamide

According to the procedure for Example 2, except substituting {2-{1-{3-chloro-6-(methyloxy)-1,5-naphthyridin-4-yl]-4-piperidinyl}ethyl)amine (0.89 mmole) for 2-[1-(6-methoxynaphthyridin-4-yl)piperidin-4-yl]ethylamine, the title compound (0.43g, 88 %) was prepared as an off-white solid following flash chromatography on silica gel (CHCl₃/MeOH, 9:1, containing 5% NH₄OH): ¹H NMR (400 MHz, CDCl₃) δ 8.55 (s, 1H), 8.45 (d, *J* = 5.3 Hz, 1 H), 8.13 Hz (d, *J* = 9.0 Hz, 1 H), 7.50 (m, 2H), 7.05 (d, *J* = 9.0 Hz, 1 H), 4.41 (m, 2H), 4.04 (s, 3H), 3.51 (s, 2H), 3.13 (m, 2H), 2.95 (m, 2H), 2.79 (s, 3H), 1.95 (m, 2H), 1.65 (m, 1 H), 1.58 (m, 4H). LC-MS (ES) m/e 548 (M)⁺.

### Example 31

### Preparation of N-(2-{4-[6-(methyloxy)-1,5-naphthyridin-4-yl]-1-piperazinyl}ethyl)-3-oxo-3,4-dihydro-2H-pyrido[3,2-b][1,4]thiazine-6-carboxamide

### a) 2,2,2-trifluoro-N-{2-[4-(6-methoxynaphthyridin-4-yl)piperazin-1-yl]ethyl}acetamide

To solution of 1,1,1-trifluoro-*N-*(2-piperazin-1-ylethyl)acetamide (2.2 g, 9.73 mmole) in DMF (5 mL) at RT was added 1 1,1-trifluoromethanesulfonic acid 6-methoxy[1,5]naphthyridin-4-yl ester (3.0 g, 9.73 mmole) and triethylamine (1.63 mL, 11.68 mmole). After 18 hr at 100 °C, the reaction solution was concentrated under vacuum and purified on silica gel (CHCl₃/MeOH, 9:1, containing 5% NH₄OH) to give the title compound as an off-white solid (2.70 g, 72 %): LC-MS (ES) m/e 384 (M + H)⁺.

### b) 2-[4-(6-methoxyquinolin-4-yl)piperazin-1-yl]etylamine

To solution of 2,2,2-trifluoro-N-{2-[4-(6-methoxyquinolin-4-yl)piperazin-1-yl] ethyl}acetamide (1.11 g, 2.9 mmole) in methanol (50 mL) at RT was added K₂CO₃ (2.0 g, 14.5 mmole) and H₂O (25 mL). After 18 hr at RT, the reaction solution was concentrated under vacuum and purified on silica gel (CHCl₃/MeOH, 9:1, containing 5% NH₄OH) to give the title compound as a thick oil (0.75 g, 90 %): ¹H NMR (400 MHz, CDCl₃) δ 8.65 (d, *J*= 5 Hz, 1H), 7.99 (d, *J*= 9 Hz, 1H), 7.36 (d, *J*= 2.8 Hz, 1H), 7.32 (m, 1H), 6.88 (d, *J* = 5 Hz, 1H), 3.95 (s, 3H), 3.25 (s, 4H), 2.89 (t, *J* = 6 Hz, 2H), 2.80 (s, 4H), 2.58 (t, *J*= 6 Hz, 2H), and 1.62 (s, 2H).

### c) N-(2-{4-[6-(methyloxy)-1,5-naphthyridin-4-yl]-1-piperazinyl}ethyl)-3-oxo-3,4-dihydro-2H-pyrido[3,2-b][1,4]thiazine-6-carboxamide

To a solution of 2-[4-(6-methoxyquinolin-4-yl)piperazin-1-yl]ethylamine (0.22 g, 0.77 mmole) in DMF (20 mL) was added HOBt (0.11 g, 0.85 mmole), EDC (0.16 g, 0.85 mmole), diisopropylethylamine (0.15 mL, 0.85 mmole) and 3-oxo-3,4-dihydro-2*H-*pyrido[3,2*-b*][1,4]thiazine-6-carboxylic acid (0.16 g, 0.77 mmole). After 12 hr at RT, the reaction contents were concentrated under vacuum and purified on silica gel (CHCl₃/MeOH containing 5% NH₄OH, 9:1) to give the title compound (0.26 g, 71 %) as an off-white solid: ¹H NMR (400 MHz, CD₃OD) 8.51 (d, *J* = 6.9 Hz, 1H), 8.25 (d, *J* = 9.2 Hz, 1 H), 7.92 (d, *J*= 7.9 Hz, 1H), 7.75 (d, *J*= 7.9 Hz, 1 H), 7.46 (d, *J* = 9.2 Hz, 1 H), 7.34 (d, *J* = 6.9 Hz, 1 H), 4.09 (s, 3H), 3.89 (m, 2H), 3.70 (m, 2H), 3.65 (m, 2H), 3.50 (m, 2H), 3.47 (m, 2H), 3.01 (m, 2H), 2.92 (m, 2H). LC-MS (ES) m/e 480 (M + H)⁺.

### Example 32

### Preparation of N-methyl-N-(2-{4-[6-(methyloxy)-1,5-naphthyridin-4-yl]-1-perazinyl}ethyl)-3-oxo-3,4-dihydro-2H-pyrido[3,2-b][1,4]thiazine-6-carboxamide

According to the procedure for Example 6, except substituting *N*-methyl-2-{4-[6-(methyloxy)-1,5-naphthyridin-4-yl]-1-piperazinyl}ethanamine (0.17 g, 0.57 mmole) for 2-[4-(6-methoxyquinolin-4-yl)piperazin-1-yl]ethylamine, the title compound (0.18g, 65 %) was prepared as an off-white solid following flash chromatography on silica gel (CHCl₃/MeOH, 9:1, containing 5% NH₄OH): ¹H NMR (400 MHz, CDCl₃) δ 8.54 Hz (m, 1 H), 8.27 Hz (m, 1 H), 8.04 (m, 1H), 7.75 (d, *J* = 7.9 Hz, 1H), 7.12 (d, J = 9.2 Hz, 1H), 6.86 (d, *J* = 5.4 Hz, 1 H), 4.05 (s, 3H), 3.79 (s, 2H), 3.70 (m, 2H), 3.52 (s, 3H), 3.17 (m, 2H), 3.02 (m, 2H), 1.95 (m, 2H), 2.87 (m, 4H). LC-MS (ES) m/e 494 (M + H)⁺.

### Example 33

### Preparation of N-methyl-N-(2-{4-[6-(methyloxy)-1,5-naphthyridin-4-yl]-1-piperazinyl}ethyl)-3-oxo-3,4-dihydro-2H-1,4-benzothiazine-6-sulfonamide

According to the procedure for Example 2, except substituting methyl 42-{4-[6-(methyloxy)-1,5-naphthyridin-4-yl]-1-piperazinyl}ethyl)amine (0.30 g, 1.0 mmole) for 2-[1-(6-methoxynaphthyridin-4-yl)piperidin-4-yl]ethylamine, the title compound (0.45g, 85 %) was prepared as an off-white solid following flash chromatography on silica gel (CHCl₃/MeOH, 9:1, containing 5% NH₄OH): ¹H NMR (400 MHz, CDCl₃) δ 8.70 (m, 1H), 8.54 Hz (d, *J* = 5.3 Hz, 1H), 8.27 Hz (d, *J* = 9.0 Hz, 1H), 7.50 (m, 2H), 7.12 (d, *J* = 9.1 Hz, 1H), 6.86 (d, *J*= 5.3 Hz, 1 H), 4.04 (s, 3H), 3.76 (s, 3H), 3.51 (m, 2H), 3.29 (m, 2H), 2.85 (m, 8H), 2.75 (m, 2H). LC-MS (ES) m/e 529 (M)⁺.

### Example 34

### Preparation of N-(2-{4-[6-(methyloxy)-1,5-naphthyridin-4-yl]-1-piperazinyl}ethyl)-3-oxo-3,4-dihydro-2H-1,4-benzothiazine-6-sulfonamide

According to the procedure for Example 2, except substituting -(2-{4-[6-(methyloxy)-1,5-naphthyridin-4-yl]-1-piperazinyl}ethyl)amine (0.15 g, 0.52 mmole) for 2-[1-(6-methoxynaphthyridin-4-yl)piperidin-4-yl]ethylamine, the title compound (0.24 g, 91 %) was prepared as an off-white solid following flash chromatography on silica gel (CHCl₃/MeOH, 9:1, containing 5% NH₄OH): ¹H NMR (400 MHz, CDCl₃) δ 8.52 Hz (d, *J*= 5.3 Hz, 1 H), 8.24 Hz (d, *J*= 9.0 Hz, 1 H), 7.50 (d , *J* = 8.2 Hz, 1H), 7.43 (d, *J*= 8.2 Hz, 1H), 7.10 (d, *J* = 9.1 Hz, 1H), 6.86 (d, *J* = 5.3 Hz, 1 H), 4.41 (m, 2H), 4.04 (s, 3H), 3.51 (s, 2H), 3.13 (m, 2H), 2.95 (m, 2H), 2.79 (s, 3H), 1.95 (m, 2H), 1.65 (m, 1H), 1.58 (m, 4H). LC-MS (ES) m/e 528 (M)⁺.

### Example 35

### Preparation of 6-{[(2-{4-[6-(methyloxy)-1,5-naphthyridin-4-yl]hexahydro-1H-1,4-diazepin-1-yl}ethyl)amino]methyl}-2H-pyrido[3,2-b][1,4]thiazin-3(4H)-one

### a) 1,1-dimethylethyl 4-(N-{[(benzyl)oxy]carbonyl}glycyl)hexahydro-1H-1,4-diazepine-1-carboxylate

1,1-dimethylethyl hexahydro-1*H*-1,4-diazepine-1-carboxylate (5.10 g., 25.50 mmole), carbobenzyloxyglycine (5.86 g., 28.00 mmole) and 1-hydroxybenzotriazole hydrate (3.80 g., 28.00 mmole) were dissolved in dichloromethane (100 mL) and treated with N,N-diisopropylethylamine (8.89 ml, 51.00 mmole) and then 1-(3-dimethylaminopropyl)-3-ethyl-carbodiimide hydrochloride (5.37 g., 28.00 mmole). The reaction was stirred under nitrogen overnight at room temperature. The reaction was then diluted with dichloromethane (250 mL) and then washed with water (2 x 250 ml) and brine (150 mL). The organic layer was dried over anhydrous potassium carbonate and concentrated *in vacuo.* The crude product was purified by silica gel chromatography with 25% ethyl acetate/hexanes to obtain the title compound ( 9.00 g., 90%) as a clear oil: LC/MS (ES) m/e 392 (M + H)⁺.

### b) Benzyl [2-(hexahydro-1H-1,4-diazepin-1-yl)-2-oxoethyl]carbamate

1,1-dimethylethyl 4-(*N*-{[(benzyl)oxy]carbonyl}glycyl)hexahydro-1*H-*1,4-diazepine-1-carboxylate (9.00 g., 23.00 mmole) was dissolved in dichloromethane (150 mL) and treated with TFA (100 mL, 1298.25 mmole) for three hours at RT. Then the reaction was concentrated *in vacuo,* dissolved in dichloromethane (100 mL) and treated with 4M HCl in dioxane (23.00 mL, 92.00 mmole) for one hour at RT. The reaction was then diluted with dichloromethane (250 mL) and then washed with 2N NaOH (2 x 250 ml) and brine (150 mL). The organic layer was dried over anhydrous potassium carbonate and concentrated *in vacuo.* The crude product was purified by silica gel chromatography with 10% methanol/chloroform, containing 5% ammonium hydroxide in the methanol, to obtain the title compound (6.30 g., 94%) as a clear oil: LC/MS (ES) m/e 292 (M + H)⁺.

### c) Benzyl [2-(hexahydro-1H-1,4-diazepin-1-yl)ethyl]carbamate

Benzyl [2-(hexahydro-1*H-*1,4-diazepin-1-yl)-2-oxoethyl]carbamate (6.30 g., 21.60 mmole) was dissolved in anhydrous THF (150 mL) and cooled to 0°C under nitrogen. The solution was then treated with a 1 M lithium aluminum hydride solution in THF ( 43.20 mL, 43.20 mmole) for one hour at 0°C and then the reaction was quenched with water (3.10 mL) and 1N NaOH (0.75 mL). The resulting suspension was filtered through Celite^{™} in a sintered glass funnel. The filtrate was dried over anhydrous potassium carbonate and concentrated *in vacuo.* The crude product was purified by silica gel chromatography with 10% methanol/chloroform, containing 5% ammonium hydroxide in the methanol, to obtain the title compound (5.00 g., 83%) as a yellow oil: LC/MS (ES) m/e 278 (M + H)⁺.

### d) Benzyl (2-{4-[6-(methyloxy)-1,5-naphthyridin-4-yl]hexahydro-1H-1,4-diazepin-1-yl}ethyl)carbamate

Benzyl [2-(hexahydro-1*H*-1,4-diazepin-1-yl)ethyl]carbamate (3.70 g., 13.33 mmole) and 6-(methyloxy)-1,5-naphthyridin-4-yl trifluoromethanesulfonate (4.10 g., 13.33 mmole) were dissolved in anhydrous dimethylformamide (10 mL), treated with diisopropylethylamine (3.50 mL, 20.00 mmole) and heated to 95°C under nitrogen over 48 hours. The solution was cooled and concentrated *in vacuo.* The crude product was purified by silica gel chromatography with 10% methanol/chloroform to obtain the title compound (2.0 g., 34%) as a brown waxy solid: LC/MS (ES) *mle* 436 (M + H)⁺.

### e) (2-{4-[6-(methyloxy)-1,5-naphthyridin-4-yl]hexahydro-1H-1,4-diazepin-1-yl} ethyl)amine hydrochloride

Benzyl (2-{4-[6-(methyloxy)-1,5-naphthyridin-4-yl]hexahydro-1*H*-1,4-diazepin-1-yl}ethyl)carbamate (1.00 g., 2.30 mmole), 4M HCl in dioxane (0.60 mL, 2.30 mmole) and Pearlman's catalyst (0.35 g.) were suspended in ethanol and placed under 50 p.s.i of hydrogen for two hours. The suspension was filtered through Celite^{™} in a sintered glass funnel and the filtrate was concentrated *in vacuo* to obtain the title compound (0.60 g., 87%) as a yellow solid: LC/MS (ES) *m*/*e* 302 (M + H)⁺

### f) 6-{[(2-{4-[6-(methyloxy)-1,5-naphthyridin-4-yl]hexahydro-1H-1,4-diazepin-1-yl} ethyl)amino]methyl}-2H-pyrido[3,2-b][1,4]thiazin-3(4H)-one

(2-{4-[6-(methyloxy)-1,5-naphthyridin-4-yl]hexahydro-1*H-*1,4-diazepin-1-yl}ethyl)amine hydrochloride (0.60 g, 1.77 mmole) and 3-oxo-3,4-dihydro-2*H-*pyrido[3,2-b][1,4]thiazine-6-carbaldehyde (0.35 g, 1.77 mmole) were dissolved in a mixture of dichloromethane (30 mL) and ethanol (20 mL) and treated with anhydrous sodium sulfate (0.10 g.) and diisopropylethylamine (0.62 mL), 3.55 mmole) under nitrogen at room temperature over eighteen hours. The reaction was then treated with sodium borohydride (0.070 g., 1.77 mmole) for one hour at room temperature. The solution was then concentrated *in vacuo* and purified by silica gel chromatography with 10% methanol/chloroform. The compound was dissolved in dichloromethane (40 mL) and treated with 4M HCl in dioxane (1.30 mL) for 30 minutes and then concentrated *in vacuo* to obtain the title compound ( 0.20 g., 24%) as an off white solid: ¹H NMR (400 MHz, DMSO-d₆) δ 8.60 (d, J = 5.6 Hz, 1H), 8.44 Hz (d, *J* = 9.0 Hz, 1H), 7.95 Hz (d, *J* = 8.9 Hz, 1 H), 7.55 (d, J = 9.0 Hz, 1 H), 7.27 (d, *J* = 5.6 Hz, 1 H), 7.17 (d, *J* = 5.3 Hz, 1 H), 4.25 (s, 2H), 4.04 (s, 3H), 3.40-3.75 (m, 16H). LC/MS (ES) m/e 480 (M + H)⁺.

### Example 36

### Preparation of N-(2-{4-[6-(methyloxy)-1,5-naphthyridin-4-yl]hexahydro-1H-1,4-diazepin-1-yl}ethyl)-3-oxo-3,4-dihydro-2H-1,4-benzothiazine-6-sulfonamide trihydrochloride

### a) N-(2-{4-[6-(methyloxy)-1,5-naphthyridin-4-yl]hexahydro-1H-1,4-diazepin-1-yl} ethyl)-3-oxo-3,4-dihydro-2H-1,4-benzothiazine-6-sulfonamide trihdyrochloride

(2-{4-[6-(methyloxy)-1,5-naphthyridin-4-yl]hexahydro-1*H*-1,4-diazepin-1-yl}ethyl)amine hydrochloride (0.32 g., 0.96 mmole) and 3-oxo-3,4-dihydro-2*H-*pyrido[3,2-*b*][1,4]thiazine-6-sulfonyl chloride (0.25 g., 0.96 mmole) were dissolved in dichloromethane (25 mL) and treated with diisopropylethylamine (0.34 mL, 1.92 mmole) under nitrogen at room temperature over eighteen hours. The solution was then concentrated *in vacuo* and purified by silica gel chromatography with 10% methanol/chloroform. The compound was dissolved in dichloromethane (40 mL) and treated with 4M HCl in dioxane (0.20 mL) for 30 minutes and then concentrated *in vacuo* to obtain the title compound (0.22 g., 38%) as an off white solid: ¹H NMR (400 MHz, DMSO-d₆) δ 8.55 (d, J = 7.1 Hz, 1 H), 8.44 Hz (d, *J* = 9.1 Hz, 1H), 7.55 (m, 2H), 7.41 (s, 1 H), 7.39 (d, *J* = 9.0 Hz, 1 H), 7.11 (d, *J* = 7.1 Hz, 1 H), 4.00 (s, 3H), 3.61 (m, 2H), 3.61 (m, 4H), 3.31 (m, 4H), 3.15 (m, 2H), 2.62 (m, 2H), 2.31 (m, 2H). LC/MS (ES) m/e 529 (M + H)⁺.

### Example 37

### Preparation of 6-{[(2-{(1S,4S)-5-[6-(methyloxy)-1,5-naphthyridin-4-yl}-2,5-diazabicyclo[2.2.1]hept-2-yl}ethyl)amino]methyl}-2H-pyrido[3,2-b][1,4]thiazin-3(4H)-one trihydrochloride

### a) 1,1-dimethylethyl (1S,4S)-5-(N-{[benzyloxy]carbonyl}glycyl)-2,5-diazabicyclo[2.2.1]heptane-2-carboxylate

1,1-dimethylethyl (1*S*,4*S*)-2,5-diazabicyclo[2.2.1]heptane-2-carboxylate (1.00 g., 5.0 mmole), carbobenzyloxyglycine (1.05 g., 5.0 mmole) and 1-hydroxybenzotriazole hydrate (0.75 g., 5.5 mmole) were dissolved in dichloromethane (25 mL) and treated with N,N-diisopropylethylamine (0.97 ml, 10.0 mmole) and then 1-(3-dimethylaminopropyl)-3-ethyl-carbodiimide hydrochloride (1.06 g., 5.5 mmole). The reaction was stirred under nitrogen overnight at room temperature. The reaction was diluted with dichloromethane (50 mL) and then washed with water (2 x 50 ml) and brine (50 mL). The organic layer was dried over anhydrous potassium carbonate and concentrated *in vacuo.* The crude product was purified by silica gel chromatography with 10% methanol/chloroform to obtain the title compound (1.80 g., 92%) as a clear oil: LC/MS (ES) m/e 390 (M + H)⁺.

### b) 1,1-dimethylethyl (1S,4S)-5-[2-({[benzyloxyl]carbonyl}amino)ethyl]-2,5-diazabicyclo[2.2.1]heptane-2-carboxylate

1,1-dimethylethyl (1*S*,4*S*)-5-(*N*-{[benzyloxy]carbonyl}glycyl)-2,5-diazabicyclo[2.2.1]heptane-2-carboxylate (1.80 g., 4.6 mmole) was dissolved in anhydrous THF (50 mL) and cooled to 0°C under nitrogen. The solution was then treated with a 1 M lithium aluminum hydride solution in THF (9.20 mL, 9.20 mmole) for one hour at 0°C and then the reaction was quenched with water (0.70 mL) and 1 N NaOH (0.20 mL). The resulting suspension was filtered through Celite^{™} in a sintered glass funnel. The filtrate was dried over anhydrous potassium carbonate and concentrated *in vacuo.* The crude product was purified by silica gel chromatography with 10% methanol/chloroform, containing 5% ammonium hydroxide in the methanol, to obtain the title compound (0.90 g., 52%) as a clear oil: LC/MS (ES) *m*/*e* 376 (M + H)⁺.

### c) benzyl{2-[(1S,4S)-2,5-diazabicyclo[2.2.1]hept-2-yl]ethyl}carbamate hydrochloride

1,1-dimethylethyl (1*S*,4*S*)-5-[2-({[benzyloxy]carbonyl}amino)ethyl]-2,5-diazabicyclo[2.2.1]heptane-2-carboxylate (0.90 g., 2.40 mmole) was dissolved in dichloromethane (25 mL) and treated with 4M HCl in dioxane (10.00 mL, 40.00 mmole) for three hours at room temperature. The solution was concentrated *in vacuo* to obtain the title compound (0.66 g., 88%) as an off white solid: LC/MS (ES) m/e 276 (M + H)⁺.

### d) benzyl (2-{(1S,4S)-5-[6-(methyloxy)-1,5-naphthyridin-4-yl]-2,5-diazabicyclo[2.2.1]hept-2-yl}ethyl)carbamate

Benzyl {2-[(1*S*,4*S*)-2,5-diazabicyclo[2.2.1]hept-2-yl]ethyl}carbamate hydrochloride (0.66 g, 2.11 mmole) and 6-(methyloxy)-1,5-naphthyridin-4-yl trifluoromethanesulfonate (0.74 g, 2.40 mmole) were dissolved in anhydrous dimethylformamide (10 mL), treated with triethylamine (1.67 mL, 12.00 mmole) and heated to 90°C under nitrogen over 18 hours. The solution was cooled and concentrated *in vacuo.* The crude product was purified by silica gel chromatography with 10% methanol/chloroform to obtain the title compound (0.25 g, 27%) as a brown oil: LC/MS (ES) m/e 434 (M + H)⁺.

### e) (2-{(1S,4S)-5-[6-(methyloxy)-1,5-naphthyridin-4-yl]-2,5-diazabicydo[2.2.1]hept-2-yl}ethyl)amine hydrochloride

Benzyl (2-{(1S,4S)-5-[6-(methyloxy)-1,5-naphthyridin-4-yl]-2,5-diazabicyclo[2.2.1]hept-2-yl}ethyl)carbamate (0.25 g, 0.57 mmole), 4M HCl in dioxane (0.14 mL, 0.57 mmole) and Pearlman's catalyst (0.20 g.) were suspended in ethanol and placed under 50 p.s.i of hydrogen for three hours. The suspension was filtered through Celite^{™} in a sintered glass funnel and the filtrate was concentrated *in vacuo* to obtain the title compound (0.10 g., 52%) as a white solid: LC/MS (ES) *m*/*e* 300 (M + H)⁺

### f) 6-{[(2-{(1S,4S)-5-[6-(methyloxy)-1,5-naphthyridin-4-yl]-2,5-diazabicyclo[2.2.1]hept-2-yl}ethyl)amino]methyl}-2H-pyrido[3,2-b][1,4]thiazin-3(4H)-one trihydrochloride

(2-{(1*S*,4*S*)-5-[6-(methyloxy)-1,5-naphthyridin-4-yl]-2,5-diazabicyclo[2.2.1]hept-2-yl}ethyl)amine hydrochloride (0.10 g., 0.30 mmole) and 3-oxo-3,4-dihydro-2*H-*pyrido[3,2-b][1,4]thiazine-6-carbaldehyde (0.060 g, 0.030 mmole) were dissolved in a mixture of dichloromethane (10 mL) and ethanol (10 mL) and treated with anhydrous sodium sulfate (0.10 g.) and diisopropylethylamine (0.05 mL, 0.060 mmole) under nitrogen at room temperature over eighteen hours. The reaction was then treated with sodium borohydride (0.011 g., 0.30 mmole) for one hour at room temperature. The solution was then concentrated *in vacuo* and purified by silica gel chromatography with 10% methanol/chloroform. The compound was dissolved in dichloromethane (20 mL) and treated with 4M HCl in dioxane (0.25 mL) for 30 minutes and then concentrated *in vacuo* to obtain the title compound (0.08 g., 27%) as an off white solid: ¹H NMR (400 MHz, DMSO-d₆) δ 10.97 (s, 1H), 9.82 (br s, 1 H), 8.55 (s, 1H), 8.37 (s, 1H), 7.90 (d, J = 7.9 Hz, 1 H), 7.55 (d, J = 8.6 Hz, 1H), 7.24 (d, J = 8.1 Hz, 1H), 6.90 (m, 1H), 4.78 (s, 1 H), 4.26 (s, 2H), 4.02 (s, 3H), 3.60 (s, 2H), 3.17-3.57 (s, 11H) LC/MS (ES) m/e 478 (M + H)⁺.

### Example 38

### Preparation of 6-({2-[4-Hydroxy-1-(6-methoxy-[1,5]naphthyridin-4-yl)-pipedin-4-yl]-ethylamino}-methyl)-4-H-pyrido[3,2-b][1,4]thiazin-3-one trihydrochloride

### a) 2,2,2-trifluoro-N-(2-{4-hydroxy-1-[6-(methyloxy)-1,5-naphthyridin-4-yl]-4-piperidinyl}ethyl)acetamide

2,2,2-Trifluoro-*N-*[2-(4-hydroxy-4-piperidinyl)ethyl]acetamide (0.95 g, 3.95 mmole) was dissolved in DMF (7 mL) and 1,1,1-trifluoro-methanesulfonic acid 6-methoxy-[1,5]naphthyridin-4-yl ester (1.22 g, 3.95 mmol) and triethylamine (2.8 mL, 19.75 mmol) was added to the reaction mixture. The reaction mixture was heated at 90 °C for 18 h and then concentrated in vacuo. The crude mixture was purified by silica gel column chromatography (50% EtOAc/hexanes - 5% methanol/dichloromethane to yield the title compound (1.46 g, 90 %) as a light brown oil: ¹H NMR (400 MHz, CD₃OD) δ 8.25 Hz (d, 1H, J = 5.1 Hz), 7.94 Hz (d, 1H, J = 9.1 Hz), 6.95 (d, 1H, J = 9.1 Hz), 6.76 Hz (d, 1H, 5.1 Hz), 3.91 (m, 3H), 3.89 (s, 3H), 3.26 (m, 4H), and 1.73 (m, 5H). LC-MS (ES) m/e 399.0 (M + H).

### b) 4-(2-Amino-ethyl)-1-(6-methoxy-[1,5]naphthyridin-4-yl)-piperidin-4-ol

To a round bottom flask equipped with a stirring bar was added 2,2,2-trifluoro-N-{2-[4-hydroxy-1-(6-methoxy-[1,5]naphthyridin-4-yl)-piperidin-4-yl]-ethyl}acetamide (1.4 g, 28.1 mmole) in methanol (150 mL) and water (30 mL). To this mixture was added potassium carbonate (14.1 mmol, 1.94 g). After 18h, the reaction mixture was filtered through a pad of Celite and concentrated in vacuo to obtain the title compound as a pale yellow oil: LC-MS (ES) m/e 303.2 (M + H).

### c) 6-({2-[4-Hydroxy-1-(6-methoxy-[1,5]naphthyridin-4-yl)-piperidin-4-yl]-ethylamino}-methyl)-4-H-pyrido[3,2-b][1,4]thiazin-3-one trihydrochloride

To a stirred solution 4-(2-amino-ethyl)-1-(6-methoxy-[1,5]naphthyridin-4-yl)-piperidin-4-ol (0.435 g, 1.44 mmole) in 40 mL of absolute ethanol and 20 mL of anhydrous DCM was added 3-oxo-3,4-dihydro-2H-pyrido[3,2-b][1,4]thiazine-6-carboxaldehyde (0.279 g, 1.44 mmol) and a spatula of sodium sulfate. The reaction was stirred vigorously under nitrogen for 18 h then sodium borohydride (1.44 mmol, 55 mg) was added and the reaction was stirred for an additional 2 h. The reaction mixture was filtered to remove the solids and the solvent evaporated to a light brown solid. The crude product was purified by silica gel column chromatography eluting with 10 % methanol/dichloromethane (with 5% NH₄OH in methanol) to afford the title compound as the free base (175 mg, 26 %). The product was dissolved in DCM and 3 eq. of 1 N HCl in diethyl ether (0.364 mmol, 0.364 mL) was added. The tris-HCl salt precipitated from the solvent and the solvent was removed to afford the title compound as a off white solid (185 mg): ¹H NMR (400 MHz, CDCl₃) δ 8.54 Hz (d, 1H, J = 5.3 Hz), 8.17 Hz (d, 1H, J = 9.1 Hz), 7.61 (d, 1 H, J = 7.8 Hz), 7.08 (d, 1H, J = 9.1 Hz), 6.97 (d, 1H, J = 7.8 Hz), 6.90 (d, 1H, J = 5.3 Hz), 4.09 (m, 2H), 4.06 (s, 3H), 3.86 (s, 2H), 3.51 (m, 2H), 3.41 (m, 2H), 3.04 (m, 2H), 1.90 (m, 4H), and 1.76 ( m, 2H). LC-MS (ES) m/e 481.2 (M + H)⁺.

### Example 39

### Preparation of 6-({2-[4-Hydroxy-1-(6-methoxy-[1,5]naphthyridin-4-yl)-piperidin-4-yl]-ethylamino}-methyl)-4-H-pyrido[3,2-b][1,4]oxazin-3-one

According to the procedure for Example 13, except substituting 3-oxo-3,4-dihydro-2*H*-pyrido[3,2*-b*][1,4]oxazine-6-carboxaldehyde (0.18 g, 1.0 mmole) for 3-oxo-3,4-dihydro-2*H-*pyrido[3,2*-b*][1,4]thiazine-6-carboxaldehyde, the title compound (0.115 mg, 24 %) was prepared as an off-white solid following flash chromatography on silica gel (CHCl₃/MeOH, 9:1, containing 5% NH₄OH): ¹H NMR (400 MHz, CDCl₃) δ 8.54 Hz (d, 1H, *J* = 5.3 Hz), 8.18 Hz (d, 1H, J = 9.1 Hz), 7.24 (d, 1H, J = 8.1 Hz), 7.08 (d, 1H, J = 9.1 Hz), 6.94 (d, 1H, J = 8.1 Hz), 6.90 (d, 1H, J = 5.3 Hz), 4.67 (s, 2H), 4.09 (m, 2H), 4.07 (s, 3H), 3.85 (s, 2H), 3.51 (m, 1H), 3.41 (m, 2H), 3.04 (m, 2H), 1.90 (m, 4H), and 1.77 (m, 2H). LC-MS (ES) m/e 465.2 (M + H)⁺.

### Example 40

### Preparation of N-(2-{4-hydroxy-1-[6-(methyloxy)-1,5-naphthyridin-4-yl]-4-piperidinyl}ethyl)-3-oxo-3,4-dihydro-2H-1,4-benzothiazine-6-sulfonamide

To a stirred solution of 2-{4-[6-(methyloxy)-1,5-naphthyridin-4-yl]-1-piperazinyl}ethanol (0.19 g, 0.63 mmole) in CH₂Cl₂ (50 mL) at RT was added 3-oxo-3,4-dihydro-2H-benzo[1,4]thiazine-6-sulfonyl chloride (0.16 g, 0.63 mmole). After 24h at RT, the reaction solution was concentrated in vacuo. The remaining solid was purified on silica (CHCl₃/MeOH containing 5% NH₄OH, 9:1) to give the title compound as an off-white solid (0.13 g, 40%): ¹H NMR (400 MHz, CDCl₃/CD₃OD) δ 8.54 Hz (d, 1H, J = 5.3 Hz), 8.21 Hz (d, 1H, J = 9.1 Hz), 7.57 (s, 2H), 7.47 (s, 1H), 7.21 (d, J = 9.1 Hz, 1H), 7.05 (d, J = 5.3 Hz, 1H), 4.14 (s, 3H), 4.12 (s, 2H), 3.57 (s, 2H), 3.52 (m, 2H), 3.48 (m, 3H), 3.22 (m, 2H), 1.98 (m, 2H), and 1.87 (m, 4H). LC-MS (ES) m/e 530.2 (M + H)⁺.

### Example 41

### Antimicrobial Activity Assay:

Whole-cell antimicrobial activity was determined by broth microdilution using the National Committee for Clinical Laboratory Standards (NCCLS) recommended procedure, Document M7-A6, "Methods for Dilution Susceptibility Tests for Bacteria that Grow Aerobically". The compounds were tested in serial two-fold dilutions ranging from 0.016 to 16 mcg/mL.

Compounds were evaluated against a panel of Gram-positive organisms, including *Staphylococcus aureus* WCUH29, *Staphylococcus epidermis, Streptococcus pneumoniae* 1629, *Streptococcus pyogenes* CN 10, *Enterococcus faecalis* 2 and *Enterococcus faecium.*

In addition, compounds were evaluated against a panel of Gram-negative strains including *Haemophilus influenzae* NEMC1, E. *coli* 7623, and *Moraxella catarrhalis* Ravasio.

The minimum inhibitory concentration (MIC) was determined as the lowest concentration of compound that inhibited visible growth. A mirror reader was used to assist in determining the MIC endpoint.

One skilled in the art would consider any compound with a MIC of less than 20 µg/mL to be a potential lead compound. Compounds of the present invention have MIC's ≤20 µg/ml versus all the organisms named above.

For instance, Examples 1 to 40, respectively, as identified in the present application had MIC's ≤20 µg/ml versus or when screened against the aforementioned organisms above.

### Example 42

### Rat Infection Model

Certain compounds of this invention were tested in the rat infection model. Specific pathogen-free male Sprague-Dawley CD rats were used for all bacterial strains. Each therapy group consists of 5 animals. Infection was carried out by intrabronchial instillation of 100 µl bacterial suspension for *H.influenzae* H128, and 50 µl of bacterial suspension for *S.pneumoniae* 1629 via non-surgical intubation. All compounds were administered at 1, 7, 24 and 31 hour post infection via oral gavage. In each experiment, an additional group of animals was included and served as untreated infected controls. Approximately 17 hour after the end of therapy, the animals were killed and their lungs excised and enumeration of the viable bacteria was conducted by standard methods. The lower limit of detection was 1.7 log10 CFU/lungs.
In vivo, activity was observed in infection models in rats versus S. *pneumoniae* 1629 at doses ranging from 25-100 mg/Kg with oral dosing and for some compounds versus *H*. *influenzae* H128 at doses from 25-100 mg/Kg with oral dosing. Certain formula (I) compounds showed a greater than 2 log drop in viable counts in the lungs compared to non-treated controls versus S. *pneumoniae* 1629. Certain compounds of formula (I) showed greater than a 4 log drop in viable counts in the lungs compared to non-treated controls versus *H. influenzae H* 128. The compounds of this invention are particularly interesting due to their low toxicity with no toxicity being observed in rats with dosing twice daily for 2 days at 50mg/Kg.

## Claims

1. A compound of formula (I) 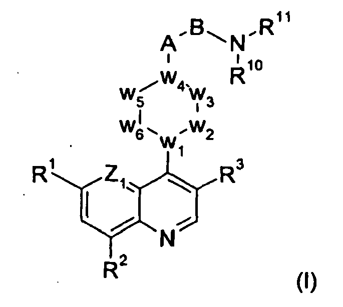 wherein:
Z₁ is N or CR^{1a};
R¹ and R^{1a} are independently hydrogen; hydroxy; (C₁₋₆)alkoxy unsubstituted or substituted by (C₁₋₆)aₗkoxy, hydroxy, amino, piperidyl, guanidino or amidino any of which is unsubstituted or N-substituted by one or two (C₁₋₆)alkyl, acyl, (C₁₋₆)alkylsulphonyl, CONH₂, hydroxy, (C₁₋₆)alkylthio, heterocyclylthio, heterocyclyloxy, arylthio, aryloxy, acylthio, acyloxy or (C₁₋₆)alkylsulphonyloxy; (C₁₋₆)alkoxy-substituted (C₁₋₆)alkyl; halogen; (C₁₋₆)alkyl; (C₁₋₆)alkylthio; trifluoromethyl; trifluoromethoxy; nitro; azido; cyano; acyl; acyloxy; acylthio; (C₁₋₆)alkylsulphonyl; (C₁₋₆)alkylsulphoxide; arylsulphonyl; arylsulphoxide; or an amino, piperidyl, guanidino or amidino group unsubstituted or N-substituted by one or two (C₁₋₆)alkyl, acyl or (C₁₋₆)alkylsulphonyl groups; or R¹ and R^{1a} may together form ethylenedioxy;
provided that when Z₁ is CR^{1a} then R¹ is not H;
R² is H or halogen;
provided that when Z₁ is N, then R² is H;
R³ is hydrogen; halogen; hydroxy; cyano; CF₃; nitro; azido acyl; aryl; heteroaryl; CO₂H; substituted by one or two (C₁₋₆)alkyl, acyl, (C₁₋₆)alkylsulphonyl, CONH₂, hydroxy, (C₁₋₆)alkylthio, heterocyclylthio, heterocyclyloxy, arylthio, aryloxy, acylthio, acyloxy or (C₁₋₆)alkylsulphonyloxy; (C₁₋₆)alkoxy unsubstituted or substituted by one or two (C₁₋₆)alkoxy, hydroxy, amino, piperidyl, guanidino or amidino any of which is unsubstituted or N-substituted by one or two (C₁₋₆)alkyl, acyl, (C₁₋₆)alkylsulphonyl, CONH₂, hydroxy, (C₁₋₆)alkylthio, heterocyclylthio, heterocyclyloxy, arylthio, aryloxy, acylthio, acyloxy or (C₁₋₆)alkylsulphonyloxy; (C₃₋₇)cycloalkyl; (C₁₋₆)alkoxy-substituted (C₁₋₆)alkyl; (C₁₋₆)alkylthio; trifluoromethoxy; (C₁₋₆)alkylsulphonyl; (C₁₋₆)alkylsulphoxide; arylsulphonyl; or arylsulphoxide; or an amino, piperidyl, guanidino or amidino group unsubstituted or N-substituted by one or two (C₁₋₆)alkyl, acyl or (C₁₋₆)alkylsulphonyl groups;
w₁ is N, C, or CR⁴;
w₂ is C=O, CR⁴, or CR⁴R⁵;
w₃ is C=O or CR⁴R⁵;
w₄ is N or CR⁴;
w₅ is C=O or CR⁴R⁵;
w₆ is C=O, CR⁴, or CR⁴R⁵;
or one of w₂, w₃, w₅ and w₆ is CR⁴R⁵CR⁴R⁵ and the others are defined as above;
wherein
each R⁴ and R⁵ is independently hydrogen; halogen; hydroxy; cyano; CF₃; nitro; azido; acyl; aryl; heteroaryl; CO₂H; acyloxy; acylthio; (C₁₋₆)alkyl unsubstituted or substituted by one or two (C₁₋₆)alkoxy, hydroxy, amino, piperidyl, guanidino or amidino any of which is unsubstituted or N-substituted by one or two (C₁₋₆)alkyl, acyl, (C₁₋₆)alkylsulphonyl, CONH₂, hydroxy, (C₁₋₆)alkylthio, heterocyclylthio, heterocyclyloxy, arylthio, aryloxy, acylthio, acyloxy or (C₁-₆)alkylsulphonyloxy; (C₁₋₆)alkoxy unsubstituted or substituted by one or two (C₁₋₆)alkoxy, hydroxy, amino, piperidyl, guanidino or amidino any of which is unsubstituted or N-substituted by one or two (C₁₋₆)alkyl, acyl, (C₁₋₆)alkylsulphonyl, CONH₂, hydroxy, (C₁₋₆)alkylthio, heterocyclylthio, heterocyclyloxy, arylthio, aryloxy, acylthio, acyloxy or (C₁₋₆)alkylsulphonyloxy; (C₃₋₇)cycloalkyl; (C₁₋₆)alkoxy-substituted (C₁₋₆)alkyl; (C₁₋₆)alkylthio; trifluoromethoxy; (C₁₋₆)alkylsulphonyl; (C₁₋₆)alkylsulphoxide; arylsulphonyl; or arylsuphoxide; or an amino, piperidyl, guanidino or amidino group unsubstituted or N-substituted by one or two (C₁₋₆)alkyl, acyl or (C₁₋₆)alkylsulphonyl groups; or two R⁵ groups are joined together to form bicycloheptane;
A is CR⁶R⁷ or C(O);
B is CR⁸R⁹ or C(O);
R⁶, R⁷, R⁸, and R⁹ are independently hydrogen; halogen; hydroxy; cyano; CF₃; nitro; azido; acyl; aryl; heteroaryl; CO₂H; acyloxy; acylthio; (C₁₋₆)alkyl unsubstituted or substituted by one or two (C₁₋₆)alkoxy, hydroxy, amino, piperidyl, guanidino or amidino any of which is unsubstituted or N-substituted by one or two (C₁₋₆)alkyl, acyl, (C₁₋₆)alkylsulphonyl, CONH₂, hydroxy, (C₁₋₆)alkylthio, heterocyclylthio, heterocyclyloxy, arylthio, aryloxy, acylthio, acyloxy or (C₁₋₆)alkylsulphonyloxy; (C₁₋₆)alkoxy unsubstituted or substituted by one or two (C₁₋₆)alkoxy, hydroxy, amino, piperidyl, guanidino or amidino any of which is unsubstituted or N-substituted by one or two (C₁₋₆)alkyl, acyl, (C₁₋₆)alkylsulphonyl, CONH₂, hydroxy, (C₁₋₆)alkylthio, heterocyclylthio, heterocyclyloxy, arylthio, aryloxy, acylthio, acyloxy or (C₁₋₆)alkylsulphonyloxy; (C₃₋₇)cycloalkyl; (C₁₋₆)alkoxy-substituted(C₁₋₆)alkyl; (C₁₋₆)alkylthio; trifluoromethoxy; (C₁₋₆)alkylsulphonyl; (C₁₋₆)alkylsulphoxide; arylsulphonyl; or arylsulphoxide; or an amino, piperidyl, guanidino or amidino group unsubstituted or N-substituted by one or two (C₁₋₆)alkyl, acyl or (C₁₋₆)alkylsulphonyl groups;
R¹⁰ is hydrogen; aryl; heteroaryl; (C₁₋₆)alkyl unsubstituted or substituted by one or two (C₁₋₆)alkoxy, acyloxy, carboxy, hydroxy, amino, piperidyl, piperazinyl, morpholino, guanidino, or amidino, any of which is unsubstituted or N-substituted by one or two aryl, heteroaryl, halogen, cyano, CF₃, unsubstituted (C₁₋₆)alkyl, acyl, (C₁₋₆)alkylsulphonyl, arylsulphonyl, hydroxy, (C₁₋₆)alkylthio, heterocyclylthio, heterocyclyloxy, arylthio, aryloxy, acylthio, acyloxy, or (C₁₋₆)alkylsulphonyloxy, so long as the substitution does not lead to an unstable compound; (C₁₋₆)alkylcarbonyl; (C₂₋₆)alkenylcarbonyl; (C₁₋₆)alkoxycarbonyl; CO₂H; or CF₃;
R¹¹ is a group -U-R¹² where R¹² is a substituted or unsubstituted bicyclic carbocyclic or heterocyclic ring system (A): 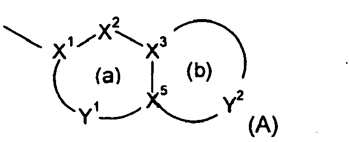 containing up to four heteroatoms in each ring in which
at least one of rings (a) and (b) is aromatic;
X¹ is C or N when part of an aromatic ring or CR¹⁴ when part of a non aromatic ring;
X² is N, NR¹³, O, S(O)ₓ, CO or CR¹⁴ when part of an aromatic or non-aromatic ring or may in addition be CR¹⁴R¹⁵ when part of a non aromatic ring;
X³ and X⁵ are independently N or C;
Y¹ is a 0 to 4 atom linker group each atom of which is independently selected from N, NR¹³, O, S(O)ₓ, CO and CR¹⁴ when part of an aromatic or non-aromatic ring or may additionally be CR¹⁴R¹⁵ when part of a non aromatic ring,
Y² is a 2 to 6 atom linker group, each atom of Y² being independently selected from N, NR¹³, O, S(O)ₓ, CO and CR¹⁴ when part of an aromatic or non-aromatic ring or may additionally be CR¹⁴R¹⁵ when part of a non aromatic ring; each of R¹⁴ and R¹⁵ is independently selected from: H; (C₁₋₄)alkylthio; halo; (C₁₋₄)alkyl; (C₂₋₄)alkenyl; hydroxy; hydroxy(C₁₋₄)alkyl; mercapto(C₁₋₄)alkyl; (C₁₋₄)alkoxy; trifluoromethoxy; nitro; cyano; carboxy; amino or aminocarbonyl unsubstituted or substituted by (C₁₋₄)alkyl;
each R¹³ is independently H; trifluoromethyl; (C₁₋₄)alkyl unsubstituted or substituted by hydroxy, carboxy, (C₁₋₄)alkoxy, (C₁₋₆)alkylthio, halo or trifluoromethyl; (C₂₋₄)alkenyl; or aminocarbonyl wherein the amino group is optionally substituted (C₁₋₄)alkyl;
each x is independently 0, 1 or 2;
U is CO, SO₂, CH₂, or GR¹⁶R¹⁷;
R¹⁶ and R¹⁷ are independently selected from H; aryl; heteroaryl; (C₁₋₆)alkyl; (C₁₋₆)alkyl substituted by (C₁₋₆)alkoxy, hydroxy, amino, piperidyl, piperazinyl, morpholino, guanidino, or amidino, any of which is substituted or N-substituted by one or two H, aryl, heteroaryl, halogen, cyano, CF₃, (C₁₋₆)alkyl, acyl, (C₁₋₆)alkylsulphonyl, arylsulphonyl, hydroxy, (C₁₋₆)alkylthio, heterocyclylthio, heterocyclyloxy, arylthio, aryloxy, acylthio, acyloxy, or (C₁₋₆)alkylsulphonyloxy, provided that the substitution does not lead to an unstable compound; (C₁₋₆)alkoxy-substituted (C₁₋₆)alkyl; hydroxy-substituted(C₁₋₆)alkyl; amino-substituted (C₁₋₆)alkyl, which is N-substituted by one or two (C₁₋₆)alkyl, acyl, (C₁₋₆)alkylsulphonyl, or arylsulphonyl; (C₁₋₆)alkylcarbonyl; (C₂₋₆)alkenylcarbonyl; (C₁₋₆)alkoxycarbonyl; CO₂H; or CF₃; or
a pharmaceutically acceptable salt thereof.

2. A compound according to claim 1, wherein R¹ is F, Cl, OCH₃, methyl, or SCH₃.

3. A compound according to claim 1 or 2, wherein R^{1a} is H, OCH₃, or OCH₂CH₂OCH₃.

4. A compound according to any preceding claim, wherein R² is H or F.

5. A compound according to any preceding claim, wherein R³ is Cl or F.

6. A compound according to any preceding claim, wherein w₁ is N or CR⁴, w₂, w₃, w₅, and w₆ are CR⁴R⁵, each R⁴ is independently H, methyl, OH, -COOH, NH₂, OCH₃ or CH₂OH and R⁵ is H.

7. A compound according to any preceding claim, wherein A is CR⁶R⁷, B is CR⁸R⁹, R⁶ and R⁸ are H, R⁷ is H or OH and R⁹ is H or OH.

8. A compound according to any preceding claim, wherein the group -U- is - CH2-.

9. A compound according to any preceding claim, wherein R¹⁰ is H.

10. A compound according to any preceding claim, wherein R¹² is:
benzo[1,2,5]thiadiazo)-5-yl;
4H-benzo[1,4] thiazin-3-one-6-yl;
2,3-dihydro-benzo[1,4]dioxin-6-yl;
benzo[1,2,3]thiadiazol-5-yl;
3-oxo-3,4-dihydro-2H-benzo[1,4]oxazin-6-yl;
7-fluoro-3-oxo-3,4-dihydro-2H-benzo[1,4] oxazin-6-yl;
2-oxo-2,3-dihydro-1H-pyrido[2,3-b][1,4]thiazin-7-yl;
2,3-dihydro-[1,4]dioxino[2,3-c]pyridin-7-yl;
3-oxo-3,4-dihydro-2H-pyrido[3,2-b][1,4]oxazin-6-yl;
[1,2,3]thiadiazolo[5,4-b]pyridin-6-yl;
3-oxo-3,4-dihydro-2*H*-pyrido[3,2-*b*][1,4]thiazin-6-yl;
7-chloro-3-oxo-3,4-dihydro-2*H*-pyrido[3,2-*b*][1,4]thiazin-6-yl;
7-fluoro-3-oxo-3,4-dihydro-2*H*-pyrido[3,2-*b*][1,4]thiazin-6-yl; or
2-oxo-2,3-dihydro-1*H*-pyrido[3,4-*b*][1,4]thiazin-7-yl.

11. A compound according to claim 1, wherein the compound is:
6-({2-[1-(6-methoxyquinolin-4-yl)piperidin-4-yl]ethylamino}methyl)-4*H*-pyrido[3,2-*b*][1,4]oxazin-3-one;
6-({2-[1-(6-methoxyquinolin-4-yl)piperidin-4-yl]ethylamino}methyl)-4*H*-pyrido[3,2-*b*][1,4]thiazin-3-one;
(2,3-dihydro-[1,4]dioxino[2,3-c]pyridin-7-ylmethyl)-{2-[1-(6-methoxyquinolin-4-yl)piperidin-4-yl]ethyl}amine;
6-({2-[1-(6-methoxynaphthyridin-4-yl)piperidin-4-yl]ethylamino} methyl)-4*H-*pyrido[3,2-*b*][1,4]oxazin-3-one;
6-({2-[1-(6-methoxynaphthyridin-4-yl)piperidin-4-yl]ethylamino} methyl)-4*H-*pyrido[3,2-*b*][1,4]thiazin-3-one;
(2,3-dihydro-[1,4]dioxino[2,3-c]pyridin-7-ylmethyl)-{2-[1-(6-methoxynaphthyridin-4-yl)piperidin-4-yl]ethyl}amine;
6-({2-[1-(3-chloro-6-methoxy-[1,5]quinolin-4-yl)phenyl] ethylamino}methyl)-4*H-*pyrido[3,2-*b*][1,4]oxazin-3-one;
6-({2-[1-(3-chloro-6-methoxy-[1,5]quinolin-4-yl)phenyl] ethylamino} methyl)-4*H-*pyrido[3,2-*b*][1,4]thiazin-3-one;
{2-[1-(3-chloro-6-methoxyquinolin-4-yl)piperidin-4-yl]ethyl}(2,3-dihydro[1,4]dioxino[2,3-*c*]pyridin-7-ylmethyl)amine;
6-({2-[1-(3-chloro-6-methoxy-[1,5]naphthyridin-4-yl)phenyl] ethylamino}methyl)-4*H-*pyrido[3,2-*b*][1,4]oxazin-3-one;
6-({2-[1-(3-chloro-6-methoxy-[1,5]naphthyridin-4-yl)phenyl] ethylamino}methyl)-4*H* pyrido[3,2-*b*][1,4]thiazin-3-one;
{2-[1-(3-chloro-6-methoxynaphthyridin-4-yl)piperidin-4-yl]ethyl}-(2,3-dihydro[1,4]dioxino[2,3-*c*]pyridin-7-ylmethyl)amine;
6-({2-[4-(6-methoxyquinolin-4-yl)piperazin-1-yl]ethylamino}methyl)-4*H*-pyrido[3,2-*b*][1,4]oxazin-3-one;
6-({2-[4-(6-methoxyquinolin-4-yl)piperazin-1-yl]ethylamino}methyl)-4*H*-pyrido[3,2-*b*][1,4]thiazin-3-one;
(2,3-dihydro-[1,4]dioxino[2,3-*c*]pyridin-7-ylmethyl)-{2-[4-(6-methoxyquinolin-4-yl)piperizin-1-yl]ethyl}amine;
6-({2-[4-(6-methoxynaphthyridin-4-yl)piperazin-1-yl] ethylamino} methyl)-4H-pyrido[3,2-*b*][1,4]oxazin-3-one;
6-({2-[4-(6-methoxynaphthyridin-4-yl)piperazin-1-yl] ethylamino} methyl)-4*H-*pyrido[3,2-*b*][1,4]thiazin-3-one;
(2,3-dihydro-[1,4]dioxino[2,3-*c*]pyridin-7-ylmethyl)-{2-[4-(6-methoxynaphthyridin-4-yl)piperizin-1-yl]ethyl}amine;
6-({2-[4-(3-chloro-6-methoxyquinolin-4-yl)piperazin-1-yl] ethylamino}methyl)-4*H-*pyrido[3,2-*b*][1,4]oxazin-3-one;
6-({2-[4-(3-chloro-6-methoxyquinolin-4-yl)piperazin-1-yl] ethylamino}methyl)-4*H-*pyrido[3,2-*b*][1,4]thiazin-3-one;
{2-[4-(3-chloro-6-methoxyquinolin-4-yl)piperazin-1-yl]ethyl}-(2,3-dihydro[1,4]dioxino[2,3-*c*]pyridin-7-ylmethyl)amine;
6-({2-[4-(3-chloro-6-methoxynaphthyridin-4-yl)piperazin-1-yl] ethylamino}methyl) 4*H*-pyrido[3,2-*b*][1,4]oxazin-3-one;
6-({2-[4-(3-chloro-6-methoxynaphthyridin-4-yl)piperazin-1-yl] ethylamino)methyl)-4*H*-pyrido[3,2-*b*][1,4]thiazin-3-one;
{2-[4-(3-chloro-6-methoxynaphthyridin-4-yl)piperazin-1-yl]ethyl}-(2,3-dihydro[1,4]dioxino[2,3-*c*]pyridin-7-ylmethyl)amine;
6-({2-[4-(6-methoxy-[1,5]naphthyridin-4-yl)-3,6-dihydro-2H -pyridin-1-yl]-2-oxo-ethylamino}-methyl)-4H-pyrido(3,2-*b*][1,4]thiazin-3-one;
N-(2-{1-[6-(methyloxy)-1,5-naphthyridin-4-yl]-4-piperidinyl}ethyl)-3-oxo-3,4-dihydro-2H-pyrido[3,2-b][1,4]thiazine-6-carboxamide;
N-(2-{1-[6-(methyloxy)-1,5-naphthyridin-4-yl]-4-piperidinyl}ethyl)-3-oxo-3,4-dihydro-2H-1,4-benzothiazine-6-sulfonamide;
N-methyl-N-(2-{1-[6-(methyloxy)-1,5-naphthyridin-4-yl]-4-piperidinyl} ethyl)-3-oxo-3,4-dihydro-2H-pyrido[3,2-b][1,4]thiazine-6-carboxamide;
N-methyl-N-(2-{1-[6-(methyloxy)-1,5-naphthyridin-4-yl]-4-piperidinyl} ethyl)-3-oxo-3,4-dihydro-2H-1,4-benzothiazine-6-sulfonamide;
N-(2-{1-[3-chloro-6-(methyloxy)-1,5-naphthyridin-4-yl]-4-piperidinyl} ethyl)-3-oxo-3,4-dihydro-2H-1,4-benzothiazine-6-sulfonamide;
N-(2-{4-[6-(methyloxy)-1,5-naphthyridin-4-yl]-1-piperazinyl}ethyl)-3-oxo-3,4-dihydro-2H-pyrido[3,2-b][1,4]thiazine-6-carboxamide;
N-methyl-N-(2-{4-[6-(methyloxy)-1,5-naphthyridin-4-yl]-1-piperazinyl}ethyl)-3-oxo-3,4-dihydro-2H-pyrido[3,2-b][1,4]thiazine-6-carboxamide;
N-(2-{4-[6-(methyloxy)-1,5-naphthyridin-4-yl]-1-piperazinyl}ethyl)-3-oxo-3,4-dihydro-2H-1,4-benzothiazine-6-sulfonamide;
N-methyl-N-(2-{4-[6-(methyloxy)-1,5-naphthyridin-4-yl]-1-piperazinyl} ethyl)-3-oxo-3,4-dihydro-2H-1,4-benzothiazine-6-sulfonamide;
6-{[(2-{4-[6-(methyloxy)-1,5-naphthyridin-4-yl]hexahydro-1H-1,4-diazepin-1-yl}ethyl)amino]methyl}-2H-pyrido[3,2-b][1,4]thiazin-3(4H)-one;
N-(2-{4-[6-(methyloxy)-1,5-naphthyridin-4-yl]hexahydro-1H-1,4-diazepin-1-yl}ethyl)-3-oxo-3,4-dihydro-2H-1,4-benzothiazine-6-sulfonamide;
6-{[(2-{(1R,4R)-5-[6-(methyloxy)-1,5-naphthyridin-4-yl]-2,5-diazabicyclo [2.2.1]hept-2-yl}ethyl)amino]methyl}-2H-pyrido[3,2-b][1,4]thiazin-3(4H)-one;
6-{[(2-{4-hydroxy-1-[6-(methyloxy)-1,5-naphthyridin-4-yl]-4-piperidinyl}ethyl) amino]methyl}-2H-pyrido[3,2-b][1,4]thiazin-3(4H)-one;
6-{[(2-{4-hydroxy-1-[6-(methyloxy)-1,5-naphthyridin-4-yl]-4-piperidinyl}ethyl) amino]methyl}-2H-pyrido[3,2-b][1,4]oxazin-3(4H)-one;
N-(2-(4-hydroxy-1-[6-(methyloxy)-1,5-naphthyridin-4-yl]-4-piperidinyl} ethyl)-3-oxo-3,4-dihydro-2H-1,4-benzothiazine-6-sulfonamide; or
a pharmaceutically acceptable salt thereof.

12. A pharmaceutical composition, comprising a compound according to claim 1 and a pharmaceutically acceptable carrier.

13. The use of a compound according to claim 1, in the manufacture of a medicament for use in the treatment of bacterial infections in mammals.

## Patentansprüche

1. Verbindung der Formel (I) 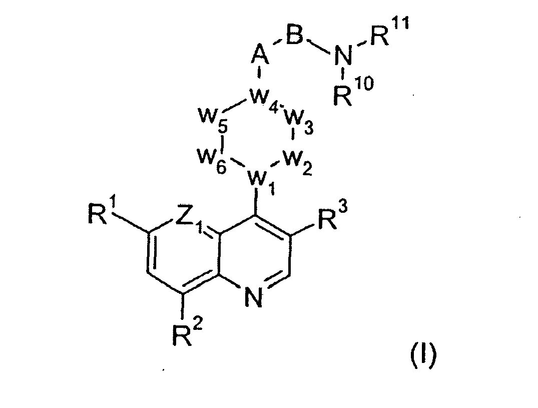 wobei:
Z₁ N oder CR^{1a} ist;
R¹ und R^{1a} unabhängig Wasserstoff; Hydroxy; (C₁₋₆)-Alkoxy, unsubstituiert oder substituiert mit (C₁₋₆-Alkoxy), Hydroxy, Amino, Piperidyl, Guanidino oder Amidino, welche jeweils unsubstituiert oder N-substituiert sind mit einem oder zwei (C₁₋₆)-Alkyl, Acyl, (C₁₋₆)-Alkylsulfonyl, CONH₂, Hydroxy, (C₁₋₆)-Alkylthio, Heterocyclylthio, Heterocyclyloxy, Arylthio, Aryloxy, Acylthio, Acyloxy oder (C₁₋₆)lkylsulfonyloxy; (C₁₋₆)-Alkoxy-substituiertes (C₁₋₆)-Alkyl; Halogen; (C₁₋₆)-Alkyl; (C₁₋₆)-Alkylthio; Trifluormethyl; Trifluormethoxy; Nitro; Azido; Cyano; Acyl; Acyloxy; Acylthio; (C₁₋₆)-Alkylsulfonyl; (C₁₋₆)-Alkylsulfoxid; Arylsulfonyl; Arylsulfoxid; oder ein Amino-, Piperidyl-, Guanidino- oder Amidinorest, unsubstituiert oder N-substituiert mit einem oder zwei (C₁₋₆)-Alkyl-, Acyl-, (C₁₋₆)-Alkylsulfonylresten, sind; oder R¹ und R^{1a} zusammen Ethylendioxy bilden können;
mit der Maßgabe, dass, wenn Z₁ CR^{1a} ist, dann R¹ nicht H ist;
R² H oder Halogen ist;
mit der Maßgabe, dass, wenn Z₁ N ist, dann R² H ist;
R³ Wasserstoff; Halogen; Hydroxy; Cyano; CF₃, Nitro; Azido; Acyl; Aryl; Heteroaryl; CO₂H; Acyloxy; Acylthio; (C₁₋₆)-Alkyl, unsubstituiert oder substituiert mit einem oder zwei (C₁₋₆)-Alkoxy, Hydroxy, Amino, Piperidyl, Guanidino oder Amidino, welche jeweils unsubstituiert oder N-substituiert sind mit einem oder zwei (C₁₋₆)-Alkyl, Acyl, (C₁₋₆)-Alkylsulfony, CONH₂, Hydroxy, (C₁₋₆)-Alkylthio, Heterocyclylthio, Heterocyclyloxy, Arylthio, Aryloxy, Acylthio, Acyloxy oder (C₁₋₆)-Alkylsulfonyloxy; (C₁₋₆)-Alkoxy, unsubstituiert oder substituiert mit einem oder zwei (C₁₋₆)-Alkoxy, Hydroxy, Amino, Piperidyl, Guanidino oder Amidino, welche jeweils unsubstituiert oder N-substituiert sind mit einem oder zwei (C₁₋₆)-Alkyl, Acyl, (C₁₋₆)-Alkylsulfonyl, CONH₂, Hydroxy, (C₁₋₆)-Alkylthio, Heterocyclylthio, Heterocyclyloxy, Arylthio, Aryloxy, Acylthio, Acyloxy oder (C₁₋₆)-Alkylsulfonyloxy; (C₃₋₇)-Cycloalkyl; (C₁₋₆)-Alkoxy-substituiertes (C₁₋₆)-Alkyl; (C₁₋₆)-Alkylthio; Trifluormethoxy; (C₁₋₆)-Alkylsulfonyl; (C₁₋₆)-Alkylsulfoxid; Arylsulfonyl oder Arylsulfoxid; oder ein Amino-, Piperidyl-, Guanidino- oder Amidinorest, unsubstituiert oder N-substituiert mit einem oder zwei (C₁₋₆)-Alkyl-, Acyl- oder (C₁₋₆)-Alkylsulfonylresten, ist;
w₁ N, C oder CR⁴ ist;
w₂ C=O, CR⁴ oder CR⁴R⁵ ist;
w₃ C=O oder CR⁴R⁵ ist;
w₄ N oder CR⁴ ist;
w₅ C=O oder CR⁴R⁵ ist;
w₆ C=O, CR⁴ oder CR⁴R⁵ ist;
oder eines von w₂, w₃, w₅ und w₆ CR⁴R⁵CR⁴R⁵ ist und die anderen wie vorstehend definiert sind;
wobei
R⁴ und R⁵ jeweils unabhängig Wasserstoff; Halogen; Hydroxy; Cyano; CF₃; Nitro; Azido; Acyl; Aryl; Heteroaryl; CO₂H; Acyloxy; Acylthio; (C₁₋₆)-Alkyl, unsubstituiert oder substituiert mit einem oder zwei (C₁₋₆)-Alkoxy, Hydroxy, Amino, Piperidyl, Guanidino oder Amidino, welche jeweils unsubstituiert oder N-substituiert sind mit einem oder zwei (C₁₋₆)-Alkyl, Acyl, (C₁₋6)-Alkylsulfonyl, CONH₂, Hydroxy, (C₁₋₆)-Alkylthio, Heterocyclylthio, Heterocyclyloxy, Arylthio, Aryloxy, Acylthio, Acyloxy oder (C₁₋₆)-Alkylsulfonyloxy; (C₁₋₆)-Alkoxy, unsubstituiert oder substituiert mit einem oder zwei (C₁₋₆)-Alkoxy, Hydroxy, Amino, Piperidyl, Guanidino oder Amidino, welche jeweils unsubstituiert oder N-substituiert sind mit einem oder zwei (C₁₋₆)-Alkyl, Acyl, (C₁₋₆)-Alkylsulfonyl, CONH₂, Hydroxy, (C₁₋₆)-Alkylthio, Heterocyclylthio, Heterocyclyloxy, Arylthio, Aryloxy, Acylthio, Acyloxy oder (C₁₋₆)-Alkylsulfonyloxy; (C₃₋₇)-Cycloalkyl; (C₁₋₆)-Alkoxy-substituiertes (C₁₋₆)-Alkyl; (C₁₋₆)-Alkylthio; Trifluormethoxy; (C₁₋₆)-Alkylsulfonyl; (C₁₋₆)-Alkylsulfoxid; Arylsulfonyl oder Arylsulfoxid; oder ein Amino-, Piperidyl-, Guanidino- oder Amidinorest, unsubstituiert oder N-substituiert mit einem oder zwei (C₁₋₆)-Alkyl-, Acyl- oder (C₁₋₆)-Alkylsulfonylresten, sind; oder zwei R⁵-Reste zusammen gebunden sind, um Bicycloheptan zu bilden;
A CR⁶R⁷ oder C(O) ist;
B CR⁸R⁹ oder C(O) ist;
R⁶, R⁷, R⁸ und R⁹ unabhängig Wasserstoff; Halogen; Hydroxy; Cyano; CF₃; Nitro; Azido; Acyl; Aryl; Heteroaryl; CO₂H, Acyloxy; Acylthio; (C₁₋₆)-Alkyl, unsustituiert oder substituiert mit einem oder zwei (C₁₋₆)-Alkoxy, Hydroxy, Amino, Piperidyl, Guanidino oder Amidino, welche jeweils unsubstituiert oder N-substituiert sind mit einem oder zwei (C₁₋₆)-Alkyl, Acyl, (C₁₋₆)-Alkylsulfonyl, CONH₂, Hydroxy, (C₁₋₆)-Alkylthio, Heterocyclylthio, Heterocyclyloxy, Arylthio, Aryloxy, Acylthio, Acyloxy oder (C₁₋₆)-Alkylsulfonyloxy; (C₁₋₆)-Alkoxy, unsubstituiert oder substituiert mit einem oder zwei (C₁₋₆)-Alkoxy, Hydroxy, Amino, Piperidyl, Guanidino oder Amidino, welche jeweils unsubstituiert oder N-substituiert sind mit einem oder zwei (C₁₋₆)-Alkyl, Acyl, (C₁₋₆)-Alkylsulfonyl, CONH₂, Hydroxy, (C₁₋₆)-Alkylthio, Heterocyclylthio, Heterocyclyloxy, Arylthio, Aryloxy, Acylthio, Acyloxy oder (C₁₋₆)-Alkylsulfonyloxy; (C₃₋₇)-Cycloalkyl; (C₁₋₆)-Alkoxy-substituiertes (C₁₋₆)-Alkyl; (C₁₋₆)-Alkylthio; Trifluormethoxy; (C₁₋₆)-Alkylsulfonyl; (C₁₋₆)-Alkylsulfoxid; Arylsulfonyl oder Arylsulfoxid; oder ein Amino-, Piperidyl-, Guanidino- oder Amidinorest, unsubstituiert oder N-substituiert mit einem oder zwei (C₁₋₆)-Alkyl-, Acyl- oder (C₁₋₆)-Alkylsulfonylresten, sind;
R¹⁰ Wasserstoff; Aryl; Heteroaryl; (C₁₋₆)-Alkyl, unsubstituiert oder substituiert mit einem oder zwei (C₁₋₆)-Alkoxy, Acyloxy, Carboxy, Hydroxy, Amino, Piperidyl, Piperazinyl, Morpholino, Guanidino oder Amidino, welche jeweils substituiert oder N-substituiert sind mit einem oder zwei Aryl, Heteroaryl, Halogen, Cyano, CF₃, unsubstituiertem (C₁₋₆)-Alkyl, Acyl, (C₁₋₆)-Alkylsulfonyl, Arylsulfonyl, Hydroxy, (C₁₋₆)-Alkylthio, Heterocyclylthio, Heterocyclyloxy, Arylthio, Aryloxy, Acylthio, Acyloxy oder (C₁₋₆)-Alkylsulfonyloxy, so lange die Substitution zu keiner instabilen Verbindung führt; (C₁₋₆)-Alkylcarbonyl; (C₂₋₆)-Alkenylcarbonyl; (C₁₋₆)-Alkoxycarbonyl; CO₂H oder CF₃ ist; R¹¹ ein Rest -U-R¹² ist, wobei R¹² ein substituiertes oder unsubstituiertes bicyclisches carbocyclisches oder heterocyclisches Ringsystem (A) ist: 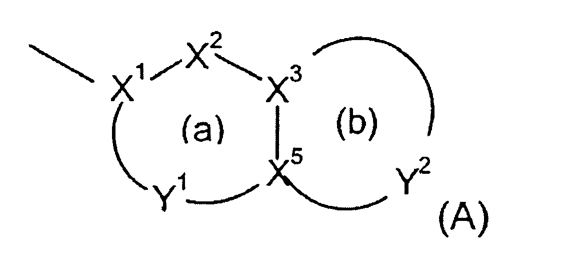 welches bis zu vier Heteroatome in jedem Ring enthält, wobei mindestens einer der Ringe (a) und (b) aromatisch ist;
X¹ C oder N ist, wenn Teil eines aromatischen Rings, oder CR¹⁴ ist, wenn Teil eines nicht aromatischen Rings;
X² N, NR¹³, O, S(O)ₓ, CO oder CR¹⁴ ist, wenn Teil eines aromatischen oder nicht aromatischen Rings, oder zusätzlich CR¹⁴R¹⁵ sein kann, wenn Teil eines nicht aromatischen Rings;
X³ und X⁵ unabhängig N oder C sind;
Y¹ ein Linkerrest mit 0 bis 4 Atomen ist, wobei die Atome jeweils unabhängig ausgewählt sind aus N, NR¹³, O, S(O)ₓ, CO und CR¹⁴, wenn Teil eines aromatischen oder nicht aromatischen Rings, oder zusätzlich CR¹⁴R¹⁵ sein kann, wenn Teil eines nicht aromatischen Rings;
Y² ein Linkerrest mit 2 bis 6 Atomen ist, wobei die Atome von Y² jeweils unabhängig ausgewählt sind aus N, NR¹³, O, S(O)ₓ, CO und CR¹⁴, wenn Teil eines aromatischen oder nicht aromatischen Rings, oder zusätzlich CR¹⁴R¹⁵ sein kann, wenn Teil eines nicht aromatischen Rings;
R¹⁴ und R¹⁵ jeweils unabhängig ausgewählt sind aus H; (C₁₋₄)-Alkylthio; Halogen; (C₁₋₄)-Alkyl; (C₂₋₄)-Alkenyl; Hydroxy; Hydroxy-(C₁₋₄)-alkyl; Mercapto-(C₁₋₄)-alkyl; (C₁₋₄)-Alkoxy; Trifluormethoxy; Nitro; Cyano; Carboxy; Amino oder Aminocarbonyl, unsubstituiert oder substituiert mit (C₁₋₄)-Alkyl;
R¹³ jeweils unabhängig H; Trifluormethyl; (C₁₋₄)-Alkyl, unsubstituiert oder substituiert mit Hydroxy, Carboxy, (C₁₋₄)-Alkoxy, (C₁₋₆)-Alkylthio, Halogen oder Trifluormethyl; (C₂₋₄)-Alkenyl; oder Aminocarbonyl ist, wobei die Aminogruppe gegebenenfalls substituiertes (C₁₋₄)-Alkyl ist;
x jeweils unabhängig 0, 1 oder 2 ist;
U CO, SO₂, CH₂ oder CR¹⁶R¹⁷ ist;
R¹⁶ und R¹⁷ unabhängig ausgewählt sind aus H; Aryl; Heteroaryl; (C₁₋₆)-Alkyl; (C₁₋₆)-Alkyl, substituiert mit (C₁₋₆)-Alkoxy, Hydroxy, Amino, Piperidyl, Piperazinyl, Morpholino, Guanidino oder Amidino, welche jeweils unsubstituiert oder N-substituiert sind mit einem oder zwei H, Aryl, Heteroaryl, Halogen, Cyano, CF₃, (C₁₋₆)-Alkyl, Acyl, (C₁₋₆)-Alkylsulfonyl, Arylsulfonyl, Hydroxy, (C₁₋₆)-Alkylthio, Heterocyclylthio, Heterocyclyloxy, Arylthio, Aryloxy, Acylthio, Acyloxy oder (C₁₋₆)-Alkylsulfonyloxy, mit der Maßgabe, dass die Substitution zu keiner instabilen Verbindung führt; (C₁₋₆)-Alkoxy-substituiertem (C₁₋₆)-Alkyl, Hydroxy-substituiertem (C₁₋₆)-Alkyl; Amino-substituiertem (C₁₋₆)-Alkyl, welches N-substituiert ist mit einem oder zwei (C₁₋₆)-Alkyl, Acyl, (C₁₋₆)-Alkylsulfonyl oder Arylsulfonyl; (C₁₋₆)-Alkylcarbonyl; (C₂₋₆)-Alkenylcarbonyl; (C₁₋₆)-Alkoxycarbonyl; CO₂H oder CF₃;
oder ein pharmazeutisch verträgliches Salz davon.

2. Verbindung gemäß Anspruch 1, wobei R¹ F, Cl, OCH₃, Methyl oder SCH₃ ist.

3. Verbindung gemäß Anspruch 1 oder 2, wobei R^{1a} H, OCH₃ oder OCH₂CH₂OCH₃ ist.

4. Verbindung gemäß einem der vorstehenden Ansprüche, wobei R² H oder F ist.

5. Verbindung gemäß einem der vorstehenden Ansprüche, wobei R³ Cl oder F ist.

6. Verbindung gemäß einem der vorstehenden Ansprüche, wobei w₁ N oder CR⁴ ist, w₂, w₃, w₅ und w₆ CR⁴R⁵ sind, R⁴ jeweils unabhängig H, Methyl, OH, -COOH, NH₂, OCH₃ oder CH₂OH ist und R⁵ H ist.

7. Verbindung gemäß einem der vorstehenden Ansprüche, wobei A CR⁶R⁷ ist, B CR⁸R⁹ ist, R⁶ und R⁸ H sind, R⁷ H oder OH ist und R⁹ H oder OH ist.

8. Verbindung gemäß einem der vorstehenden Ansprüche, wobei der Rest -U- -CH₂- ist.

9. Verbindung gemäß einem der vorstehenden Ansprüche, wobei R¹⁰ H ist.

10. Verbindung gemäß einem der vorstehenden Ansprüche, wobei R¹²
Benzo[1,2,5]thiadiazol-5-yl;
4*H*-Benzo[1,4]thiazin-3-on-6-yl;
2,3-Dihydrobenzo[1,4]dioxin-6-yl;
Benzo[1,2,3]thiadiazol-5-yl;
3-Oxo-3,4-dihydro-2*H*-benzo[1,4]oxazin-6-yl;
7-Fluor-3-oxo-3,4-dihydro-2*H*-benzo[1,4]oxazin-6-yl;
2-Oxo-2,3-dihydro-1*H*-pyrido[2,3-*b*][1,4]thiazin-7-yl;
2,3-Dihydro[1,4]dioxino[2,3-*c*]pyridin-7-yl;
3-Oxo-3,4-dihydro-2*H*-pyrido[3,2-*b*][1,4]oxazin-6-yl;
[1,2,3]-Thiadiazolo[5,4-b]pyridin-6-yl;
3-OXo-3,4-dihydro-2*H*-pyrido[3,2-*b*][1,4]thiazin-6-yl;
7-Chlor-3-oxo-3,4-dihydro-2*H*-pyrido[3,2-*b*][1,4]thiazin-6-yl;
7-Fluor-3-oxo-3,4-dihydro-2*H*-pyrido[3,2-*b*][1,4] thiazin-6-yl; oder
2-Oxo-2,3-dihydro-1*H*-pyrido[3,4-*b*][1,4]thiazin-7-yl ist.

11. Verbindung gemäß Anspruch 1, wobei die Verbindung
6-({2-[1-(6-Methoxychinolin-4-yl)piperidin-4-yl]ethylamino}methyl-4*H*-pyrido[3,2*b*]-[1,4]oxazin-3-on;
6-({2-[1-(6-Methoxychinolin-4-yl)piperidin-4-yl]ethylamino}methyl)-4*H*-pyrido[3,2*b*]-[1,4]thiazin-3-on;
(2,3-Dihydro-[1,4]dioxino[2,3-*c*]pyridin-7-ylmethyl)-{2-[1-(6-methoxychinolin-4-yl)-piperidin-4-yl]ethyl} amin;
6-({2-[1-(6-Methoxynaphthyridin-4-yl)piperidin-4-yl]ethylamino}methyl)-4*H*-pyrido-[3,2-*b*][1,4]oxazin-3-on;
6-({2-[1-(6-Methoxynaphthyridin-4-yl)piperidin-4-yl]ethylamino}methyl)-4*H*-pyrido-[3,2-*b*][1,4]thiazin-3-on;
(2,3-Dihydro[1,4]dioxino[2,3-*c*]pyridin-7-ylmethyl)-{2-[1-(6-methoxynaphthyridin-4-yl)piperidin-4-yl]ethyl} amin;
6-({2-[1-(3-Chlor-6-methoxy[1,5]chinolin-4-yl)phenyl]ethylamino}methyl)-4*H*-pyrido-[3,2-*b*][1,4]oxazin-3-on;
6-({2-[1-(3-Chlor-6-methoxy[1,5]chinolin-4-yl)phenyl]ethylamino}methyl}-4*H*-pyrido-[3,2-*b*][1,4]thiazin-3-on;
{2-(1-(3-Chlor-6-methoxychinolin-4-yl)piperidin-4-yl]ethyl}-(2,3-dihydro[1,4]dioxino-[2,3-*c*]pyridm-7-ylmethyl)amin;
6-({2-[1-(3-Chlor-6-methoxy[1,5]naphthyridin-4-yl)phenyl]ethylamino}methyl}4*H-*pyrido[3,2-*b*][1,4]oxazin-3-on;
6-({2-[1-(3-Chlor-6-methoxy[1,5]naphthyridin-4-yl)phenyl]ethylamino}methyl-4*H-*pyrido[3,2-*b*][1,4]thiazin-3-on;
{2-[1-(3-Chlor-6-methoxynaphthyridin-4-yl)piperidin-4-yl]ethyl}-(2,3-dihydro[1,4]-dioxino[2,3-*c*]pyridin-7-ylmethyl)amin;
6-({2-[4-(6-Methoxychinolin-4-yl)piperazin-1-yl]ethylamino}methyl)-4*H*-pyrido[3,2*b*]-[1,4]oxazin-3-on;
6-({2-[4-(6-Methoxychinolin-4-yl)piperazin-1-yl]ethylamino}methyl)-4H-pyrido[3,2b]-[1,4]thiazin-3-on;
(2,3-Dihydro[1,4]dioxino[2,3-*c*]pyridin-7-ylmethyl)-{2-[4-(6-methoxychinolin-4-yl)-piperizin-1-yl]ethyl}amin;
6-({2-[4-(6-Methoxynaphthyridin-4-yl)piperazin-1-yl]ethylamino}methyl)-4*H*-pyrido-[3,2-*b*][1,4]oxazin-3-on;
6-({2-[4-(6-Methoxynaphthyridin-4-yl)piperazin-1-yl]ethylamino}methyl)-4*H*-pyrido-[3,2-*b*][1,4]thiazin-3-on;
(2,3-Dihydro[1,4]dioxino[2,3-*c*]pyridin-7-ylmethyl)-{2-[4-(6-methoxynaphthyridin-4-yl)piperizin-1-yl]ethyl}amin;
6-({2-[4-(3-Chlor-6-methoxychinolin-4-yl)piperazin-1-yl]ethylamino}methyl)-4*H-*pyrido[3,2-*b*][1,4]oxazin-3-on;
6-({2-[4-(3-Chlor-6-methoxychinolin-4-yl)piperazin-1-yl]ethylamino}methyl)-4*H-*pyrido[3,2-*b*][1,4]thiazin-3-on;
{2-[4-(3-Chlor-6-methoxychinolin-4-yl)piperazin-1-yl]ethyl}-(2,3-dihydro[1,4]dioxino-[2,3-*c*]pyridin-7-ylmethyl)amin;
6-({2-[4-(3-Chlor-6-methoxynaphthyridin-4-yl)piperazin-1-yl]ethylamino}methyl}-4*H-*pyrido[3,2-*b*][1,4]oxazin-3-on;
6-({2-[4-(3-Chlor-6-methoxynaphthyridin-4-yl)piperazin-1-yl]ethylamino)methyl}-4*H-*pyrido[3,2-*b*][1,4]thiazin-3-on;
{2-[4-(3-Chlor-6-methoxynaphthyridin-4-yl)piperazin-1-yl]ethyl}-(2,3-dihydro[1,4]-dioxino[2,3-*c*]pyridin-7-ylmethyl)amin;
6-({2-[4-(6-Methoxy[1,5]naphthyridin-4-yl)-3,6-dihydro-2*H*-pyridin-1-yl]-2-oxo-ethylamino} methyl)-4*H*-pyrido[3,2-*b*][1,4]thiazin-3-on;
N-(2-{1-[6-(Methyloxy)-1,5-naphthyridin-4-yl]-4-piperidinyl}ethyl)-3-oxo-3,4-dihydro-2*H*-pyrido[3,2-*b*][1,4]thiazin-6-carboxamid;
N-(2-{1-[6-(Methyloxy)-1,5-naphthyridin-4-yl]-4-piperidinyl}ethyl)-3-oxo-3,4-dihydro-2*H*-1,4-benzothiazin-6-sulfonamid;
N-Methyl-N-(2-{1-[6-(methyloxy)-1,5-naphthyridin-4-yl]-4-piperidinyl}ethyl)-3-oxo-3,4-dihydro-2*H*-pyrido[3,2-*b*][1,4]thiazin-6-carboxamid;
N-Methyl-N-(2-{1-[6-(methyloxy)-1,5-naphthyridin-4-yl]-4-piperidinyl}ethyl)-3-oxo-3,4-dihydro-2*H*-1,4-benzothiazin-6-sulfonamid;
N-(2-{1-[3-Chlor-6-(methyloxy)-1,5-naphthyridin-4-yl]-4-piperidinyl}ethyl)-3-oxo-3,4-dihydro-2*H*-1,4-benzothiazin-6-sulfonamid;
N-(2-{4-[6-(Methyloxy}-1,5-naphthyridin-4-yl]-1-piperazinyl]ethyl)-3-oxo-3,4-dihydro-2*H*-pyrido[3,2-*b*][1,4]thiazin-6-carboxamid;
N-Methyl-N-(2-{4-[6-(methyloxy)-1,5-naphthyridin-4-yl]-1-piperazinyl}ethyl)-3-oxo-3,4-dihydro-2*H*-pyrido[3,2-*b*][1,4]thiazin-6-carboxamid;
N-(2-{4-[6-(Methyloxy)-1,5-naphthyridin-4-yl]-1-piperazinyl}ethyl)-3-oxo-3,4-dihydro-2*H*-1,4-benzothiazin-6-sulfonamid;
N-Methyl-N-(2-{4-[6-(methyloxy)-1,5-naphthyridin-4-yl]-1-piperazinyl}ethyl)-3-oxo-3,4-dihydro-2*H*-1,4-benzothiazin-6-sulfonamid;
6-{[(2-{4-[6-(Methyloxy)-1,5-naphthyridin-4-yl]hexahydro-1*H*-1,4-diazepin-1-yl}ethyl)-amino]methyl}-2*H*-pyrido[3,2-*b*][1,4]thiazin-3(4*H*)-on;
N-(2-{4-[6-(Methyloxy)-1,5-naphthyridin-4-yl]hexahydro-1*H*-1,4-diazepin-1-yl}ethyl)-3-oxo-3,4-dihydro-2*H*-1,4-benzothiazin-6-sulfonamid;
6- {[(2-{(1R,4R)-5-[6-(Methyloxy)-1,5-naphthyridin-4-yl]-2,5-diazabicyclo[2.2.1]hept-2-yl}ethyl)amino]methyl}-2*H*-pyrido[3,2-*b*][1,4 ]thiazin-3(4*H*)-on;
6-{[(2-{4-Hydroxy-1-[6-(methyloxy}-1,5-naphthyridin-4-yl]-4-piperidinyl}ethyl)amino]-methyl}-2*H*-pyrido[3,2-*b*][1,4]thiazin-3(4*H*)-on;
6- {[(2-{4-Hydroxy-1-[6-(methyloxy)-1,5-napthyridin-4-yl]-4-piperidinyl}ethyl)amino]-methyl}-2*H*-pyrido[3,2-*b*][1,4]oxazin-3(4*H*)-on;
N-(2-{4-Hydroxy-1-[6-(methyloxy)-1,5-naphthyridin-4-yl]-4-piperidinyl}ethyl)-3-oxo-3,4-dihydro-2*H*-1,4-benzothiazin-6-sulfonamid; oder
oder ein pharmazeutisch verträgliches Salz davon ist.

12. Arzneimittel, umfassend eine Verbindung gemäß Anspruch 1 und einen pharmazeutisch verträglichen Träger.

13. Verwendung einer Verbindung gemäß Anspruch 1 zur Herstellung eines Medikaments zur Verwendung bei der Behandlung von bakteriellen Infektionen in Säugern.

## Revendications

1. Composé de formule (I) 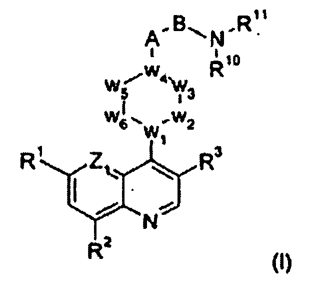 dans laquelle :
Z₁ représente N ou un groupe CR^{1a} ;
R¹ et R^{1a} représentent indépendamment un atome d'hydrogène ; un groupe hydroxy ; alkoxy en C₁ à C₆ non substitué ou substitué avec un substituant alkoxy en C₁ à C₆, hydroxy, amino, pipéridyle, guanidino ou amidino, dont n'importe lequel est non substitué ou N-substitué avec un ou deux substituants alkyle en C₁ à C₆, acyle, alkylsulfonyle en C₁ à C₆, CONH₂, hydroxy, alkylthio en C₁ à C₆, hétérocyclylthio, hétérocyclyloxy, arylthio, aryloxy, acylthio, acyloxy ou alkylsulfonyloxy en C₁ à C₆ ; alkyle en C₁ à C₆ à substituant alkoxy en C₁ à C₆ ; halogéno ; alkyle en C₁ à C₆ ; alkylthio en C₁ à C₆ ; trifluorométhyle ; trifluorométhoxy ; nitro ; azido ; cyano ; acyle ; acyloxy ; acylthio ; alkylsulfonyle en C₁ à C₆ ; alkylsulfoxyde en C₁ à C₆ ; arylsulfonyle ; arylsulfoxyde ; ou un groupe amino, pipéridyle, guanidino ou amidino non substitué ou N-substitué avec un ou deux groupes alkyle en C₁ à C₆, acyle ou alkylsulfonyle en C₁ à C₆ ; ou bien R¹ et R^{1a} peuvent former conjointement un groupe éthylènedioxy ;
sous réserve que, lorsque Z₁ représente un groupe CR^{1a}, alors R¹ ne représente pas H ;
R² représente H ou un atome d'halogène ;
sous réserve que, lorsque Z₁ représente un groupe N, alors R² représente H ;
R³ représente un atome d'hydrogène ; un atome d'halogène ; un groupe hydroxy ; cyano ; CF₃ ; nitro ; azido ; acyle ; aryle ; hétéroaryle ; CO₂H ; acyloxy ; acylthio ; alkyle en C₁ à C₆ non substitué ou substitué avec un ou deux substituants alkoxy en C₁ à C₆, hydroxy, amino, pipéridyle, guanidino ou amidino, n'importe lequel étant non substitué ou N-substitué avec un ou deux substituants alkyle en C₁ à C₆, acyle, alkylsulfonyle en C₁ à C₆, CONH₂, hydroxy, alkylthio en C₁ à C₆, hétérocyclylthio, hétérocyclyloxy, arylthio, aryloxy, acylthio, acyloxy ou alkylsulfonyloxy en C₁ à C₆ ; alkoxy en C₁ à C₆ non substitué ou substitué avec un ou deux substituants alkoxy en C₁ à C₆, hydroxy, amino, pipéridyle, guanidino ou amidino, n'importe lequel étant non substitué ou N-substitué avec un ou deux substituants alkyle en C₁ à C₆, acyle, alkylsulfonyle en C₁ à C₆, CONH₂, hydroxy, alkylthio en C₁ à C₆, hétérocyclylthio, hétérocyclyloxy, arylthio, aryloxy, acylthio, acyloxy ou alkylsulfonyloxy en C₁ à C₆ ; cycloalkyle en C₃ à C₇ ; alkyle en C₁ à C₆ à substituant alkoxy en C₁ à C₆ ; alkylthio en C₁ à C₆ ; trifluorométhoxy ; alkylsulfonyle en C₁ à C₆ ; alkylsulfoxyde en C₁ à C₆ ; arylsulfonyle ; ou arylsulfoxyde ; ou un groupe amino, pipéridyle, guanidino ou amidino non substitué ou N-substitué avec un ou deux groupes alkyle en C₁ à C₆, acyle ou alkylsulfonyle en C₁ à C₆ ;
w₁ représente N, C ou un groupe CR⁴ ;
w₂ représente un groupe C=O, CR⁴ ou CR⁴R⁵ ;
w₃ représente un groupe C=O ou CR⁴R⁵ ;
w₄ représente N ou un groupe CR⁴ ;
w₅ représente un groupe C=O ou CR⁴R⁵ ;
w₆ représente un groupe C=O, CR⁴ ou CR⁴R⁵;
ou bien un de w₂, w₃, w₅ et w₆ représente un groupe CR⁴R⁵CR⁴R⁵ et les autres répondent aux définitions précitées ;
où
chacun des groupes R⁴ et R⁵ représente indépendamment un atome d'hydrogène ; un atome d'halogène ; un groupe hydroxy ; cyano ; CF₃ ; nitro ; azido ; acyle ; aryle ; hétéroaryle ; CO₂H ; acyloxy ; acylthio ; alkyle en C₁ à C₆ non substitué ou substitué avec un ou deux substituants alkoxy en C₁ à C₆, hydroxy, amino, pipéridyle, guanidino ou amidino, n'importe lequel de ces substituants étant non substitué ou N-substitué avec un ou deux substituants alkyle en C₁ à C₆, acyle, alkylsulfonyle en C₁ à C₆, CONH₂, hydroxy, alkylthio en C₁ à C₆, hétérocyclylthio, hétérocyclyloxy, arylthio, aryloxy, acylthio, acyloxy ou alkylsulfonyloxy en C₁ à C₆ ; alkoxy en C₁ à C₆ non substitué ou substitué avec un deux substituants alkoxy en C₁ à C₆, hydroxy, amino, pipéridyle, guanidino ou amidino, n'importe lequel de ces substituants étant non substitué ou N-substitué avec un ou deux substituants alkyle en C₁ à C₆, acyle, alkylsulfonyle en C₁ à C₆, CONH₂, hydroxy, alkylthio en C₁ à C₆, hétérocyclylthio, hétérocyclyloxy, arylthio, aryloxy, acylthio, acyloxy ou alkylsulfonyloxy en C₁ à C₆ ; cycloalkyle en C₃ à C₇ ; alkyle en C₁ à C₆ à substituant alkoxy en C₁ à C₆ ; alkylthio en C₁ à C₆ ; trifluorométhoxy ; alkylsulfonyle en C₁ à C₆ ; alkylsulfoxyde en C₁ à C₆ ; arylsulfonyle ; ou arylsulfoxyde ; ou un groupe amino, pipéridyle, guanidino ou amidino non substitué ou N-substitué avec un ou deux groupes alkyle en C₁ à C₆, acyle ou alkylsulfonyle en C₁ à C₆ ; ou bien deux groupes R⁵ sont joints l'un à l'autre pour former un groupe bicyloheptane ;
A représente un groupe CR⁶R⁷ ou C (O) ;
B représente un groupe CR⁸R⁹ ou C (O) ;
R⁶, R⁷, R⁸ et R⁹ représentent indépendamment un atome d'hydrogène ; un atome d'halogène ; un groupe hydroxy ; cyano ; CF₃ ; nitro ; azido ; acyle ; aryle ; hétéroaryle ; CO₂H ; acyloxy ; acylthio ; alkyle en C₁ à C₆ non substitué ou substitué avec un ou deux substituants alkoxy en C₁ à C₆, hydroxy, amino, pipéridyle, guanidino ou amidino, n'importe lequel de ces substituants étant non substitué ou N-substitué avec un ou deux substituants alkyle en C₁ à C₆, acyle, alkylsulfonyle en C₁ à C₆, CONH₂, hydroxy, alkylthio en C₁ à C₆, hétérocyclylthio, hétérocyclyloxy, arylthio, aryloxy, acylthio, acyloxy ou alkylsulfonyloxy en C₁ à C₆ ; alkoxy en C₁ à C₆ non substitué ou substitué avec un ou deux substituants alkoxy en C₁ à C₆, hydroxy, amino, pipéridyle, guanidino ou amidino, n'importe lequel de ces substituants étant non substitué ou N-substitué avec un ou deux substituants alkyle en C₁ à C₆, acyle, alkylsulfonyle en C₁ à C₆, CONH₂, hydroxy, alkylthio en C₁ à C₆, hétérocyclylthio, hétérocyclyloxy, arylthio, aryloxy, acylthio, acyloxy ou alkylsulfonyloxy en C₁ à C₆ ; cycloalkyle en C₃ à C₇ ; alkyle en C₁ à C₆ à substituant alkoxy en C₁ à C₆ ; alkylthio en C₁ à C₆ ; trifluorométhoxy ; alkylsulfonyle en C₁ à C₆, alkylsulfoxyde en C₁ à C₆ ; arylsulfonyle ; ou arylsulfoxyde ; ou un groupe amino, pipéridyle, guanidino ou amidino non substitué ou N-substitué avec un ou deux groupes alkyle en C₁ à C₆, acyle ou alkylsulfonyle en C₁ à C₆ ;
R¹⁰ représente un atome d'hydrogène ; un groupe aryle ; hétéroaryle ; alkyle en C₁ à C₆ non substitué ou substitué avec un ou deux substituants alkoxy en C₁ à C₆, acyloxy, carboxy, hydroxy, amino, pipéridyle, pipérazinyle, morpholino, guanidino ou amidino, n'importe lequel de ces substituants étant non substitué ou N-substitué avec un ou deux substituants aryle, hétéroaryle, halogéno, cyano, CF₃, alkyle en C₁ à C₆ non substitué, acyle, alkylsulfonyle en C₁ à C₆, arylsulfonyle, hydroxy, alkylthio en C₁ à C₆, hétérocyclylthio, hétérocyclyloxy, arylthio, aryloxy, acylthio, acyloxy ou alkylsulfonyloxy en C₁ à C₆, du moment que la substitution ne conduit pas à un composé instable ; (alkyle en C₁ à C₆) carbonyle ; (alcényle en C₂ à C₆) carbonyle ; (alkoxy en C₁ à C₆) carbonyle ; CO₂H ; ou CF₃ ;
R¹¹ représente un groupe -U-R¹² dans lequel R¹² représente un système de noyau carbocyclique ou hétérocyclique bicyclique non substitué ou substitué (A) : 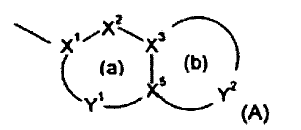 contenant jusqu'à quatre hétéroatomes dans chaque noyau, dans lequel
au moins un des noyaux (a) et (b) est aromatique ;
X¹ représente C ou N lorsqu'il fait partie d'un noyau aromatique ou un groupe CR¹⁴ lorsqu'il fait partie d'un noyau non aromatique ;
X² représente N, un groupe NR¹³, 0, S(O)ₓ, CO ou CR¹⁴ lorsqu'il fait partie d'un noyau aromatique ou non aromatique ou peut représenter en outre un groupe CR¹⁴R¹⁵ lorsqu'il fait d'un noyau non aromatique ;
X³ et X⁵ représentent indépendamment N ou C ;
Y¹ représente un groupe de liaison de 0 à 4 atomes dont chaque atome est choisi indépendamment entre N, NR¹³, O, S(O)ₓ, CO et CR¹⁴ lorsqu'il fait partie d'un noyau aromatique ou non aromatique ou peut représenter en outre un groupe CR¹⁴R¹⁵ lorsqu'il fait partie d'un noyau non aromatique ;
Y² représente un groupe de liaison de 2 à 6 atomes, chaque atome de Y² étant choisi indépendamment entre N, NR¹³, O, S(O)ₓ, CO et CR¹⁴, lorsqu'il fait partie d'un noyau aromatique ou non aromatique ou peut représenter en outre un groupe CR¹⁴R¹⁵ lorsqu'il fait partie d'un noyau non aromatique ; chacun des groupes R¹⁴ et R¹⁵ est choisi indépendamment entre : H ; des groupes alkylthio en C₁ à C₄ ; halogéno ; alkyle en C₁ à C₄ ; alcényle en C₂ à C₄ ; hydroxy ; hydroxyalkyle en C₁ à C₄ mercapto (alkyle en C₁ à C₄) alkoxy en C₁ à C₄ ; trifluorométhoxy ; nitro ; cyano ; carboxy ; amino ou aminocarbonyle non substitué ou substitué avec un substituant alkyle en C₁ à C₄ ;
chaque groupe R¹³ représente indépendamment H; un groupe trifluorométhyle ; alkyle en C₁ à C₄ non substitué ou substitué avec un substituant hydroxy, carboxy, alkoxy en C₁ à C₄, alkylthio en C₁ à C₆, halogéno ou trifluorométhyle ; alcényle en C₂ à C₄ ; ou aminocarbonyle dans lequel le groupe amino est facultativement substitué avec un substituant alkyle en C₁ à C₄ ;
chaque x est indépendamment égal à 0, 1 ou 2 ;
U représente un groupe CO, SO₂, CH₂ ou CR¹⁶R¹⁷;
R¹⁶ et R¹⁷ sont choisis indépendamment entre H ; des groupes aryle ; hétéroaryle ; alkyle en C₁ à C₆ ; alkyle en C₁ à C₆ substitué avec un substituant alkoxy en C₁ à C₆, hydroxy, amino, pipéridyle, pipérazinyle, morpholino, guanidino ou amidino, n'importe lequel de ces substituants étant substitué ou N-substitué avec un ou deux substituants H, aryle, hétéroaryle, halogéno, cyano, CF₃, alkyle en C₁ à C₆, acyle, alkylsulfonyle en C₁ à C₆, arylsulfonyle, hydroxy, alkylthio en C₁ à C₆, hétérocyclylthio, hétérocyclyloxy, arylthio, aryloxy, acylthio, acyloxy ou alkylsulfonyloxy en C₁ à C₆, sous réserve que la substitution ne conduise pas à un composé instable ; alkyle en C₁ à C₆ à substituant alkoxy en C₁ à C₆ ; alkyle en C₁ à C₆ à substituant hydroxy ; alkyle en C₁ à C₆ à substituant amino, qui est N-substitué avec un ou deux substituants alkyle en C₁ à C₆, acyle, alkylsulfonyle en C₁ à C₆ ou arylsulfonyle ; (alkyle en C₁ à C₆) carbonyle ; (alcényle en C₂ à C₆) carbonyle ; (alkoxy en C₁ à C₆) carbonyle ; CO₂H ; ou CF₃ ; ou
un de ses sels pharmaceutiquement acceptables.

2. Composé suivant la revendication 1, dans lequel R¹ représente un groupe F, Cl, OCH₃, méthyle ou SCH₃.

3. Composé suivant la revendication 1 ou 2, dans lequel R^{1a} représente H, un groupe OCH₃ ou OCH₂CH₂OCH₃.

4. Composé suivant l'une quelconque des revendications précédentes, dans lequel R² représente H ou F.

5. Composé suivant l'une quelconque des revendications précédentes, dans lequel R³ représente un groupe Cl ou F.

6. Composé suivant l'une quelconque des revendications précédente, dans lequel W₁ représente N ou un groupe CR⁴, W_{2,} W₃, W₅ et W₆ représentent des groupes CR⁴R⁵, chaque groupe R⁴ représente indépendamment H, un groupe méthyle, OH, -COOH, NH₂, OCH₃ ou CH₂OH et R⁵ représente H.

7. Composé suivant l'une quelconque des revendications précédentes, dans lequel A représente un groupe CR⁶R⁷, B représente un groupe CR⁸R⁹, R⁶ et R⁸ représentent H, R⁷ représente H ou un groupe OH et R⁹ représente H ou un groupe OH.

8. Composé suivant l'une quelconque des revendications précédentes, dans lequel le groupe -U- est un groupe -CH₂-.

9. Composé suivant l'une quelconque des revendications précédentes, dans lequel R¹⁰ représente H.

10. Composé suivant l'une quelconque des revendications précédentes, dans lequel R¹² représente un groupe :
benzo[1,2,5]thiadiazole-5-yle ;
4H-benzo[1,4]thiazine-3-one-6-yle ;
2,3-dihydro-benzo[1,4]dioxine-6-yle ;
benzo[1,2,3]thiadiazole-5-yle ;
3-oxo-3,4-dihydro-2H-benzo[1,4]oxazine-6-yle ;
7-fluoro-3-oxo-3,4-dihydro-2H-benzo[1,4]oxazine-6-yle ;
2-oxo-2,3-dihydro-1H-pyrido[2,3-b][1,4]thiazine-7-yle ;
2,3-dihydro-[1,4]dioxino[2,3-c]pyridine-7-yle ;
3-oxo-3,4-dihydro-2H-pyrido[3,2-b][1,4]oxazine-6-yle ;
[1,2,3]thiadiazole[5,4-b]pyridine-6-yle ;
3-oxo-3,4-dihydro-2*H*-pyrido[3,2-*b*][1,4]thiazine-6-yle ;
7-chloro-3-oxo-3,4-dihydro-2*H*-pyrido[3,2-*b*][1,4]thiazine-6-yle ;
7-fluoro-3-oxo-3,4-dihydro-2*H*-pyrido[3,2-*b*][1,4]thiazine-6-yle ; ou
2-oxo-2,3-dihydro-1*H*-pyrido[3,4-*b*][1,4]thiazine-7-yle.

11. Composé suivant la revendication 1, ledit composé étant :
la 6-({2-[1-(6-méthoxyquinoléine-4-yl)pipéridine-4-yl]-éthylamino}méthyl)-4*H*-pyrido[3,2-*b*][1,4]oxazine-3-one ;
la 6-({2-[1-(6-méthoxyquinoléine-4-yl)pipéridine-4-yl]-éthylamino}méthyl)-4*H*-pyrido[3,2-*b*][1,4]thiazine-3-one ;
la (2,3-dihydro-[1,4]dioxino[2,3-c]pyridine-7-ylméthyl)-12-[1-(6-méthoxyquinoléine-4-yl)pipéridine-4-yl]éthyllamine ;
la 6-({2-[1-(6-méthoxynaphtyridine-4-yl)pipéridine-4-yl]-éthylamino}méthyl)-4*H*-pyrido[3,2-*b*][1,4]oxazine-3-one ;
la 6-({2-[1-(6-méthoxynaphtyridine-4-yl)pipéridine-4-yl]-éthylamino}méthyl)-4*H*-pyrido[3,2-*b*][1,4]thiazine-3-one ;
la (2,3-dihydro-[1,4]dioxino[2,3-*c*]pyridine-7-ylméthyl)-{2-[1-(6-méthoxynaphtyridine-4-yl)pipéridine-4-yl]éthyl}amine ;
la 6-({2-[1-(3-chloro-6-méthoxy-[1,5]quinoléine-4-yl)-phényl]éthylamino}méthyl)-4*H*-pyrido[3,2-*b*][1,4]oxazine-3-one ;
la 6-({2-[1-(3-chloro-6-méthoxy-[1,5]quinoléine-4-yl)-phényl]éthylamino}méthyl)-4*H*-pyrido[3,2-*b*][1,4]thiazine-3-one ;
la {2-[1-(3-chloro-6-méthoxyquinoléine-4-yl)pipéridine-4-yl]éthyl}-(2,3-dihydro[1,4]dioxino[2,3-c]pyridine-7-ylméthyl)-amine ;
la 6-({2-[1-(3-chloro-6-méthoxy-[1,5]naphtyridine-4-yl)-phényl]éthylamino}méthyl)-4*H*-pyrido[3,2-*b*][1,4]oxazine-3-one ;
la 6-({2-[1-(3-chloro-6-méthoxy-[1,5]naphtyridine-4-yl)-phényl]éthylamino}méthyl)-4*H*-pyrido[3,2-*b*][1,4]thiazine-3-one ;
la {2-[1-(3-chloro-6-méthoxynaphtyridine-4-yl)pipéridine-4-yl]éthyl}-(2,3-dihydro[1,4]dioxino[2,3-c]pyridine-7-ylméthyl)-amine ;
la 6-({2-[4-(6-méthoxyquinoléine-4-yl)pipérazine-1-yl]-éthylamino}méthyl)-4*H*-pyrido[3,2-*b*][1,4]oxazine-3-one ;
la 6-({2-[4-(6-méthoxyquinoléine-4-yl)pipérazine-1-yl]-éthylamino}méthyl)-4*H*-pyrido[3,2-*b*][1,4]thiazine-3-one ;
la (2,3-dihydro-[1,4]dioxino[2,3-c]pyridine-7-ylméthyl)-{2-[4-(6-méthoxyquinoléine-4-yl)pipérizine-4-yl]éthyl}amine ;
la 6-({2-[4-(6-méthoxynaphtyridine-4-yl)pipérazine-1-yl]-éthylamino}méthyl)-4*H*-pyrido[3,2-*b*][1,4]oxazine-3-one ;
la 6-({2-[4-(6-méthoxynaphtyridine-4-yl)pipérazine-1-yl]-éthylamino}méthyl)-4*H*-pyrido[3,2-*b*][1,4]thiazine-3-one ;
la (2,3-dihydro-[1,4]dioxino[2,3-*c*]pyridine-7-ylméthyl)-{2-[4-(6-méthoxynaphtyridine-4-yl)pipérizine-1-yl]éthyl}amine ;
la 6-({2-[4-(3-chloro-6-méthoxyquinoléine-4-yl)pipérazine-1-yl]éthylamino}méthyl)-4*H*-pyrido[3,2-*b*][2,4]oxazine-3-one ;
la 6-({2-[4-(3-chloro-6-méthoxyquinoléine-4-yl)pipérazine-1-yl]éthylamino}méthyl)-4*H*-pyrido[3,2-*b*][2,4]thiazine-3-one ;
1a {2-[4-(3-chloro-6-méthoxyquinoléine-4-yl)pipérazine-1-yl]éthyl}-2,3-dihydro[1,4]dioxino[2,3-*c*]pyridine-7-ylméthyl)-amine ;
la 6-({2-[4-(3-chloro-6-méthoxynaphtyridine-4-yl)pipérazine-1-yl]éthylamino}méthyl)-4*H*-pyrido[3,2-*b*][2,4]oxazine-3-one ;
la 6-({2-[4-(3-chloro-6-méthoxynaphtyridine-4-yl)pipérazine-1-yl]éthylamino}méthyl)-4*H*-pyrido[3,2-*b*][2,4]thiazine-3-one ;
la {2-[4-(3-chloro-6-méthoxynaphtyridine-4-yl)pipérazine-1-yl]éthyl}-2,3-dihydro[1,4]dioxino[2,3-*c*]pyridine-7-ylméthyl)-amine ;
la 6-({2-[4-(6-méthoxy-[1,5]naphtyridine-4-yl)-3,6-dihydro-2H-pyridine-1-yl]-2-oxo-éthylamino}-méthyl)-4H-pyrido[3,2-b]-[1,4]-thiazine-3-one ;
le N-(2-{1-[6-(méthyloxy)-1,5-naphtyridine-4-yl]-4-pipéridinyl}éthyl)-3-oxo-3,4-dihydro-2H-pyrido[3,2-b][1,4]-thiazine-6-carboxamide ;
le N-(2-{1-[6-(méthyloxy)-1,5-naphtyridine-4-yl]-4-pipéridinyl}éthyl)-3-oxo-3,4-dihydro-2H-1,4-benzothiazine-6-sulfonamide ;
le N-méthyl-N-(2-{1-[6-(méthyloxy)-1,5-naphtyridine-4-yl]-4-pipéridinyl}éthyl)-3-oxo-3,4-dihydro-2H-pyrido[3,2-b]-[1,4]-thiazine-6-carboxamide ;
le N-méthyl-N-(2-{1-[6-(méthyloxy)-1,5-naphtyridine-4-yl]-4-pipéridinyl}éthyl)-3-oxo-3,4-dihydro-2H-1,4-benzothiazine-6-sulfonamide ;
le N-(2-{1-[3-chloro-6-(méthyloxy)-1,5-naphtyridine-4-yl}-4-pipéridinyl}éthyl)-3-oxo-3,4-dihydro-2H-1,4-benzothiazine-6-sulfonamide ;
le N-(2-{4-[6-(méthyloxy)-1,5-naphtyridine-4-yl]-1-pipérazinyl}éthyl)-3-oxo-3,4-dihydro-2H-pyrido[3,2-b][1,4]-thiazine-6-carboxamide ;
le N-méthyl-N-(2-{4-[6-(méthyloxy)-1,5-naphtyridine-4-yl]-1-pipérazinyl}éthyl)-3-oxo-3,4-dihydro-2H-pyrido[3,2-b]-[1,4]-thiazine-6-carboxamide ;
le N-(2-{4-[6-(méthyloxy)-1,5-naphtyridine-4-yl]-1-pipérazinyl}éthyl)-3-oxo-3,4-dihydro-2H-1,4-benzothiazine-6-sulfonamide ;
le N-méthyl-N-(2-{4-[6-(méthyloxy)-1,5-naphtyridine-4-yl]-1-pipérazinyl}éthyl)-3-oxo-3,4-dihydro-2H-1,4-benzothiazine-6-sulfonamide ;
la 6-{[(2-{4-[6-(méthyloxy)-1,5-naphtyridine-4-yl]hexahydro-1H-1,4-diazépine-1-yl}éthyl)amino]méthyl}-2H-pyrido[3,2-b]-[1,4]thiazine-3(4H)-one ;
le N-(2-{4-[6-(méthyloxy)-1,5-naphtyridine-4-yl]hexahydro-1H-1,4-diazépine-1-yl}éthyl)-3-oxo-3,4-dihydro-2H-1,4-benzothiazine-6-sulfonamide ;
la 6-{[(2-{(1R,4R)-5-[6-(méthyloxy)-1,5-naphtyridine-4-yl]-2,5-diazabicyclo[2.2.1]hept-2-yl}éthyl)amino]méthyl}-2H-pyrido[3,2-b][1,4]thiazine-3(4H)-one ;
la 6-{[(2-{4-hydroxy-1-[6-(méthyloxy)-1,5-naphtyridine-4-yl]-4-pipéridinyl}éthyl)amino]méthyl}-2H-pyrido[3,2-b]-[1,4]thiazine-3(4H)-one ;
la 6-{[(2-{4-hydroxy-1-[6-(méthyloxy)-1,5-naphtyridine-4-yl]-4-pipéridinyl}éthyl)amino]méthyl}-2H-pyrido[3,2-b]-[1,4]oxazine-3(4H)-one ;
le N-(2-{4-hydroxy-1-[6-(méthyloxy)-1,5-naphtyridine-4-yl]-4-pipéridinyl}éthyl)-3-oxo-3,4-dihydro-2H-1,4-benzothiazine-6-sulfonamide ; ou
un de ses sels pharmaceutiques.

12. Composition pharmaceutique, comprenant un composé suivant la revendication 1 et un support pharmaceutiquement acceptable.

13. Utilisation d'un composé suivant la revendication 1, dans la production d'un médicament destiné à être utilisé dans le traitement d'infections bactériennes chez des mammifères .
